# EUROPEAN PATENT SPECIFICATION

(11) **EP 1 606 407 B1**
(45) Date of publication and mention of the grant of the patent: **18.12.2013**
(21) Application number: 04722479.5
(22) Date of filing: 22.03.2004
(51) Int. Cl.: C12N 15/113, A61K 48/00

(54) **MODULATION OF EXON RECOGNITION IN PRE-MRNA BY INTERFERING WITH THE SECONDARY RNA STRUCTURE**
MODULATION DER EXON-ERKENNUNG IN DER PRE-MRNS DURCH INTERFERENZ MIT DER RNS SEKUNDÄRSTRUKTUR
MODULATION DE L'IDENTIFICATION D'EXONS DANS UN ARNM PREMESSAGER, PAR INTERFERENCE DANS UNE STRUCTURE ARN SECONDAIRE

(30) Priority: 21.03.2003 WO PCT/NL03/00214
(43) Date of publication of application: 21.12.2005
(73) Proprietor: Academisch Ziekenhuis Leiden, 2333 ZA Leiden (NL)
(72) Inventor: VAN OMMEN, Garrit-Jan, Boudewijn, NL-1015 LW Amsterdam (NL); VAN DEUTEKOM, Judith, Christina, Theodora, NL-3315 DK Dordrecht (NL); DEN DUNNEN, Johannes, Theodorus, NL-3069 LA Rotterdam (NL); AARTSMA-RUS, Annemieke, 2134 BE Hoofddorp (NL)
(74) Representative: Nederlandsch Octrooibureau
(86) International application number: PCT/NL2004/000196
(87) International publication number: WO 2004/083446

(56) References cited:
- EP-A- 1 191 097
- WO-A-02/29056
- MANN CHRISTOPHER J ET AL: "Antisense-induced exon skipping and synthesis of dystrophin in the mdx mouse." PROCEEDINGS OF THE NATIONAL ACADEMY OF SCIENCES OF THE UNITED STATES, vol. 98, no. 1, 2 January 2001 (2001-01-02), pages 42-47, XP002250903 January 2, 2001 ISSN: 0027-8424
- MANN CHRISTOPHER J ET AL: "Improved antisense oligonucleotide induced exon skipping in the mdx mouse model of muscular dystrophy." THE JOURNAL OF GENE MEDICINE. ENGLAND 2002 NOV-DEC, vol. 4, no. 6, November 2002 (2002-11), pages 644-654, XP009015057 ISSN: 1099-498X
- DUNCKLEY MATTHEW G ET AL: "Modification of splicing in the dystrophin gene in cultured Mdx muscle cells by antisense oligoribonucleotides." HUMAN MOLECULAR GENETICS, vol. 7, no. 7, July 1998 (1998-07), pages 1083-1090, XP002250904 ISSN: 0964-6906
- VAN DEUTEKOM JUDITH C T ET AL: "Antisense-induced exon skipping restores dystrophin expression in DMD patient derived muscle cells." HUMAN MOLECULAR GENETICS, vol. 10, no. 15, 2001, pages 1547-1554, XP002250908 ISSN: 0964-6906
- AARTSMA-RUS ANNEMIEKE ET AL: "Targeted exon skipping as a potential gene correction therapy for Duchenne muscular dystrophy." NEUROMUSCULAR DISORDERS: NMD. ENGLAND OCT 2002, vol. 12 Suppl 1, October 2002 (2002-10), pages S71-S77, XP002250906 ISSN: 0960-8966
- DE ANGELIS FERNANDA GABRIELLA ET AL: "Chimeric snRNA molecules carrying antisense sequences against the splice junctions of exon 51 of the dystrophin pre-mRNA induce exon skipping and restoration of a dystrophin synthesis in Delta 48-50 DMD cells." PROCEEDINGS OF THE NATIONAL ACADEMY OF SCIENCES OF THE UNITED STATES OF AMERICA. UNITED STATES 9 JUL 2002, vol. 99, no. 14, 9 July 2002 (2002-07-09), pages 9456-9461, XP002250907 ISSN: 0027-8424
- SUTER D ET AL: "DOUBLE-TARGET ANTISENSE U7 SNRNAS PROMOTE EFFICIENT SKIPPING OF AN ABERRANT EXON IN THREE HUMAN BETA-THALASSEMIC MUTATIONS" HUMAN MOLECULAR GENETICS, OXFORD UNIVERSITY PRESS, SURREY, GB, vol. 8, December 1999 (1999-12), pages 2415-2423, XP000973208 ISSN: 0964-6906 cited in the application
- DICKSON G ET AL: "Screening for antisense modulation of dystrophin pre-mRNA splicing." NEUROMUSCULAR DISORDERS: NMD. ENGLAND OCT 2002, vol. 12 Suppl 1, October 2002 (2002-10), pages S67-S70, XP009015078 ISSN: 0960-8966
- RANDO THOMAS A: "Oligonucleotide-mediated gene therapy for muscular dystrophies." NEUROMUSCULAR DISORDERS : NMD. OCT 2002, vol. 12 Suppl 1, October 2002 (2002-10), pages S55-S60, XP002299065 ISSN: 0960-8966

## Description

The invention relates to the fields of molecular biology and medicine. More in particular the invention relates to a method for generating an oligonucleotide for the restructuring of mRNA produced from pre-mRNA.

The central dogma of biology is that genetic information resides in the DNA of a cell and is expressed upon transcription of this information, where after production of the encoded protein follows by the translation machinery of the cell. This view of the flow of genetic information has prompted the pre-dominantly DNA based approach for interfering with the protein content of a cell. This view is slowly changing and alternatives for interfering at the DNA level are being pursued.

In higher eukaryotes the genetic information for proteins in the DNA of the cell is encoded in exons which are separated from each other by intronic sequences. These introns are in some cases very long. The transcription machinery generates a pre-mRNA which contains both exons and introns, while the splicing machinery, often already during the production of the pre-mRNA, generates the actual coding region for the protein by splicing together the exons present in the pre-mRNA.

Although much is known about the actual processes involved in the generation of an mRNA from a pre-mRNA, much also remains hidden. In the present invention it has been shown possible to influence the splicing process such that a different mRNA is produced. The process allows for the predictable and reproducible restructuring of mRNA produced by a splicing machinery. An oligonucleotide capable of hybridising to pre-mRNA at a location of an exon that is normally included in the mature mRNA can direct the exclusion of the thus targeted exon or a part thereof.

In the present invention means and methods are provided for the design of appropriate complementary oligonucleotides. To this end the invention provides a method for generating an oligonucleotide comprising determining, from a (predicted) secondary structure of RNA from an exon, a region that assumes a structure that is hybridised to another part of said RNA (closed structure) and a region that is not hybridised in said structure (open structure), and subsequently generating an oligonucleotide, which at least in part is complementary to said closed structure and which at least in part is complementary to said open structure. RNA molecules exhibit strong secondary structures, mostly due to base pairing of complementary or partly complementary stretches within the same RNA. It has long since been thought that structures in the RNA play a role in the function of the RNA. Without being bound by theory, it is believed that the secondary structure of the RNA of an exon plays a role in structuring the splicing process. Through its structure, an exon is recognized as a part that needs to be included in the mRNA. Herein this signalling function is referred to as an exon inclusion signal. A complementary oligonucleotide of the disclosure is capable of interfering with the structure of the exon and thereby capable of interfering with the exon inclusion signal of the exon. It has been found that many complementary oligonucleotides indeed comprise this capacity, some more efficient than others. Oligonucleotides of the disclosure, i.e. those with the said overlap directed toward open and closed structures in the native exon RNA, are a selection from all possible oligonucleotides. The selection encompasses oligonucleotides that can efficiently interfere with an exon inclusion signal. Without being bound by theory it is thought that the overlap with an open structure improves the invasion efficiency of the oligonucleotide (i.e. increases the efficiency with which the oligonucleotide can enter the structure), whereas the overlap with the closed structure subsequently increases the efficiency of interfering with the secondary structure of the RNA of the exon, and thereby interfere with the exon inclusion signal. It is found that the length of the partial complementarity to both the closed and the open structure is not extremely restricted. We have observed high efficiencies with oligonucleotides with variable lengths of complementarity in either structure. The term complementarity is used herein to refer to a stretch of nucleic acids that can hybridise to another stretch of nucleic acids under physiological conditions. It is thus not absolutely required that all the bases in the region of complementarity are capable of pairing with bases in the opposing strand. For instance, when designing the oligonucleotide one may want to incorporate for instance a residue that does not base pair with the base on the complementary strand. Mismatches may to some extent be allowed, if under the circumstances in the cell, the stretch of nucleotides is capable of hybridising to the complementary part. In a preferred embodiment a complementary part (either to said open or to said closed structure) comprises at least 3, and more preferably at least 4 consecutive nucleotides. The complementary regions are preferably designed such that, when combined, they are specific for the exon in the pre-mRNA. Such specificity may be created with various lengths of complementary regions as this depends on the actual sequences in other (pre-)mRNA in the system. The risk that also one or more other pre-mRNA will be able to hybridise to the oligonucleotide decreases with increasing size of the oligonucleotide. It is clear that oligonucleotides comprising mismatches in the region of complementarity but that retain the capacity to hybridise to the targeted region(s) in the pre-mRNA, can be used in the present invention. However, preferably at least the complementary parts do not comprise such mismatches as these typically have a higher efficiency and a higher specificity, than oligonucleotides having such mismatches in one or more complementary regions. It is thought that higher hybridisation strengths, (i.e. increasing number of interactions with the opposing strand) are favourable in increasing the efficiency of the process of interfering with the splicing machinery of the system.

The secondary structure is best analysed in the context of the pre-mRNA wherein the exon resides. Such structure may be analysed in the actual RNA. However, it is currently possible to predict the secondary structure of an RNA molecule (at lowest energy costs) quite well using structure-modelling programs. A non-limiting example of a suitable program is RNA mfold version 3.1 server (Mathews et al 1999, J. Mol. Biol. 288: 911-940). A person skilled in the art will be able to predict, with suitable reproducibility, a likely structure of the exon, given the nucleotide sequence. Best predictions are obtained when providing such modelling programs with both the exon and flanking intron sequences. It is typically not necessary to model the structure of the entire pre-mRNA.

In a first aspect there is provided a method according to claim 1.

The open and closed structure to which the oligonucleotide is directed, are preferably adjacent to one another. It is thought that in this way the annealing of the oligonucleotide to the open structure induces opening of the closed structure whereupon annealing progresses into this closed structure. Through this action the previously closed structure assumes a different conformation. The different conformation results in the disruption of the exon inclusion signal. However, when potential (cryptic) splice acceptor and/or donor sequences are present within the targeted exon, occasionally a new exon inclusion signal is generated defining a different (neo) exon, i.e. with a different 5' end, a different 3' end, or both. This type of activity is within the scope of the present invention as the targeted exon is excluded from the mRNA. The presence of a new exon, containing part of the targeted exon, in the mRNA does not alter the fact that the targeted exon, as such, is excluded. The inclusion of a neo-exon can be seen as a side effect which occurs only occasionally. There are two possibilities when exon skipping is used to restore (part of) an open reading frame that was disrupted as a result of a mutation. One is that the neo-exon is functional in the restoration of the reading frame, whereas in the other case the reading frame is not restored. When selecting oligonucleotides for restoring reading frames by means of exon-skipping it is of course clear that under these conditions only those oligonucleotides are selected that indeed result in exon-skipping that restores the open reading frame, with or without a neo-exon.

Pre-mRNA can be subject to various splicing events, for instance through alternative splicing. Such events may be induced or catalysed by the environment of a cell or artificial splicing system. Thus, from the same pre-mRNA several different mRNA's may be produced. The different mRNA's all included exonic sequences, as that is the definition of an exon. However, the fluidity of the mRNA content necessitates a definition of the term exon in the present invention. An exon according to the invention is a sequence present in both the pre-mRNA and mRNA produced thereof, wherein the sequence included in the mRNA is, in the pre-mRNA, flanked on one side (first and last exon) or both sides (any other exon then the first and the last exon) by sequences not present in the mRNA. In principle any mRNA produced from the pre-mRNA qualifies for this definition. However, for the present invention, so-called dominant mRNA's are preferred, i.e. mRNA that makes up at least 5% of the mRNA produced from the pre-mRNA under the set conditions. Human immuno-deficiency virus in particular uses alternative splicing to an extreme. Some very important protein products are produced from mRNA making up even less than 5% of the total mRNA produced from said virus. The genomic RNA of retroviruses can be seen as pre-mRNA for any spliced product derived from it. As alternative splicing may vary in different cell types the exons are defined as exons in the context of the splicing conditions used in that system. As a hypothetical example; an mRNA in a muscle cell may contain an exon that as absent in an mRNA produced from the same pre-mRNA in a nerve cell. Similarly, mRNA in a cancer cell may contain an exon not present in mRNA produced from the same mRNA in a normal cell.

Alternative splicing may occur by splicing from the same pre-mRNA. However, alternative splicing may also occur through a mutation in the pre-mRNA for instance generating an additional splice acceptor and/or splice donor sequence. Such alternative splice sequences are often referred to as cryptic splice acceptor/donor sequences. Such cryptic splice sites can result in new exons (neo-exons). Inclusion of neo-oxons into produced mRNA can be at least in part prevented using a method of the disclosure. In case a neo-exon is flanked by a cryptic and a "normal" splice donor/acceptor sequence, the neo-exon encompasses the old (paleo) exon. If in this case the original splice donor/acceptor sequence, for which the cryptic splice donor/acceptor has taken its place, is still present in the pre-mRNA, it is possible to enhance the production of mRNA containing the paleo-exon by interfering with the exon-recognition signal of the neo-exon. This interference can be both in the part of the neo-exon corresponding to the paleo-exon, or the additional part of such neo-exons. This type of exon skipping can be seen as splice correction.

The exon skipping technique can be used for many different purposes. Preferably, however, exon skipping is used for restructuring mRNA that is produced from pre-mRNA exhibiting undesired splicing in a subject. The restructuring may be used to decrease the amount of protein produced by the cell. This is useful when the cell produces a particular undesired protein. In the disclosure however, restructuring is used to promote the production of a functional protein in a cell, i.e. restructuring leads to the generation of a coding region for a functional protein. The latter disclosure is preferably used to restore an open reading frame that was lost as a result of a mutation. Preferred genes comprise a Duchenne muscular dystrophy gene, a collagen VI alpha 1 gene (COL6A1), a myotubular myopathy 1 gene (MTM1), a dysferlin gene (DYSF), a laminin-alpha 2 gene (LAMA2), an emery-dreyfuse muscular dystrophy gene (EMD), and/or a calpain 3 gene (CAPN3). The invention is further delineated by means of examples drawn from the Duchenne muscular dystrophy gene. Although this gene constitutes a particularly preferred gene in the present invention, the invention is not limited to this gene.

Duchenne muscular dystrophy (DMD) and Becker muscular dystrophy (BMD) are both caused by mutations in the DMD gene, that is located on the X chromosome and codes for dystrophin (1-6). DMD has an incidence of 1:3500 newborn males. Patients suffer from progressive muscle weakness, are wheelchair bound before the age of 13 and often die before the third decade of their life (7). The generally milder BMD has an incidence of 1:20,000. BMD patients often remain ambulant for over 40 years and have longer life expectancies when compared to DMD patients (8).

Dystrophin is an essential component of the dystrophin-glycoprotein complex (DGC), which amongst others maintains the membrane stability of muscle fibers (9, 10). Frame-shifting mutations in the DMD gene result in dystrophin deficiency in muscle cells. This is accompanied by reduced levels of other DGC proteins and results in the severe phenotype found in DMD patients (11, 12). Mutations in the DMD gene that keep the reading frame intact, generate shorter, but partly functional dystrophins, associated with the less severe BMD (13, 14).

Despite extensive efforts, no clinically applicable and effective therapy for DMD patients has yet been developed (15), although a delay of the onset and/or progression of disease manifestations can be achieved by glucocorticoid therapy (16). Promising results have recently been reported by us and others on a genetic therapy aimed at restoring the reading frame of the dystrophin pre-mRNA in cells from the mdx mouse model and DMD patients (17-23). By the targeted skipping of a specific exon, a DMD phenotype can be converted into a milder BMD phenotype. The skipping of an exon can be induced by the binding of antisense oligoribonucleotides (AONs) targeting either one or both of the splice sites, or exon-internal sequences. Since an exon will only be included in the mRNA when both the splice sites are recognised by the spliceosome complex, splice sites are obvious targets for AONs. This was shown to be successful, albeit with variable efficacy and efficiency (17, 18, 20, 21). We hypothesised that targeting exon-internal sequences might increase specificity and reduce interference with the splicing machinery itself. Some exons have weak splice sites and appear to require binding of a SR protein to an exon recognition sequence (ERS) or an exonic splicing enhancer (ESE) to be properly recognised by the splicing machinery (24). SR proteins are a highly conserved family of arginine/serine rich, spliceosome associated phosphoproteins essential for pre-mRNA splicing (50, 51). SR proteins appear to act early in splicing by promoting splice site recognition and spliceosome assembly. SR proteins also play a regulatory role, because they can determine alternative splice site usage in vivo and in vitro. SR proteins appear to be recruited from nuclear "speckles", in which they are concentrated, to sites of transcription in order to spatially coordinate transcription and pre-mRNA splicing within the cell nucleus (49, 52). Disruptive point mutations or AONs that block these sequences have been found to result in exon skipping (19, 22, 24-28). Using exon-internal AONs specific for an ERS-like sequence in exon 46, we were previously able to modulate the splicing pattern in cultured myotubes from two different DMD patients with an exon 45 deletion (19). Following AON treatment, exon 46 was skipped, which resulted in a restored reading frame and the induction of dystrophin synthesis in at least 75% of the cells. We have recently shown that exon skipping can also efficiently be induced in human control muscle cells for 15 different DMD exons using exon-internal AONs (23, unpublished results). In contrast to the previous opinion that skipping can only be achieved with weak splice sites or exons containing ERS-like sequences, we have seen that of the exons that were skipped in the present disclosure most do not have weak splice sites nor do they contain ERS-like sequences. Thus binding of the AONs to the targeted exon per se is sufficient to cause exon skipping, either by interfering with one or more components of the splicing machinery or by altering the secondary structure of the RNA in such a manner that the splicing machinery no longer recognizes the exon. In a preferred embodiment the exon to be skipped comprises exons 2, 8, 9, 17, 19, 29, 40-46, 48-53, 55 or 59. More preferably, exons 2, 8, 9, 17, 40, 41, 42, 44, 46, 48, 49-52, 55or 59. In yet another embodiment the exon to be skipped comprises exons 2, 29, 40, 41, 42, 43, 44, 45, 46, 48, 49, 50, 51, 53 or 55.

Any oligonucleotides fulfilling the requirements of the invention may be used to induce exon skipping in the DMD gene. In the disclosure an oligonucleotide comprises a sequence as depicted as active in exon-skipping in table 2, or a functional equivalent thereof comprising a similar, preferably the same hybridisation capacity in kind, not necessarily in amount. Disclosed is an oligonucleotide comprising a sequence as depicted in table 2, derived from the exons 2, 40, 41, 42, 43, 44, 45, 46, 48, 49, 50, 51, 53 or 55, active in exon skipping. The disclosure thus further provides an oligonucleotide of table 2, or an equivalent thereof. The disclosure provides an oligonucleotide of table capable of inducing exon skipping as depicted in table 2. The disclosure further provides an oligonucleotide of table 2, complementary to exons 2, 40, 41, 42, 43, 44, 45, 46, 48, 49, 50, 51, 53 or 55 of the human DMD gene.

Reading frame correction can be achieved by skipping one or two exons flanking a deletion, by skipping in-frame exons containing a nonsense mutation, or by skipping duplicated exons. This results in proteins similar to those found in various BMD patients (2, 29). A survey of the Leiden DMD mutation database [www.dmd.nl; (30)] learns that we can thus correct over 75% of DMD causing mutations (see Table 4). We show the actual therapeutic effect of exon skipping for 7 different mutations. In all patient muscle cell cultures, we were able to restore dystrophin synthesis in 75% to 80% of treated cells.

The complementary oligonucleotide generated through a method of the invention is preferably complementary to a consecutive part of between 13 and 50 nucleotides of said exon RNA. In another embodiment the complementary oligonucleotide generated through a method of the invention is complementary to a consecutive part of between 16 and 50 nucleotides of said exon RNA. Preferably, the oligonucleotide is complementary to a consecutive part of between 13-25 nucleotides of said exon RNA. Preferably between 14 and 25 nucleotides of said exon RNA. Different types of nucleic acid may be used to generate the oligonucleotide. Preferably, the oligonucleotide comprises RNA, as RNA/RNA hybrids are very stable. Since one of the aims of the exon skipping technique is to direct splicing in subjects it is preferred that the oligonucleotide RNA comprises a modification providing the RNA with an additional property, for instance resistance to endonucleases and RNaseH, additional hybridisation strength, increased stability (for instance in a bodily fluid), increased or decreased flexibility, reduced toxicity, increased intracellular transport, tissue-specificity, etc. Preferably said modification comprises a 2'-O-methyl-phosphorothioate oligoribonucleotide modification. Preferably said modification comprises a 2'-O-methyl-phosphorothioate oligodeoxyribonucleotide modification. Herein is disclosed a hybrid oligonucleotide comprising an oligonucleotide comprising a 2'-O-methyl-phosphorothioate oligo(deoxy)ribonucleotide modification and locked nucleic acid. This particular combination comprises better sequence specificity compared to an equivalent consisting of locked nucleic acid, and comprises improved effectivity when compared with an oligonucleotide consisting of 2'-O-methyl-phosphorothioate oligo(deoxy)ribonucleotide modification.

With the advent of nucleic acid mimicking technology it has become possible to generate molecules that have a similar, preferably the same hybridisation characteristics in kind not necessarily in amount as nucleic acid itself. Such equivalents are of course also part of the invention. Examples of such mimics equivalents are peptide nucleic acid, locked nucleic acid and/or a morpholino phosphorodiamidate. Suitable but non-limiting examples of equivalents of oligonucleotides of the disclosure can be found in (Wahlestedt, C. et al. Potent and non-toxic antisense oligonucleotides containing locked nucleic acids. Proc Natl Acad Sci U S A 97, 5633-8. (2000). Elayadi, A.N. & Corey, D.R. Application of PNA and LNA oligomers to chemotherapy. Curr Opin Investig Drugs 2, 558-61. (2001). Larsen, H.J., Bentin, T. & Nielsen, P.E. Antisense properties of peptide nucleic acid. Biochim Biophys Acta 1489, 159-66. (1999). Braasch, D.A. & Corey, D.R. Novel antisense and peptide nucleic acid strategies for controlling gene expression. Biochemistry 41, 4503-10. (2002). Summerton, J. & Weller, D. Morpholino antisense oligomers: design, preparation, and properties. Antisense Nucleic Acid Drug Dev 7, 187-95(1997). Hybrids between one or more of the equivalents among each other and/or together with nucleic acid are of course also part of the invention. In a preferred embodiment an equivalent comprises locked nucleic acid, as locked nucleic acid displays a higher target affinity and reduced toxicity and therefore shows a higher efficiency of exon skipping.

An oligonucleotide of the disclosure typically does not have to overlap with a splice donor or splice acceptor of the exon.

An oligonucleotide of the disclosure, or equivalent thereof, may of course be combined with other methods for interfering with the structure of an mRNA. It is for instance possible to include in a method at least one other oligonucleotide that is complementary to at least one other exon in the pre-mRNA. This can be used to prevent inclusion of two or more exons of a pre-mRNA in mRNA produced from this pre-mRNA. In a preferred embodiment, said at least one other oligonucleotide is an oligonucleotide, or equivalent thereof, generated through a method of the invention. This part of the disclosure is further referred to as double-or multi-exon skipping. In most cases double-exon skipping results in the exclusion of only the two targeted (complementary) exons from the pre-mRNA. However, in other cases it was found that the targeted exons and the entire region in between said exons in said pre-mRNA were not present in the produced mRNA even when other exons (intervening exons) were present in such region. This multi-skipping was notably so for the combination of oligonucleotides derived from the DMD gene, wherein one oligonucleotide for exon 45 and one oligonucleotide for exon 51 was added to a cell transcribing the DMD gene. Such a set-up resulted in mRNA being produced that did not contain exons 45 to 51. Apparently, the structure of the pre-mRNA in the presence of the mentioned oligonucleotides was such that the splicing machinery was stimulated to connect exons 44 and 52 to each other.

In a second aspect there is provided a method according to claim 14.

In the present disclosure it has found possible to specifically promote the skipping of also the intervening exons by providing a linkage between the two complementary oligonucleotides. To this end the disclosure provides a compound capable of hybridising to at least two exons in a pre-mRNA encoded by a gene, said compound comprising at least two parts wherein a first part comprises an oligonucleotide having at least 8 consecutive nucleotides that are complementary to a first of said at least two exons, and wherein a second part comprises an oligonucleotide having at least 8 consecutive nucleotides that are complementary to a second exon in said pre-mRNA. The at least two parts are linked in said compound so as to form a single molecule. The linkage may be through any means but is preferably accomplished through a nucleotide linkage. In the latter case the number of nucleotides that not contain an overlap between one or the other complementary exon can be zero, but is preferably between 4 to 40 nucleotides. The linking moiety can be any type of moiety capable of linking oligonucleotides. Currently, many different compounds are available that mimic hybridisation characteristics of oligonucleotides. Such a compound is also suitable for the present disclosure if such equivalent comprises similar hybridisation characteristics in kind not necessarily in amount. Suitable equivalents were mentioned earlier in this description. One or preferably, more of the oligonucleotides in the compound are generated by a method for generating an oligonucleotide of the present invention. As mentioned, oligonucleotides of the disclosure do not have to consist of only oligonucleotides that contribute to hybridisation to the targeted exon. There may be additional material and/or nucleotides added.

As mentioned, a preferred gene for restructuring mRNA is the DMD gene. The DMD gene is a large gene, with many different exons. Considering that the gene is located on the X-chromosome, it is mostly boys that are affected, although girls can also be affected by the disease, as they may receive a bad copy of the gene from both parents, or are suffering from a particularly biased inactivation of the functional allele due to a particularly biased X chromosome inactivation in their muscle cells. The protein is encoded by a plurality of exons (79) over a range of at least 2,6 Mb. Defects may occur in any part of the DMD gene. Skipping of a particular exon or particular exons can, very often, result in a restructured mRNA that encodes a shorter than normal but at least partially functional dystrophin protein. A practical problem in the development of a medicament based on exon-skipping technology is the plurality of mutations that may result in a deficiency in functional dystrophin protein in the cell. Despite the fact that already multiple different mutations can be corrected for by the skipping of a single exon, this plurality of mutations, requires the generation of a large number of different pharmaceuticals as for different mutations different exons need to be skipped. An advantage of a compound of the disclosure, i.e. a compound capable of inducing skipping of two or more exons, is that more than one exon can be skipped with a single pharmaceutical. This property is not only practically very useful in that only a limited number of pharmaceuticals need to be generated for treating many different Duchenne or Becker mutations. Another option now open to the person skilled in the art is to select particularly functional restructured dystrophin proteins and produce compounds capable of generating these preferred dystrophin proteins. Such preferred end results are further referred to as mild phenotype dystrophins. The structure of the normal dystrophin protein can be schematically represented as two endpoints having structural function (the beads), which are connected to each other by a long at least partly flexible rod. This rod is shortened in many Becker patients. The disclosure provides a method for treating a DMD patient comprising a mutation as depicted in table 4, comprising providing said patient with an oligonucleotide effective in inducing exon-skipping of the exon mentioned in the first column of 4, or an equivalent thereof. In the disclosure said oligonucleotide comprises an oligonucleotide effective in inducing exon-skipping mentioned in table 2, or an equivalent thereof.

The observations mentioned above led the field to the conclusion that not so much the length of the rod but the presence of a rod and the composition thereof (with respect to particular hinge regions in the protein), is crucial to the function per se of the dystrophin protein. Though the size of the rod may have an impact on the amount of functionality of the resulting (Becker) protein, there are many notable exceptions. These exceptions will be detailed below. There are especially benign mutations that can have a very short rod. It was noted by the inventors that many more different types of Becker patients should have been detected in the patient population. However, some types of shortened dystrophin proteins, that according to this hypothesis should have a Becker phenotype, are not detected in human population. For some of these "theoretical" Becker forms, this could just be a matter of chance. However, in the disclosure it has been found, that at least some of these "potential" Becker patients have such a benign phenotype that subjects having these types of mutations do not present themselves to a doctor, or are not diagnosed as suffering from Becker's disease. With a compound of the disclosure it is possible to restructure DMD pre-mRNA of many different Duchenne and even Becker patients such that a mild phenotype dystrophin is generated after translation of the restructured mRNA. The disclosure thus provides particularly preferred compound, wherein the parts of the compounds at least comprise a first part comprising an oligonucleotide or equivalent thereof, complementary to exon 17 and a second part comprising an oligonucleotide or equivalent thereof, complementary to exon 48. The resulting restructured mRNA encodes an in-frame shortened dystrophin protein, lacking all exons from 17 to 48. This shortened dystrophin protein mimics a mild phenotype dystrophin as mentioned above. The compound (referred to as the 17-48 compound) should according to current databases be able to deal with as much as 20% of the patients having a DMD mutation currently characterised. Another preferred compound is the 45-55 compound. This compound should according to the same calculations be able to deal with 38% of the patients having a DMD mutation thus far characterised. In the disclosure the compound comprises a 42-55 compound or a 49-59 compound, capable of dealing with respectively 65% and 18% of the currently characterized DMD patients. In the disclosure, the compound comprises a 42-55 compound. Disclosed are a 45-49 compound and a 45-51 compound preferably in the form as disclosed in the experimental part, having the potential to treat respectively 4% and 8% of the DMD patients characterised thus far. In the disclosure said compound comprises an oligonucleotide of table 2, or an equivalent thereof. Preferably, said compound comprises at least two oligonucleotides of table 2 one or more equivalents thereof. In the disclosure said compound comprises at least one oligonucleotide or equivalent thereof of table 2, directed against exon 42 and an oligonucleotide or equivalent thereof of table 2, directed against exon 55. In the disclosure said compound comprises at least one oligonucleotide or equivalent thereof of table 2, directed against exon 45 and an oligonucleotide or equivalent thereof of table 2, directed against exon 51.

Also part of the disclosure is a compound capable of hybridising to one exon in a pre-mRNA encoded by a gene, said compound comprising at least two parts wherein a first part comprises an oligonucleotide of which at least a part of said oligonucleotide is complementary to said closed structure and wherein second part comprises an oligonucleotide of which at least part is complementary to said open structure. The open and closed structures are of course determined from a secondary structure of RNA from said exon. Preferably a compound having two distinguishable parts complementary to a single exon, comprises an oligonucleotide, or equivalent thereof, or combination thereof as mentioned above in the method for generating said oligonucleotide.

The disclosure further provides a composition comprising a first oligonucleotide of the invention capable of hybridising to an exon in a pre-mRNA of a gene or an equivalent of said first oligonucleotide, and at least a second oligonucleotide of the disclosure capable of hybridising to another exon in a pre-mRNA of a gene or an equivalent of said second oligonucleotide. In the disclosure said first and at least said second oligonucleotide or equivalent thereof are capable of hybridising to different exons on the same pre-mRNA. The composition can be used to induce exon skipping of the respective exons. It has been observed that when the composition comprises oligonucleotides or equivalents thereof directed toward exons 45 and 51, or 42 and 55 of the human DMD gene, that as an exception to the rule that only the targeted exons are excluded from the resulting mRNA, instead the targeted exons and the entire intervening region is excluded from the resulting mRNA. In the present disclosure this feature is used to correct a variety of different debilitating mutations of the DMD gene. Thus the disclosure provides a method for the treatment of a subject comprising a mutation in the human DMD gene, wherein as a result of said mutation the DMD gene is not appropriately translated into a functional dystrophin protein, comprising providing said subject with a composition as mentioned above. Mutations that can be corrected in this way are typically mutations that lie within or adjacent to the targeted exon or in the intervening region. However, it is also possible to correct frame-shifting mutations that lie further outside the mentioned exons and intervening region.

A transcription system containing a splicing system can be generated in vitro. The art has suitable systems available. However, the need for mRNA restructuring is of course predominantly felt for the manipulation of living cells. Preferably, cells in which a desired effect can be achieved through the restructuring of an mRNA. Preferred mRNA's that are restructured are listed herein above. Preferably, genes active in muscle cells are used in the present invention. Muscle cells (i.e. myotubes) are multinucleated cells in which many but not all muscle cell specific genes are transcribed via long pre-mRNA. Such long pre-mRNA's are preferred for the present disclosure as restructuring of mRNA's produced from such long mRNA's is particularly efficient. It is thought, though it need not necessarily be so, that the relatively long time needed to generate the full pre-mRNA aids the efficiency of restructuring using a method or means of the disclosure, as more time is allowed for the process to proceed. The preferred group of genes of which the mRNA is preferably restructured in a method of the invention comprises: COL6A1 causing Bethlem myopathy, MTM1 causing myotubular myopathy, DYSF (dysferlin causing Miyoshi myopathy and LGMD, LAMA2 (laminin alpha 2) causing Merosin-deficient muscular dystrophy, EMD (emerin) causing Emery-Dreyfuss muscular dystrophy, the DMD gene causing Duchenne muscular dystrophy and Becker muscular dystrophy, and CAPN3 (calpain) causing LGMD2A. Any cell may be used, however, as mentioned, a preferred cell is a cell derived from a DMD patient. Cells can be manipulated in vitro, i.e. outside the subject's body. However, ideally the cells are provided with a restructuring capacity in vivo. Suitable means for providing cells with an oligonucleotide, equivalent or compound of the disclosure are present in the art. Improvements in these techniques are anticipated considering the progress that has already thus far been achieved. Such future improvements may of course be incorporated to achieve the mentioned effect on restructuring of mRNA using a method of the disclosure. At present suitable means for delivering an oligonucleotide, equivalent or compound of the disclosure to a cell in vivo comprise, polyethylenimine (PEI) or synthetic amphiphils (SAINT-18) suitable for nucleic acid transfections. The amphiphils show increased delivery and reduced toxicity, also when used for in vivo delivery. Preferably compounds mentioned in ( 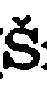 misterová, J., Wagenaar, A., Stuart, M.C.A., Polushkin, E., ten Brinke, G., Hulst, R., Engberts, J.B.F.N., Hoekstra, D., 'Molecular shape of the Cationic Lipid Controls the Structure of the Cationic Lipid/ Dioleylphosphatidylethanolamine-DNA Complexes and the Efficiency of Gene Delivery', J. Biol. Chem. 2001, 276, 47615). The synthetic amphiphils preferably used are based upon the easily synthetically available 'long tailed' pyridinium head group based materials. Within the large group of amphiphils synthesized, several show a remarkable transfection potential combined with a low toxicity in terms of overall cell survival. The ease of structural modification can be used to allow further modifications and the analysis of their further (in vivo) nucleic acid transfer characteristics and toxicity.

An oligonucleotide, equivalent thereof, or a compound according to the disclosure may be used for at least in part altering recognition of said exon in a pre-mRNA. In the disclosure the splicing machinery is at least in part prevented from linking the exon boundaries to the mRNA. The oligonucleotide, equivalent or compound of the disclosure is at least in part capable of altering exon-recognition in a pre-mRNA. This use is thus also provided in the disclosure. The prevention of inclusion of a targeted exon in an mRNA is also provided as a use for at least in part stimulating exon skipping in a pre-mRNA. As mentioned above, the targeted exon is not included in the resulting mRNA. However, part of the exon (a neo-exon) may occasionally be retained in the produced mRNA. This sometimes occurs when the targeted exon contains a potential splice acceptor and/or splice donor sequence. In the disclosure the splicing machinery is redirected to utilize a previously not (or underused) splice acceptor/donor sequence, thereby creating a new exon (neo-exon). The neo-exon may have one end in common with the paleo-exon, although this does not always have to be the case. Thus in the disclosure an oligonucleotide, equivalent or compound of the invention is used for altering the efficiency with which a splice donor or splice acceptor is used by a splicing machinery.

In view of the above, the present disclosure further provides the use of an oligonucleotide, an equivalent thereof or a compound of the disclosure for the preparation of a medicament. Further provided is a pharmaceutical preparation comprising an oligonucleotide, equivalent thereof or a compound according to the disclosure. Said an oligonucleotide, an equivalent thereof or a compound of the disclosure can be used for the preparation of a medicament for the treatment of an inherited disease. Similarly provided is a method for altering the efficiency with which an exon in a pre-mRNA is recognized by a splicing machinery, said pre-mRNA being encoded by a gene comprising at least two exons and at least one intron, said method comprising providing a transcription system comprising said splicing machinery and said gene, with an oligonucleotide, equivalent thereof or a compound according to the disclosure, wherein said oligonucleotide, equivalent thereof or compound is capable of hybridising to at least one of said exons, and allowing for transcription and splicing to occur in said transcription system. Preferably, said gene comprises at least 3 exons.

An oligonucleotide of the disclosure, may be provided to a cell in the form of an expression vector wherein the expression vector encodes a transcript comprising said oligonucleotide. The expression vector is preferably introduced into the cell via a gene delivery vehicle. A preferred delivery vehicle is a viral vector such as an adenoviral vector and more preferably an adeno-associated virus vector. The disclosure thus also provides such expression vectors and delivery vehicles. It is within the skill of the artisan to design suitable transcripts. Disclosed are PolIII driven transcripts. Preferably, in the form of a fusion transcript with an Ulor U7 transcript. Such fusions may be generated as described in references 53 and 54.

### Examples

### Example 1

### Results

This study includes 6 DMD patients affected by different mutations (Table 1). Patient DL 515.2 carries an exon 45-50 deletion; hence exon 51 skipping would be frame correcting. Patient DL 363.2 has a deletion of exon 45-54; the reading frame for this patient would be corrected by an exon 44 skip. For patient 50685.1, who is affected by an exon 48-50 deletion, reading frame correction requires an exon 51 skip. Patient DL 589.2 has an exon 51-55 deletion; the reading frame would be corrected by an exon 50 skip. Patient 53914.1 carries a single exon 52 deletion. Notably, in this case both the skipping of exon 51 or exon 53 would be frame correcting. Finally, patient 50423.1 has a deletion of a single base pair in exon 49, at position 7389 on cDNA level, resulting in a frame-shift and a premature stop codon in exon 49. Since exon 49 is an in-frame exon, skipping of this exon would correct the reading frame for this patient.

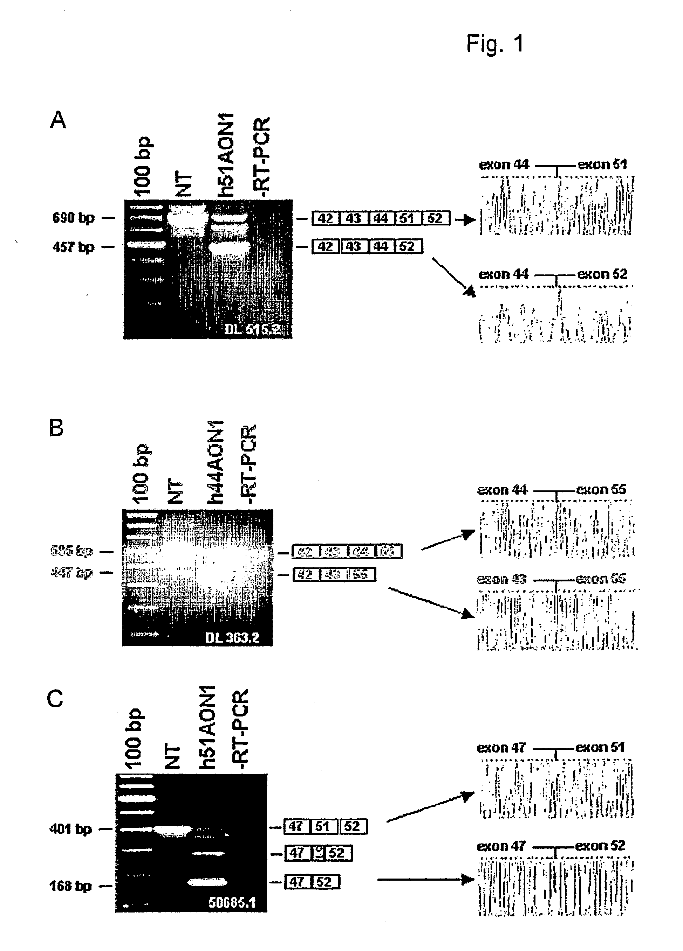

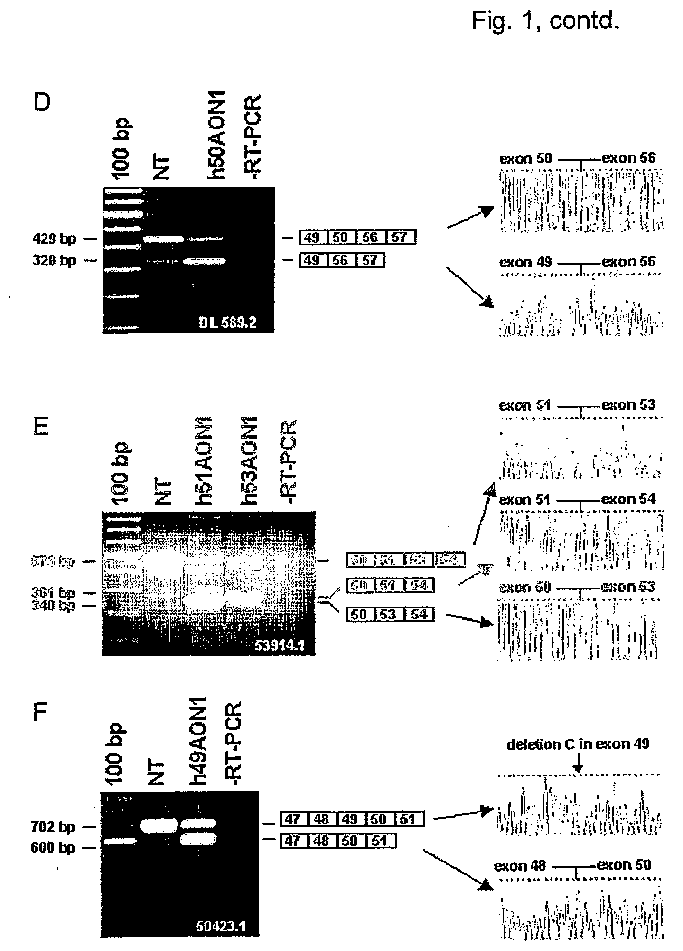

We have previously identified AONs with which the skipping of the mentioned target exons 44, 49, 50, 51 and 53 can be induced at concentrations of 1 µM (23). In subsequent dose-response experiments, however, we have obtained substantial skipping efficiencies with lower concentrations of 500 nM or 200 nM, and even 100 nM for most AONs (data not shown). This had the extra advantageous effect of lower doses of PEI required for transfection, which significantly reduced the levels of cytotoxicity as found in our earlier transfection experiments. Myotube cultures from the 6 DMD patients were transfected with the relevant AONs. On average 70% to 90% of cells showed specific nuclear uptake of fluorescent AONs. RNA was isolated 24 hours post-transfection and analysed by RT-PCR (Fig. 1). In all patients, the targeted exons were skipped at high efficiencies, and precisely at the exon boundaries, as confirmed by sequence analysis of the novel shorter transcripts (Fig. 1). For patient 50685.1, an additional transcript fragment was found (Fig. 1C). Sequence analysis showed that this was generated by the activation of a cryptic splice site in exon 51. This was previously also observed in human control cells treated with the same AON (23). Remarkably, low levels of spontaneous exon skipping were observed in untreated cells derived from patients DL 363.2 (exon 44 skip), DL 589.2 (exon 50 skip), and 53914.1 (exon 53 skip). RT-PCR analysis on several larger areas of the DMD gene transcript did not reveal additional, unexpected, aberrant splicing patterns induced by the AON-treatment.

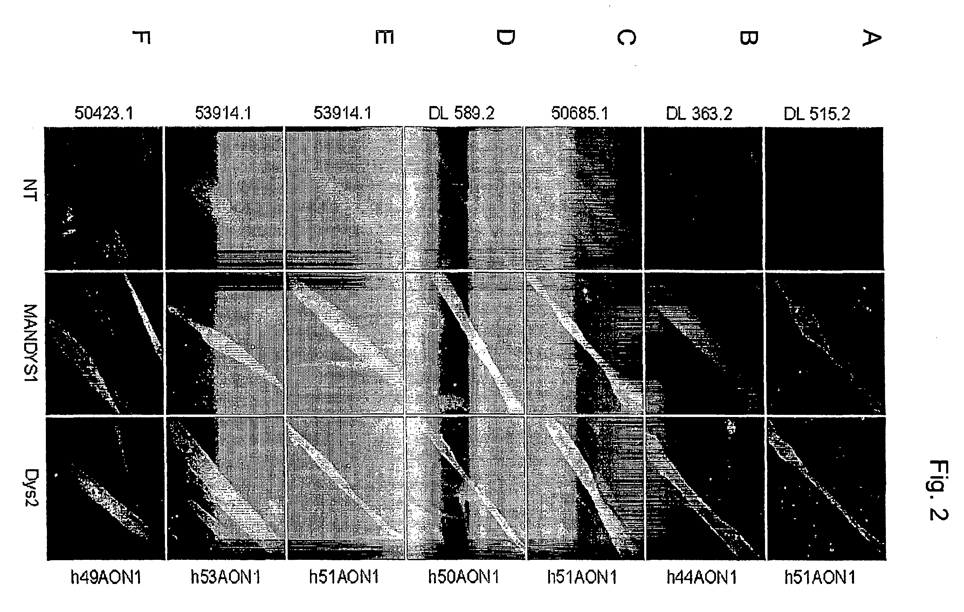

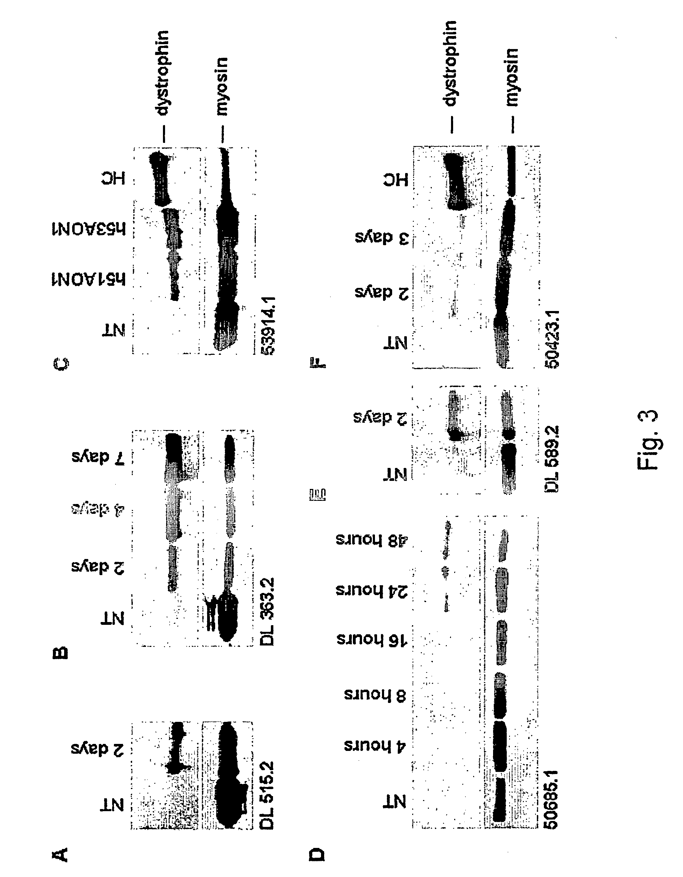

The resulting in-frame transcripts should restore dystrophin synthesis. Indeed, immuno-histochemical analysis of transfected myotube cultures detected dystrophin in the majority of myotubes for each patient (Fig. 2). The therapeutic efficiency was determined by double staining, using antibodies against myosin, to identify sufficiently differentiated myotubes, and dystrophin. On average, 75% to 80% of myosin-positive myotubes showed dystrophin expression. We observed clear membrane-bound dystrophin for patients DL 363.2, DL 589.2 and 53914.1 two days post-transfection (Fig. 2B, D, E). The presence of dystrophin was confirmed for each patient by Western blot analysis (Fig. 3). For patients 50685.1 and DL 363.2 we performed time course experiments, which indicated that dystrophin can be detected as soon as 16 hours post-transfection (Fig. 3D) and at increasing levels up to 7 days post-transfection (Fig. 3B). The dystrophin proteins from patients DL515.2, DL 363.2 and DL 589.2 are significantly shorter than the human control, which is due to the size of the deletion.

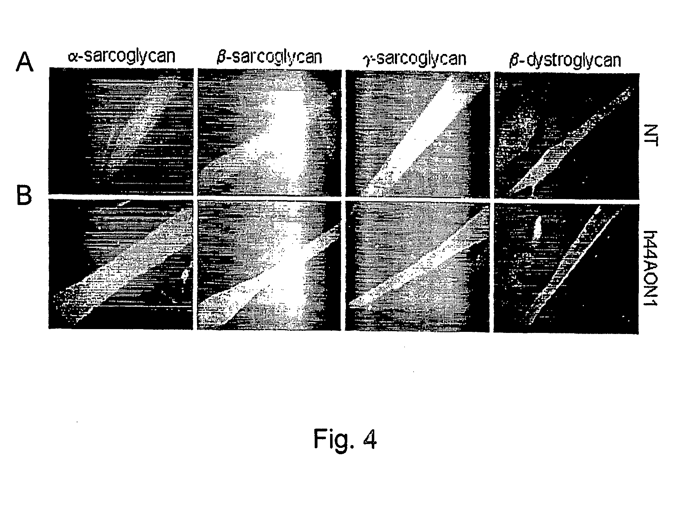

For one patient, DL 363.2, we also assessed whether the induction of the dystrophin synthesis resulted in the restoration of the DGC (Fig. 4). Prior to AON treatment we found reduced, mainly cytoplasmatic alpha, beta, gamma sarcoglycan and beta-dystroglycan signals (30%, 30%, 40% and 80%, respectively) (Fig. 4A). Following AON transfection, increased levels of mainly membrane-bound alpha-, beta- and gamma-sarcoglycans and beta-dystroglycan were detected in 70%, 90%, 90% and 80% of the treated myotube cultures, respectively (Fig. 4B).

### DISCUSSION

The reading frame correction strategy for DMD patients is aimed at antisense-induced, targeted exon skipping. This would convert a severe DMD phenotype into a mostly milder BMD phenotype. We determined the broad applicability in 6 patients, carrying 5 different deletions and a point mutation in an exon 49 (Table 1). Following AON treatment, we show for each patient the precise skipping of the targeted exon on RNA level, and a dystrophin protein in 75% to 80% of the treated myotubes. In particular, we here report, for the first time, the application of a single AON treatment (i.e. the induced skipping of exon 51) to correct the reading frame for several different deletions.

Interestingly, the levels of exon skipping observed in the DMD patient cells are significantly higher than those previously obtained in human control cells (23). Typically, the novel skip transcript is the major product. This can be explained by the action of the nonsense-mediated decay (NMD) process (25, 32). In control cells, the skip of an out-of-frame exon results in an out-of-frame transcript, which will be susceptible to NMD. In patient cells, the skip of a target exon results in an in-frame transcript that would be resistant to NMD and thus more stable than the out-of-frame transcript originally present.

For three of the patients (DL 363.2, DL 589.2 and 53914.1) we detected low levels of spontaneous skipping of exons 44, 50 and 53 in untreated cells. This phenomenon has previously also been described for so-called revertant muscle fibers (33-35). These dystrophin positive fibers are present in low amounts (2% to 10%) in DMD muscles, and are considered to be the result of secondary somatic mutations and/or alternative splicing that restore the reading frame. The existence of revertant fibers has been suggested to correlate with the severity of the disease (36, 37).

Restoration of the dystrophin synthesis could be detected as soon as 16 hours post-transfection. At two days post-transfection, dystrophin was detected at the membrane indicating that these novel BMD-like proteins are likely in part functional. Furthermore, we show that restoration of the dystrophin synthesis appears to re-establish the formation of the dystrophin-glycoprotein complex.

In patients DL 363.2 and DL 589.2, the targeted exon skipping enlarged the deletions to span exons 44-54 and 50-55, respectively. So far, these deletions, have not been reported in DMD or BMD patients. This means that they either do not exist, or generate a very mild phenotype not diagnosed as BMD. Considering both the large variety of BMD mutations and the markedly lower incidence of BMD observed, we consider the last explanation more plausible than the first. The out-of-frame deletions from patients DL 515.1, 50685.1 and 50423.1 were converted into in-frame deletions as observed in BMD patients carrying deletions of exon 45-51, exon 48-51 and exon 49 (30, 38-40). Noteworthy, the exon 48-51 deletion has even been described in an asymptomatic person (40). On the other hand however, there are also DMD patients carrying such deletions (38, 41-43). Since most of these theoretically in-frame deletions have been detected on DNA level only, we hypothesize that the dystrophin deficiency in these DMD patients may be caused by additional aberrant splicing patterns on RNA level, resulting in an out-of-frame transcript.

It is feasible to correct over 75% of the mutations reported in the Leiden DMD-mutation database (30). Our results indicate that, antisense-induced reading frame correction will be a promising therapeutic approach for many DMD patients carrying different deletions and point mutations. Towards the establishment of clinical trials, we are currently investigating and optimising delivery methods in muscle tissue of mice in vivo.

### MATERIAL AND METHODS

### AONs and primers

The AONs applied (Table 1) were previously described (23). They contain a 5' fluorescein group (6-FAM), a full-length phosphorothioate backbone and 2'-O-methyl modified ribose molecules (Eurogentec, Belgium). To avoid interference with the fluorescent signals of the secondary antibodies, unlabelled AONs were used for immuno-histochemical analyses. Primers for RT-PCR analysis (sequences available upon request) were synthesised by Eurogentec (Belgium) or by Isogen Bioscience BV (The Netherlands).

### Myogenic cell cultures and AON transfections

Primary human myoblasts from patients DL 515.2 (deletion exon 45-50), DL 363.2 (deletion exon 45-54), 50685.1 (deletion exon 48-50), DL 589.2 (deletion exon 51-55) and 53914.1 (deletion exon 52) were isolated from a muscle biopsy and cultured as described (44). Cultures were seeded in collagen pre-coated flasks and plates (Vitrogen 100, Cohesion). Myotubes were obtained from confluent myoblast cultures, following 7 to 14 days of serum-deprivation. They were subsequently transfected using polyethylenimine (PEI) for 3 h in low-serum medium, according to the manufacturer's instructions (ExGen500; MBI Fermentas), and with 3.5 µl PEI applied per µg of transfected AON. For RT-PCR analysis, concentrations of 500 nM AON were used. At this concentration highest skipping levels can be obtained, albeit with moderate levels of cell death. Since for immunohistochemical and western blot analysis more viable myotubes are required, concentrations of 200 nM were applied.

For patient 50423.1, who carries a point mutation in exon 49, only fibroblasts were available. Following infection (MOI 50-100) with an adenoviral vector containing the MyoD gene (Ad50MyoD), the fibroblasts were forced into myogenesis according to protocols described previously (45-47). Two hours post-infection the medium was replaced by low serum medium, and cells were incubated for 8 to 10 days until myotubes were formed. Transfection conditions were identical to those described above.

### RNA isolation and RT-PCR analysis

At 24 h post-transfection, total RNA was isolated from the myotube cultures (RNA-Bee RNA isolation solvent, Campro Scientific, The Netherlands). 300 ng of total RNA was used for RT-PCR analysis using C.therm polymerase (Roche Diagnostics, The Netherlands) in a 20 µl reaction at 60 °C for 30 min, primed with different DMD gene-specific reverse primers (Table 1). Primary PCRs were performed by 20 cycles of 94 °C (40 sec), 60 °C (40 sec) and 72 °C (60 sec). One µl of these reactions was then reamplified in nested PCRs by 32 cycles of 94 °C (40 sec), 60 °C (40 sec) and 72 °C (60 sec). PCR products were analysed on 1.5% or 2% agarose gels. Noteworthy, no evidence for a significant preference for the amplification of shorter fragments was obtained in PCR analyses on a defined series of mixtures of known quantities of the normal and shorter transcript fragments (data not shown).

### Sequence Analysis

RT-PCR products were isolated from agarose gels using the QIAquick Gel Extraction Kit (Qiagen). Direct DNA sequencing was carried out by the Leiden Genome Technology Center (LGTC) using the BigDye Terminator Cycle Sequencing Ready Reaction kit (PE Applied Biosystems) and analysed on an ABI 3700 Sequencer (PE Applied Biosystems).

### Protein isolation and Western blot analysis

Protein extracts were isolated from treated myotube cultures (25 cm2 flasks), using 150 µl of treatment buffer (75 mM Tris-HCl pH 6.8, 15% SDS, 5% b-mercaptoethanol, 2% glycerol, 0.001% bromophenol blue), at 2 to 4 days post-transfection depending on the survival rate of the myotubes. For the time course experiments, protein extracts were isolated 4h, 8h, 16h, 24h and 48h post-transfection (for patient 50685.1) or at 2 days, 4 days and 7 days post-transfection (for patient DL 363.2).

Polyacrylamide gel electrophoresis and Western blotting were performed as described by Anderson et al, with some minor adjustments (48). Briefly, samples (75 µl) were run overnight at 4 °C on a 4% to 7% polyacrylamide gradient gel. Gels were blotted to nitrocellulose for 5 to 6 hours at 4 °C. Blots were blocked for 1 h with 5% non-fat dried milk in TBST buffer (10 mM Tris-HCl, 0.15 M NaCl, 0.5% Tween 20, pH 8) followed by an overnight incubation with NCL-DYS2 (which recognizes dystrophin) diluted 1:50. HRP-conjugated anti-mouse (Santa Cruz) diluted 1: 10,000 was used as a secondary antibody. Immuno-reactive bands were visualised using Lumi-Lightplus Western Blotting Substrate and scanned with a Lumi-Imager (Roche Diagnostics, The Netherlands).

### Immuno-histochemical Analysis

Treated myotube cultures were fixed in -20°C methanol at 1 to 4 days post-transfection, depending of the survival rate of the myotubes. Prior to reaction with the different antibodies, the cells were incubated for 1 h in a blocking solution containing 5% horse serum (Gibco BRL) and 0.05 % Tween-20 (Sigma) in PBS (Gibco BRL). All antibodies used were diluted in this blocking solution. The following antibodies were applied: desmin polyclonal antibody (ICN Biomedicals) diluted 1:100, myosin monoclonal antibody diluted 1:100 (MF20; Developmental Studies Hybridoma Bank, University of Iowa), myosin polyclonal antibody L53 diluted 1:100 (a gift from Dr M. van den Hoff, AMC, The Netherlands), MANDYS1 (a gift from Dr G. Morris, North East Wales Institute, UK) diluted 1:10 and NCL-DYS2 (Novacastra Laboratories Ltd) diluted 1:10 to detect dystrophin, NCL-a-SARC (Novacastra Laboratories Ltd) diluted 1:75, NCL-b-SARC (Novacastra Laboratories Ltd) diluted 1:50, NCL-g-SARC (Novacastra Laboratories Ltd) diluted 1:50 and NCL-b-DG (Novacastra Laboratories Ltd) diluted 1:50 to detect α-sarcoglycan, β-sarcoglycan, γ-sarcoglycan and β-dystroglycan, respectively. After 1 h incubation, slides were rinsed and incubated for 1 h with the secondary antibodies Alexa Fluor 594 goat anti-rabbit conjugate diluted 1:1000 or Alexa Fluor 488 goat anti-mouse conjugate diluted 1:250 (Molecular Probes Inc). The slides were analysed using a Leica confocal microscope equipped with epifluorescence optics. Digital images were captured using a CCD camera (Photometrics).

### Example 2

### Materials and methods

### AONs and primers

A series of AONs (two per exon, see Table 2) was designed to bind to exon-internal target sequences showing a relatively high purine-content and, preferably, an open secondary pre-mRNA structure (at 37 °C), as predicted by the RNA mfold version 3.1 server [22]. The AONs varied in length between 15 and 24 bp, with G/C contents between 26 and 67%. They were synthesized with the following chemical modifications: a 5'-fluorescein group (6-FAM), a full-length phosphorothioate backbone and 2'-O-methyl modified ribose molecules (Eurogentec, Belgium). The primers used for reverse transcription-polymerase chain reaction (RT-PCR) analysis (Table 3) were synthesized by Eurogentec (Belgium) or by Isogen Bioscience BV (The Netherlands).

### In vitro experiments

Primary human myoblasts were isolated from a muscle biopsy from a non-affected individual (KM108) by enzymatic dissociation. Briefly, the tissue was homogenized in a solution containing 5 mg/ml collagenase type VIII (Sigma), 5 mg/ml bovine albumin fraction V (Sigma), 1% trypsin (Gibco BRL) in PBS (Gibco BRL). Following serial incubation steps of 15 min at 37 °C, suspensions containing the dissociated cells were added to, and pooled in, an equal volume of proliferation medium (Nut.Mix F-10 (HAM) with GlutaMax-1, Gibco BRL) supplemented with 20% fetal bovine serum (Gibco BRL) and 1% penicillin/streptomycin solution (Gibco BRL). After centrifugation, the cells were plated and further cultured in proliferation medium, using flasks that were pre-coated with purified bovine dermal collagen (Vitrogen 100; Cohesion). The myogenic cell content of the culture, as determined by the percentage of desmin-positive cells in an immunohistochemical assay, was improved to 58% by repetitive preplating [23]. Myotubes were obtained from confluent myoblast cultures following 7-14 days of incubation in low-serum medium (DMEM (Gibco BRL), supplemented with 2% GlutaMax-1,1% glucose, 2% fetal bovine serum and 1% penicillin/streptomycin solution). For transfection of the myotube cultures, we used polyethylenimine (PEI; ExGen 500) according to the manufacturer's instructions (MBI Fermentas). The cultures were transfected for 3 h in low-serum medium with 1 mM of each AON linked to PEI at a ratio-equivalent of 3.5.

RNA isolation and RT-PCR analysis At 24 h post-transfection, total RNA was isolated from the myotube cultures using RNAzol B according to the manufacturer's instructions (Campro Scientific, The Netherlands). One microgram of RNA was then used for RT-PCR analysis using C. therm polymerase (Roche Diagnostics) in a 20 µl reaction at 60 °C for 30 min, primed with different DMD gene-specific reverse (RT) primers (Table 3). Primary PCRs were carried out with outer primer sets (see Table 3), for 20 cycles of 94 °C (40 s), 60 °C (40 s), and 72 °C (90 s). One microliter of this reaction was then reamplified in nested PCRs using the appropriate primer combinations (Table 3) for 32 cycles of 94 °C (40 s), 60 °C (40 s), and 72 °C (60 s). PCR products were analysed on 1.5 or 2% agarose gels.

Sequence analysis RT-PCR products were isolated from agarose gels using the QIAquick Gel Extraction kit (Qiagen). Direct DNA sequencing was carried out by the Leiden Genome Technology Center (LGTC) using the BigDye Terminator Cycle Sequencing Ready Reaction kit (PE Applied Biosystems), and analysed on an ABI 3700 Sequencer (PE Applied Biosystems).

### Results

### In vitro exon skipping

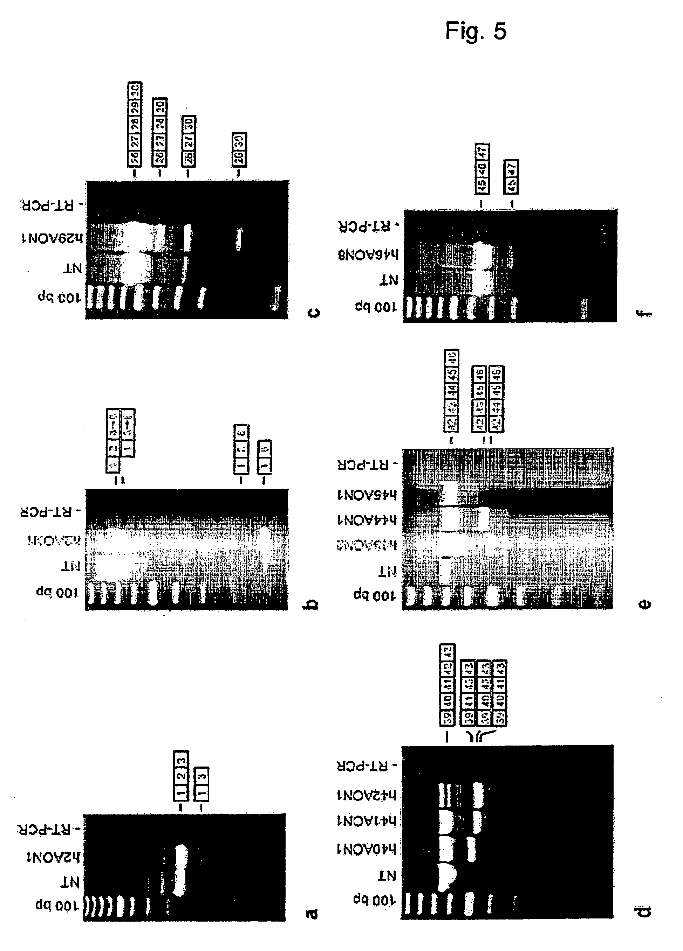

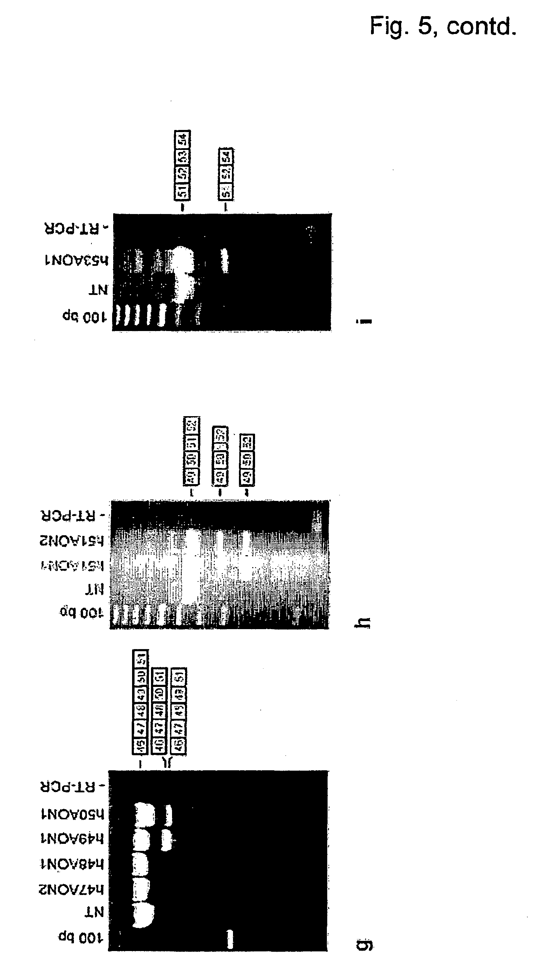

AONs were empirically analysed for the induction of exon skipping following transfection into human control myotube cultures, using the cationic polymer polyethylenimine (PEI). As determined by the nuclear uptake of the fluorescent AONs, average transfection efficiencies of 60-80% were obtained. At 24 h post-transfection, transcripts were analysed by RT-PCR using different primer combinations encompassing the targeted exons (Table 3). Of the 30 AONs tested, a total of 21 (70%) reproducibly generated shorter transcript fragments with sizes corresponding to the specific skipping of the targeted exons (Fig. 5 and Table 2). In fact, as confirmed by sequence analysis of the shorter transcripts (data not shown), we could induce the specific skipping of 13 out of the 15 exons targeted (five out of the seven in-frame exons, and eight out of the eight out-of-frame exons). No skipping of exons 47 and 48 was detected (Fig. 5e,g).

In the specific transcript regions that were screened in these experiments, we observed in the non-transfected control myotubes alternative splicing patterns around exons 2 and 29 (Fig. 5b,c). The alternative products were sequenced and found to be due to the skipping of exons 2-7 (in-frame), exons 3-7 (out-of-frame), exons 28-29 (in-frame), and exons 27-29 (in-frame). This genuinely occurring exon skipping was also detected previously in human skeletal muscle [24,25]. Remarkably, the level of the alternative splicing was significantly enhanced by the AON treatment of the transfected myotube cultures. Noteworthy also is the observation that h2AON1 not only induced exon 2 skipping in the normal transcript, but also in one of the alternative transcripts consisting of exons 1 and 2 spliced to exon 8 (Fig. 5b).

The majority of AONs induced the precise skipping of the targeted exons, using the original splice sites of the adjacent exons. However, in response to h51AON2, an in-frame cryptic splice site was used in exon 51 (Fig. 5h). The level of this alternatively spliced product was variable in serial transfection experiments. Finally, in some of the transfection experiments, additional aberrant splicing fragments were detected due to the co-skipping of adjacent exons. Their incidence, however, was inconsistent, and at very low levels.

References to example 2 (numbering in this part refers strictly to numbering maintained in example 2)
[1] Hoffman EP, Brown Jr RH, Kunkel LM. Dystrophin: the protein product of the Duchenne muscular dystrophy locus. Cell 1987;51:919-928.
[2] Monaco AP, Bertelson CJ, Liechti-Gallati S, Moser H, Kunkel LM. An explanation for the phenotypic differences between patients bearing partial deletions of the DMD locus. Genomics 1988;2:90-95.
[3] Koenig M, Beggs AH, Moyer M, et al. The molecular basis for Duchenne versus Becker muscular dystrophy: correlation of severity with type of deletion. Am J Hum Genet 1989;45:498-506.
[4] Zubrzycka-Gaarn EE, Bulman DE, Karpati G, et al. The Duchenne muscular dystrophy gene product is localized in sarcolemma of human skeletal muscle. Nature 1988;333:466-469.
[5] Yoshida M, Ozawa E. Glycoprotein complex anchoring dystrophin to sarcolemma. J Biochem (Tokyo) 1990;108:748-752.
[6] Ervasti JM, Campbell KP. Membrane organization of the dystrophinglycoprotein complex. Cell 1991;66:1121-1131.
[7] Koenig M, Monaco AP, Kunkel LM. The complete sequence of dystrophin predicts a rod-shaped cytoskeletal protein. Cell 1988;53:219-226.
[8] van Deutekom JC, Floyd SS, Booth DK, et al. Implications of maturation for viral gene delivery to skeletal muscle. Neuromuscul Disord 1998;8:135-148.
[9] Mayeda A, Hayase Y, Inoue H, Ohtsuka E, Ohshima Y. Surveying cis-acting sequences of pre-mRNA by adding antisense 20-O-methyl oligoribonucleotides to a splicing reaction. J Biochem (Tokyo) 1990;108:399-405.
[10] Galderisi U, Cascino A, Giordano A. Antisense oligonucleotides as therapeutic agents. J Cell Physiol 1999;181:251-257.
[11] Baker BF, Monia BP. Novel mechanisms for antisense-mediated regulation of gene expression. Biochim Biophys Acta 1999;1489:3-18.
[12] Kole R, Sazani P. Antisense effects in the cell nucleus: modification of splicing. Curr Opin Mol Ther 2001;3:229-234.
[13] Sicinski P, Geng Y, Ryder-Cook AS, Barnard EA, Darlison MG, Barnard PJ. The molecular basis of muscular dystrophy in the mdx mouse: a point mutation. Science 1989;244:1578-1580.
[14] Dunckley MG, Manoharan M, Villiet P, Eperon IC, Dickson G. Modification of splicing in the dystrophin gene in cultured Mdx muscle cells by antisense oligoribonucleotides. Hum Mol Genet 1998;7:1083-1090.
[15] Mann CJ, Honeyman K, Cheng AJ, et al. Antisense-induced exon skipping and synthesis of dystrophin in the mdx mouse. Proc Natl Acad Sci USA 2001;98:42-47.
[16] Wilton SD, Lloyd F, Carville K, et al. Specific removal of the nonsense mutation from the mdx dystrophin mRNA using anti-sense oligonucleotides. Neuromuscul Disord 1999;9:330-338.
[17] Takeshima Y, Wada H, Yagi M, et al. Oligonucleotides against a splicing enhancer sequence led to dystrophin production in muscle cells from a Duchenne muscular dystrophy patient. Brain Dev 2001;23:788-790.
[18] Pramono ZA, Takeshima Y, Alimsardjono H, Ishii A, Takeda S, Matsuo M. Induction of exon skipping of the dystrophin transcript in lymphoblastoid cells by transfecting an antisense oligodeoxynucleotide complementary to an exon recognition sequence. Biochem Biophys Res Commun 1996;226:445-449.
[19] Watakabe A, Tanaka K, Shimura Y. The role of exon sequences in splice site selection. Genes Dev 1993;7:407-418.
[20] Tanaka K, Watakabe A, Shimura Y. Polypurine sequences within a downstream exon function as a splicing enhancer. Mol Cell Biol 1994;14:1347-1354.
[21] van Deutekom JC, Bremmer-Bout M, Janson AA, et al. Antisenseinduced exon skipping restores dystrophin expression in DMD patient derived muscle cells. Hum Mol Genet 2001;10:1547-1554.
[22] Mathews DH, Sabina J, Zuker M, Turner DH. Expanded sequence dependence of thermodynamic parameters improves prediction of RNA secondary structure. J Mol Biol 1999;288:911-940.
[23] Richler C, Yaffe D. The in vitro cultivation and differentiation capacities of myogenic cell lines. Dev Biol 1970;23:1-22.
[24] Surono A, Takeshima Y, Wibawa T, Pramono ZA, Matsuo M. Six novel transcripts that remove a huge intron ranging from 250 to 800 kb are produced by alternative splicing of the 50 region of the dystrophin gene in human skeletal muscle. Biochem Biophys Res Commun 1997;239:895-899.
[25] Shiga N, Takeshima Y, Sakamoto H, et al. Disruption of the splicing enhancer sequence within exon 27 of the dystrophin gene by a nonsense mutation induces partial skipping of the exon and is responsible for Becker muscular dystrophy. J Clin Invest 1997;100:2204-2210.
[26] Wells DJ, Wells KE, Asante EA, et al. Expression of human fulllength and minidystrophin in transgenic mdx mice: implications for gene therapy of Duchenne muscular dystrophy. Hum Mol Genet 1995;4:1245-1250.
[27] Sironi M, Pozzoli U, Cagliani R, Comi GP, Bardoni A, Bresolin N. Analysis of splicing parameters in the dystrophin gene: relevance for physiological and pathogenetic splicing mechanisms. Hum Genet 2001;109:73-84.
   A. Aartsma-Rus et al. / Neuromuscular Disorders 12 (2002) S71-S77.

### Example 3

### RESULTS

### Double-exon skipping in two DMD patients

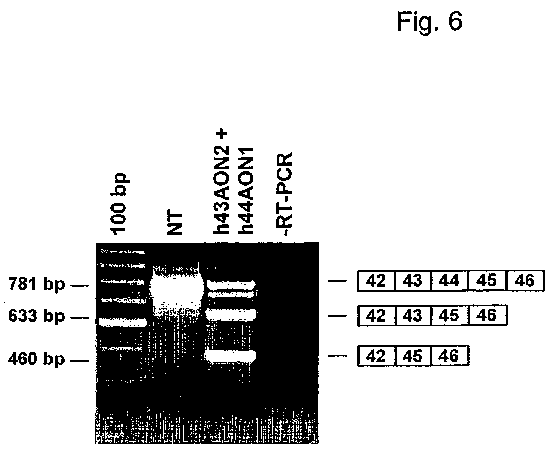

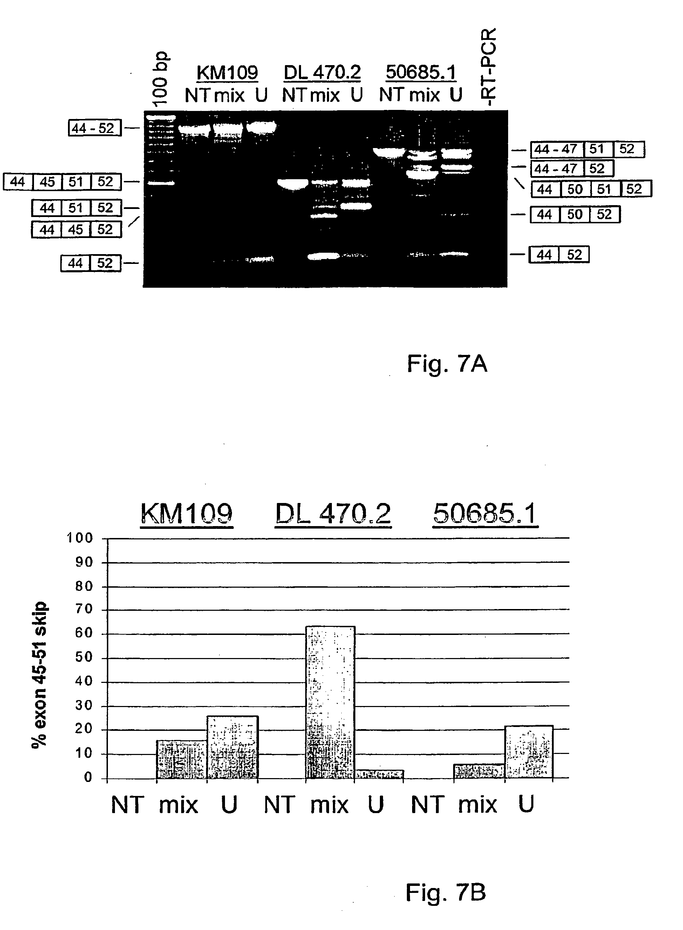

This study includes two DMD patients affected by different frame-disrupting mutations in the DMD gene that require the skip of two exons for correction of the reading frame (Table 5). Patient DL 90.3 carries a nonsense mutation in exon 43. Considering that this single exon is out-of-frame, the skipping of exon 43 would remove the nonsense mutation but not restore the reading frame. Since the combination with exon 44 is in-frame, we aimed in this patient at double-exon skipping, targeting both these exons. Patient DL 470.2 is affected by a deletion of exons 46 to 50. Frame restoration would require a double-exon skipping of both exons flanking the deletion. Myotubes cultures from both patients were transfected with a mixture of exon 43 and 44 specific AONs (DL90.3) or exon 45 and 51 specific AONs (DL470.2). The individual AONs (Table 5) were previously highly effective in single exon skipping. Transfection efficiencies were typically over 80%, as indicated by the number of cells with specific nuclear uptake of the fluorescent AONs. RT-PCR analysis at 24 to 48 hours post-transfection, indeed demonstrated the feasibility of specific double-exon skipping in both samples (Fig 6 and 7). This was confirmed by sequence analysis (data not shown). Additional shorter transcript fragments were obtained due to single-exon skipping: in patient DL 90.3 exon 44 skipping (Fig.6), and in patient DL470.2 exon 51 skipping (Fig.7).

### Multi-exon skipping

The splicing of exon 44 directly to exon 52 (as induced in DL470.2) generates an in-frame transcript. We hypothesized that by inducing the skipping of the entire stretch of exons in between, i.e. multi-exon skipping, we would induce a BMD-like deletion (45-51) that covers and restores several known, smaller, DMD mutations. This would further enlarge the group of DMD patients that would benefit from one type of frame correction. The feasibility of multi-exon skipping was first shown in human control myotubes that were treated with a mixture of the exon 45 and 51 specific AONs (Fig. 7; KM 109). We then applied it to myotubes from a third DMD patient carrying an exon 48-50 deletion (50685.1). By the AON-induced skipping of the (remaining) stretch of exons in between, and including, exons 45 and 51, we obtained the anticipated smaller in-frame transcript with exon 44 spliced to exon 52 (Fig.7).

### Double- and multi-exon skipping using a U-linked AON-combination

The skipping of more than one exon from one pre-mRNA molecule requires that both AONs are present in the same nucleus, targeting the same molecule. To enlarge this chance, we here studied the feasibility of one combined AON carrying both AONs specific for exons 45 and 51 (h45AON5 and h51AON2) linked by 10 uracil nucleotides (Table 5). Following transfection of this "U-linker AON" into myotubes from human control, and the DMD patients DL470.2 and 50685.1, RT-PCR analysis demonstrated its efficacy to generate the anticipated in-frame transcript with exon 44 spliced to exon 52 (Fig.7). This multi-exon skipping occurred specifically and precisely at the exon-boundaries as confirmed by sequence analysis (data not shown). In contrast to patient DL 470.2, the U-linker AON was a slightly more efficient than the mixture of AONs in the human control and in patient 50685.1.

### MATERIAL AND METHODS

### AONs and primers

AONs (Table 5) targeting exons 43, 44 and 51 were previously described [Aartsma-Rus, 2002]. AONs targeting exon 45 were newly designed (sequences upon request). All AONs contain a 5' fluorescein group (6-FAM), a full-length phosphorothioate backbone and 2'-O-methyl modified ribose molecules (Eurogentec, Belgium). To avoid interference with the fluorescent signals of the secondary antibodies, unlabelled AONs were used for immuno-histochemical analyses. Primers for RT-PCR analysis (Table 5, sequences available upon request) were synthesised by Eurogentec (Belgium).

### RNA isolation and RT-PCR analysis

At 24 to 48 h post-transfection, total RNA was isolated from the myotube cultures (RNA-Bee RNA isolation solvent, Campro Scientific, The Netherlands). 300 ng of total RNA were used for RT-PCR analysis using C.therm polymerase (Roche Diagnostics, The Netherlands) in a 20 µl reaction at 60 °C for 30 min, primed with different DMD gene-specific reverse primers (Table 5). Primary PCRs were performed by 20 cycles of 94 °C (40 sec), 60 °C (40 sec) and 72 °C (60 sec). One µl of these reactions was then re-amplified in nested PCRs by 32 cycles of 94 °C (40 sec), 60 °C (40 sec) and 72 °C (60 sec). PCR products were analysed on 1.5% or 2% agarose gels. For quantification of the transcript products, nested PCR's were performed using 24 cycles. PCR products were analysed using the DNA 7500 LabChip® Kit and the Agilent 2100 bioanalyzer (Agilent Technologies, The Netherlands).

### Sequence Analysis

RT-PCR products were isolated from agarose gels using the QIAquick Gel Extraction Kit (Qiagen). Direct DNA sequencing was carried out by the Leiden Genome Technology Center (LGTC) using the BigDye Terminator Cycle Sequencing Ready Reaction kit (PE Applied Biosystems) and analysed on an ABI 3700 Sequencer (PE Applied Biosystems).

### Example 4.

### Expression vectors encoding a transcript comprising an oligonucleotide of the invention.

Due to the defined turnover rate of both the dystrophin pre-mRNA and the AONs, our DMD frame-correction therapy would require repetitive administrations of AONs. In addition, relatively high levels of antisense RNA will be necessary within the nucleus, where transcription and splicing of the dystrophin pre-mRNA occur. Therefore, we have set up a vector system in which specific AON sequences are incorporated into a modified gene. In this example this embodiment is described for U7 small nuclear RNA (U7snRNA). U7snRNA is the RNA component of the U7 ribonucleoprotein particle (U7snRNP) that is involved in the processing of the 3' end of histone pre-mRNAs. Inherent to its function, U7snRNA is efficiently transported back from the cytoplasm to the nucleus in which it gets subsequently incorporated into very stable U7snRNP complexes. A similar approach was successfully applied in AON-based gene therapy studies on ß-thalassemia (53, 54). In these studies, different plasmids were engineered containing a modified U7snRNA gene from which the natural antisense sequence directed to the histone pre-mRNA was replaced with antisense sequences targeted to different ß-thalassemia-associated aberrant splicing sites in the ß-globin gene. Following transfection of these plasmids, correct splicing and expression of the full-length ß-globin protein could be restored with an efficiency of up to 65% in cultured cells expressing the different mutant ß-globin genes.

We have engineered various U7snRNA gene constructs as described in reference 53 with the modification that the ß-globin sequences were exactly replaced by the antisense sequences derived from the different AONs. In this example, the sequences were replaced by the antisense sequences of m46AON4, 6, 9, or 11 that were effective in inducing the skipping of mouse exon 46. A sense construct was included as negative control (m46SON6). Following construct validation by sequencing, the plasmids were tested in vitro by transfection into cultured C2C12 mouse myoblasts. The U7snRNA-m46AON6 construct was most efficient.

To enhance delivery of the AON-U7snRNA gene constructs, we have cloned them into recombinant adeno-associated viral (rAAV) vectors. AAV is a single-stranded DNA parvovirus that is non-pathogenic and shows a helper-dependent life cycle. In contrast to other viruses (adenovirus, retrovirus, and herpes simplex virus), rAAV vectors have demonstrated to be very efficient in transducing mature skeletal muscle. Whereas application of rAAV in classical DMD "gene addition" studies has been hindered by its restricted packaging limits (< 5 kb), we apply rAAV for the efficient delivery of the much smaller U7snRNA antisense constructs (< 600 bp) to mature murine skeletal muscle.

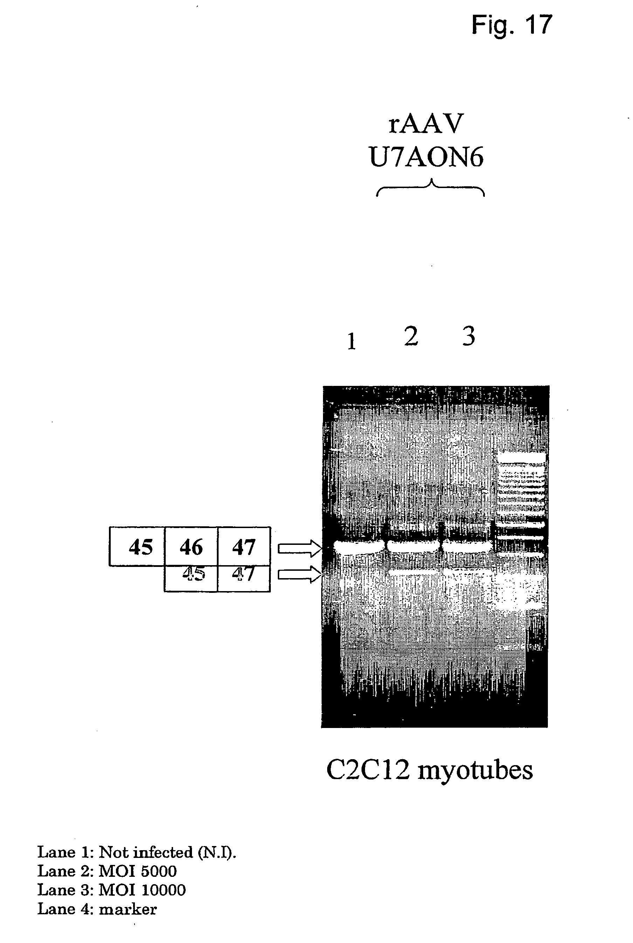

The U7-m46AON6 construct was most effective in inducing exon 46 skipping following transfection into mouse C2C12 myotubes, and was cloned as a NotI-fragment into a rAAV vector (Stratagene). This vector contains the gene for green fluorescence protein (GFP-cDNA) cloned in between AAV-derived ITR sequences. The GFP protein allows determination of transduction efficiencies in muscle, post-infection. We engineered various vectors, containing the U7-AON construct, either alone, or proximal and distal of the GFP gene. The vectors were validated for their insert content by sequence analysis. For rAAV virus production, we co-transfected 293 cells with the U7-AON-rAAV plasmids in combination with the packaging/helper plasmid, pDP-5. This is an improved helper plasmid, containing all AAV and adenoviral sequences required for amplification and packaging of AAV vectors. The pDP-5 plasmid, obtained from Dr. J. Kleinschmidt (dkfz Heidelberg, Germany), facilitates the production of high titers up to 10¹² viral particles/ml of infectious rAAV, and without contamination of wild type AAV or adenovirus. Following purification and concentration, we subsequently tested two constructs, rAAV-U7m46AON6 and rAAV-GFP-U7m46AON6, by infecting C2C12 mouse myotubes at two different MOIs (Multiplicity Of Infection). At 7 days post-infection, RNA was isolated and RT-PCR analysis performed (as described in Material and Methods). The rAAV-m46AON6 construct was most efficient and induced exon 46 skipping at levels of ∼20% (see Figure 17).

### Example 5

### Title:

### Double and multi-exon skipping.

Using a combination of AONs, double skipping of exon 43 and 44 was induced, and dystrophin synthesis was restored in myotubes from one patient affected by a nonsense mutation in exon 43. For another patient, with an exon 46-50 deletion, the therapeutic double skipping of exon 45 and 51 was achieved. Remarkably, in control myotubes, the latter combination of AONs caused the skipping of the entire stretch of exons from 45 through 51. This in-frame multi-exon skipping would be therapeutic for a series of patients carrying different DMD-causing mutations. In fact, we here demonstrate its feasibility in myotubes from a patient with an exon 48-50 deletion. The application of multi-exon skipping provides a more uniform methodology for a larger group of patients with DMD.

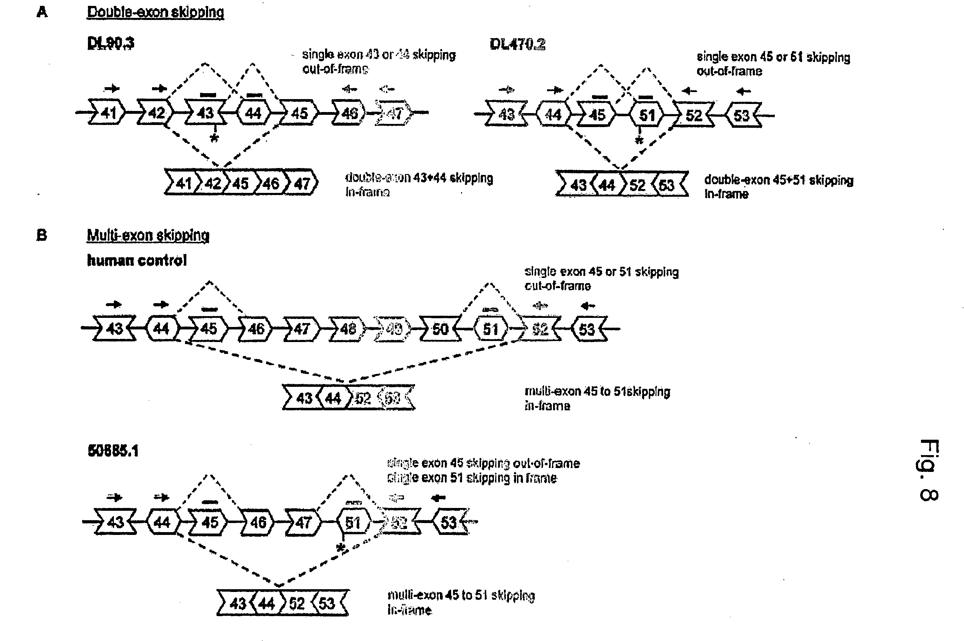

(AONs) have recently become an attractive tool for the study and treatment of human disease. Initially, AONs were used for the sequence-specific inhibition of genes, either to elucidate developmental processes or to suppress malignant or aberrant gene expression (Dennis et al. 1998; Stevenson et al. 1999; Nasevicius and Ekker 2000; Corey and Abrams2001; Dove 2002). In these studies, AONs mediated RNAse H degradation of dsRNA, or they blocked transcription or translation initiation. However, AONs are also capable of modulating the splicing of pre-mRNA(Sierakowska et al. 1996). Since it has been estimated that at least 15% of disease-causing point mutations resulting RNA splicing defects (Krawczak et al. 1992; Cartegniet al. 2002; Buratti et al. 2003), this latter application may be highly relevant for future genetic therapies. For instance, RNAase H-resistant AONs have successfully been used to induce the skipping of pseudo-exons by blocking cryptic splice sites in the b-globin gene (Sierakowska et al. 1996) and the *cystic fibrosis transmembrane conductance regulator* gene (Friedman et al. 1999). Alternatively, AONs linked to 10 arginine-serine dipeptide repeats for the artificial recruitment of splicing enhancer factors have been applied in vitro to induce inclusion of mutated *BRCA1* and *SMN2* exons that otherwise would be skipped (Cartegni and Krainer 2003). AONs have also been effective in altering the ratio of alternative splicing, which was applied for cancer-related genes to direct malignant toward nonmalignant isoforms (Mercatanteet al. 2001, 2002). Last, but not least, a promising, recently developed application of AONs is to induce the specific skipping of exons in order to correct the reading frame of a mutated transcript so that it can be translated into a partially functional protein. The *DMD* gene, which codes for dystrophin, is well suited for this latter application. The protein consists of an N-terminal domain that binds to actin filaments, a central rod domain, and a C-terminal cysteine-rich domain that binds to the dystrophin-glycoprotein complex (Hoffman et al. 1987; Koenig et al. 1988; Yoshida and Ozawa1990). Mutations in the *DMD* gene that interrupt the reading frame result in a complete loss of dystrophin function, which causes the severe Duchenne muscular dystrophy (DMD [MIM 310200]) (Hoffman et al. 1988;Koenig et al. 1989; Ervasti et al. 1990). The milder Becker muscular dystrophy (BMD [MIM 300376]), on the other hand, is the result of mutations in the same gene that are not frameshifting and result in an internally deleted but partially functional dystrophin that has retained its N- and C-terminal ends (Koenig et al. 1989;Di Blasi et al. 1996). Over two-thirds of patients with DMD and BMD have a deletion of one or more exons (den Dunnen et al. 1989). Remarkably, patients have been described who exhibit very mild BMD and who lack up to 67% of the central rod domain (England et al. 1990;Winnard et al. 1993; Mirabella et al. 1998). This suggests that, despite large deletions, a partially functional dystrophin can be generated, provided that the deletions render the transcript in frame. AONs to alter splicing so that the open reading frame is restored and the severe DMD phenotype is converted into a milder BMD phenotype. Several studies have shown therapeutic AON-induced single-exon skipping in cells derived from the *mdx* mouse model (Dunckley et al. 1998;Wilton et al. 1999; Mann et al. 2001, 2002;Lu et al. 2003) and various DMD patients (Takeshima et al. 2001; van Deutekom et al. 2001; Aartsma-Rus et al. 2002, 2003; De Angelis et al. 2002). To date, we have identified a series of AONs that can be used to induce the skipping of 20 different exons (exons 2, 8,17, 19, 29, 40-46, 48-53, 55, and 59) (Aartsma-Ruset al. 2002). Of all patients with DMD, more than 75% would benefit from the skipping of these exons. So far, we have successfully applied single-exon skipping in cells derived from eight different patients with DMD (van Deutekomet al. 2001; Aartsma-Rus et al. 2003). In these studies, the skipping of exons flanking out-of-frame deletions or an in-frame exon containing a nonsense mutation restored the reading frame and induced the synthesis of BMD-like dystrophins in approximately 75%-80% of treated cells. These novel dystrophins could be detected as early as 16 h after transfection; the dystrophins increased to significant levels within 4 d and were maintained for at least 7 d (Aartsma-Rus et al. 2003). Here, we significantly extend the therapeutic applicability of this technique by demonstrating double and multi-exon skipping (fig. 8). Following the simultaneous skipping of two exons (double-exon skipping), the reading frame was restored for one patient with a nonsense mutation in an out-of-frame exon and for another patient with a deletion that could not be bypassed by the skipping of only one exon. Furthermore, through the skipping of an entire stretch of consecutive exons (multi-exon skipping), a BMD-like deletion was generated with the potential to restore up to 14% of all known *DMD* mutations.

### Material and Methods

### AONs and Primers

Exon-internal AONs targeting exons 44 (h44AON1) and 51 (h51AON2) were described elsewhere (Aartsma-Rus et al. 2002). The AONs targeting exons 43 and 45 were specifically designed for this study (h43AON5:CUGUAGCUUCACCCUUUCC; h45AON5: GCCCAAUGCCAUCCUGG).BLAST analysis of the AONs did not reveal perfect homology to other sequences in the human genome (maximum homology 94%, minimum *E* value 0.3). All AONs contain a 5'- fluorescein group (6-FAM), a full-length phosphorothioate backbone, and 2'-*O*-methyl-modified ribose molecules (Eurogentec). To avoid interference with the fluorescent signals of the secondary antibodies, unlabeled AONs were used for immunohistochemical analyses. Primers for RT-PCR analysis (fig. 8) were synthesized by Eurogentec (sequences available upon request).

### Myogenic Cell Cultures and AOIV Transfections

Primacy myoblasts from a human control and from two patients with DMD (DL470.2 [exon 46-50 deletion] and 50685.1 [exon 48-50 deletion]) were isolated from a muscle biopsy and cultured as described elsewhere (Aartsma-Rus et al. 2002). Myotubes were obtained from confluent myoblast cultures, after 7-14 d of serum deprivation. These were transfected with mixtures of 200 nM of each AON. Polyethylenemine (PEI) was used as transfection reagent, according to the manufacturer's instructions (ExGen 500 [Fermentas]). Separate AON-PEI dilutions were made for each AON, with 3.5 µl PEI applied per µg of transfected AON. For patient DL90.3, who has a point mutation in exon43, only fibroblasts were available. Following infection(multiplicity of infection [MOI] 50-100) with an adenoviral vector containing the *MyoD* gene (Ad50MyoD),the fibroblasts were forced into myogenesis, according to protocols described elsewhere (Murry et al. 1996;Roest et al. 1996; Aartsma-Rus et al. 2002; Havenga et al. 2002). Transfection conditions were identical to those described above.

### RNA Isolation and RT-PCR Analysis of the Skip Products

RNA isolation, RT-PCR, and sequence analysis were performed as described elsewhere (Aartsma-Rus et al.2002). See figure 8 for the location of the primers. For quantification of the skip products, nested PCRs were performed using 24 cycles. PCR products were analyzed using the DNA 1000 LabChip kit and the 2100 Bioanalyzer(Agilent Technologies).To analyze the splicing of the entire *DMD* gene, RTreactions were performed with 1 µg RNA, random hexamer primers, and SuperScript III (Invitrogen). PCR analyses were performed using the protein-truncation test (PTT) primers described elsewhere (Roest et al.1993). Since some of the PCR primers were located in exons that were deleted for patient DL470.2, we specifically designed additional primers in exons 41, 42, 45,53, and 54 (sequences available on request).

### Analysis of the Dystrophin Protein

Immunohistochemical and Western blot analyses were performed as described elsewhere (Aartsma-Rus et al.2002). Myosin polyclonal antibody L53 (a gift from Dr. M. van den Hoff, Amsterdam Medical Center, The Netherlands)was used to detect myosin. MANDYS1 (a gift from Dr. G. Morris, North East Wales Institute, United Kingdom), NCL-DYS2 (Novacastra Laboratories), and NCL-DYS1 (Novacastra Laboratories) were used to detect dystrophin. For the Western blot analysis, dystrophin levels were quantified using LumiAnalyst 3.0(Roche).

### Results

### Double-Exon Skipping in Two Patients with DMD

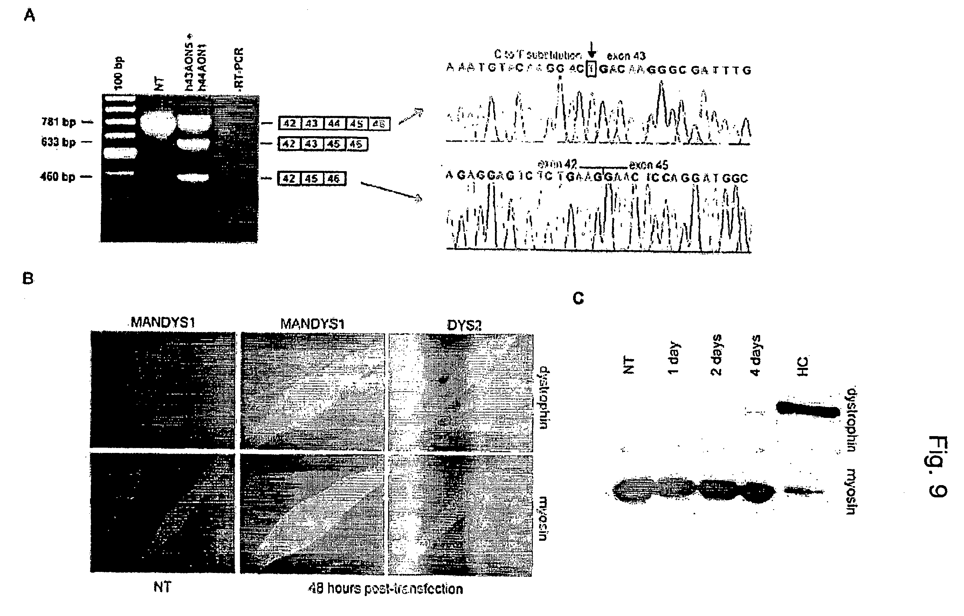

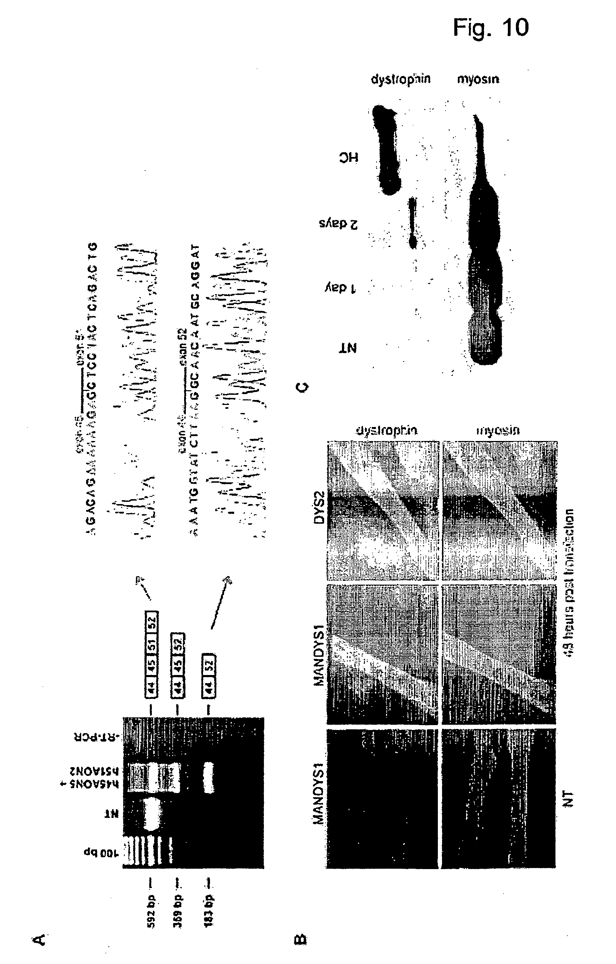

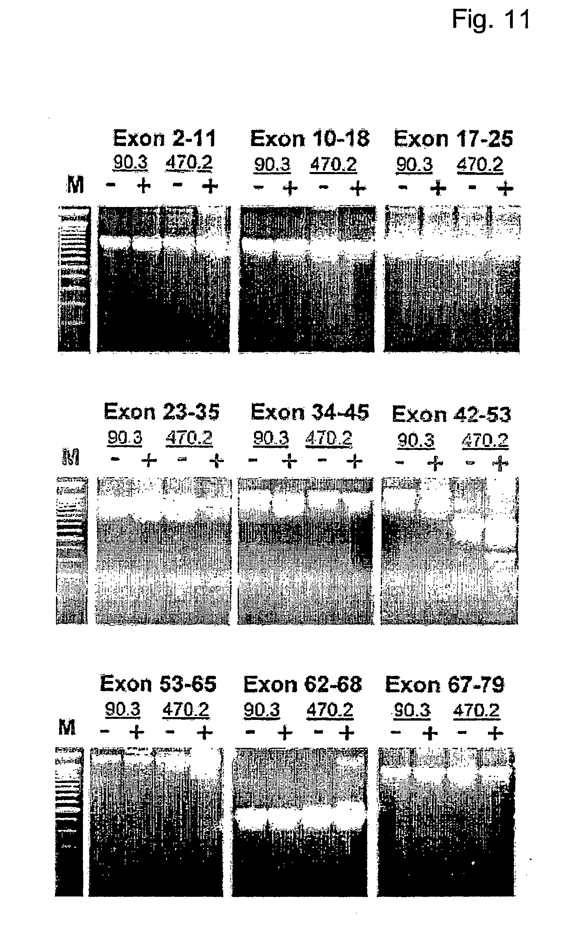

The skipping of only a single exon is not sufficient to restore the reading frame for every mutation. In a significant fraction of mutations, it is necessary to skip two exons simultaneously (fig. 8A). For instance, patientDL90.3 carries a nonsense mutation in exon 43. Considering that this single exon is out of frame, the skipping of exon 43 removes the nonsense mutation but does not restore the reading frame. Since the combination of exons 43 and 44 is in frame, we aimed at the simultaneous skipping of both exons. Patient DL470.2 is affected by a deletion of exons 46-50. Frame restoration requires the joint skipping of the two exons flanking the deletion(fig. 8*A*).Myotube cultures from these patients were transfected with a mixture of either exon 43- and 44-specific AONs(patient DL90.3) or exon 45- and 51-specific AONs (patient DL470.2). Transfection efficiencies were typically more than 80%, as indicated by the number of cells with specific nuclear uptake of fluorescent AONs. RT-PCR analysis at 24 or 48 h after transfection indeed demonstrated the feasibility of specific double-exon skipping in both patients (30% and 75% of total transcript fragments for patients DL90.3 and DL470.2, respectively),which was confirmed by sequence analysis (figs. 9A and 10*A*). As expected, in addition to double-exon skipping, we also detected single-exon skipping in patient DL90.3(exon 44; 27% of total transcripts) and patient DL470.2(exon 51; 12% of total transcripts).To verify that no larger or other regions of the *DMD* transcript were affected by the AON treatments, we performed RT-PCR analysis on the entire *DMD* gene, with consecutive sets of exons. In both treated and untreated myotubes, we did not detect additional aberrant splicing patterns in the *DMD* gene (fig. 11).Immunohistochemical analysis using two different antibodies directed against internal (MANDYS1) and C-terminal(Dys2) parts of dystrophin showed that the in frame transcripts derived from double-exon skipping produced BMD-like dystrophins. On average, 70% of the myosin-positive myotubes showed dystrophin expression in response to AON transfection (figs. 9*B* and 10B).Western blot analysis confirmed the presence of dystrophin for patient DL90.3 (after 4 d) and patient DL470.2 (after 2d), at levels of 3.3% and 1.8%, respectively, when compared to control myotubes (figs. 9C and 10*C*).

### Multi-exon Skipping

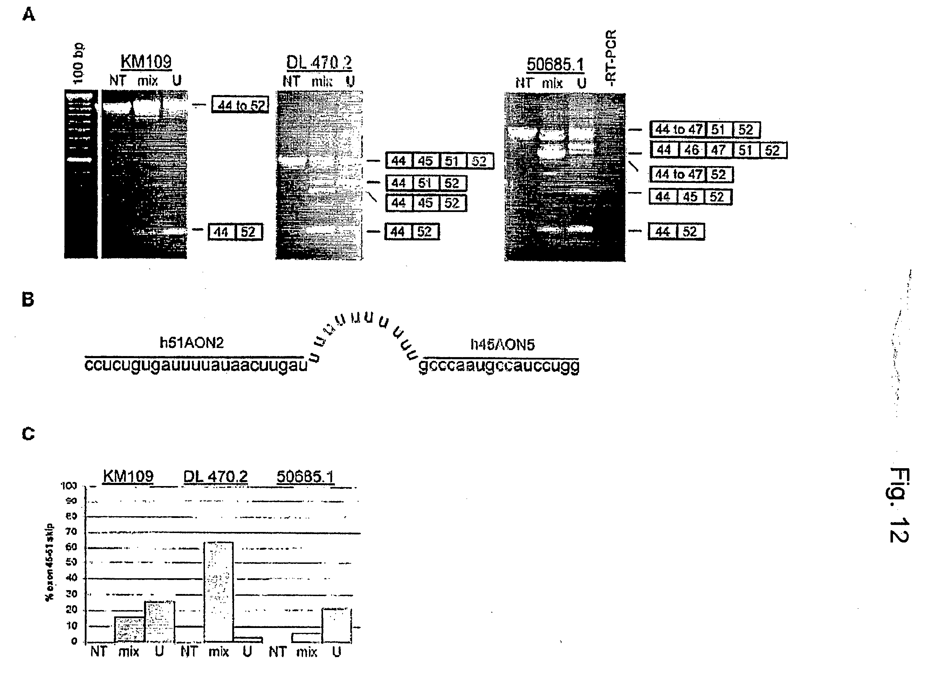

The splicing of exon 44 directly to exon 52 (as induced in patient DL470.2) generates an in-frame transcript. Inducing the skipping of this entire stretch of exons-that is, multi-exon skipping-would generate a BMD-like deletion (exons 45-51) spanning a range of smaller, internal *DMD* mutations (fig. 8*B*). Multi-exon skipping was first tested in human control myotubes (individualKM109) treated with a mixture of 200 nM of both the exon 45- and 51-specific AONs (fig. 12*A*). We observed a novel, shorter transcript, corresponding to a size that would result from the targeted skipping of exons 45-51. Indeed, sequence analysis revealed that exon 44 was directly spliced to exon 52 (data not shown). We then applied it to myotubes derived from a patient with DMD(50685.1) carrying an exon 48-50 deletion (fig. 8*B*). The AON-induced skipping of all exons from 45 to 51yielded the intended in-frame transcript (fig. 12A).

### Double and Multi-exon Shipping Using a U-Linked Combination of AONs

The skipping of more than one exon from one pre-mRNA molecule requires both AONs to be present in the same nucleus, targeting the same molecule. To specifically enhance this probability, we designed a combined AON containing both the exon 45 and 51 AONs(h45AON5 and h51AON2), linked by 10 uracil nucleotides(fig. 12*B*). After transfection of this "U-linked AON" into myotubes from the human control (individualKM109) and the two patients with DMD (DL470.2and 50685.1), RT-PCR analysis demonstrated its efficacy to generate the in-frame transcript with exon 44spliced to exon 52 (fig. 12A). This multi-exon skipping occurred specifically and precisely at the exon boundaries, as confirmed by sequence analysis (data not shown). Quantification showed that the U-linked AON was more efficient than the mixture of AONs in myotubes from patient 50685.1 and the human control but not in myotubes from patient DL470.2 (fig. 12*C*).

### Discussion

Induced-induced skipping of a single exon has shown therapeutic potential to correct the reading frame and induce the synthesis ofBMD-like dystrophins in cultured muscle cells from patients with DMD (Takeshima et al.2001; van Deutekom et al. 2001; De Angelis et al. 2002;Aartsma-Rus et al. 2003). Here, we demonstrate the feasibility of targeted antisense-induced multi-exon skipping for therapeutic purposes. The spontaneous skipping of multiple exons frequently occurs in nature, albeit at low levels. This has been detected both in patients with DMD and in unaffected individuals (Sironi et al. 2002).Furthermore, this phenomenon has been suggested to be the underlying mechanism for the occurrence of dystrophin-positive "revertant" fibers in the *mdx* mouse model and in patients with DMD (Sherratt et al. 1993; Thanhet al. 1995; Lu et al. 2000). Multi-exon skipping has also been observed in DMD gene-therapy studies aimed at the targeted skipping of the mutated exon 23 in *mdx-*mouse muscle cells (Dunckley et al. 1998; Wilton et al.1999; Bertoni et al. 2003). Both AONs and chimeric DNA-RNA oligonucleotides directed at the 3'-splice site of this exon generated shorter in-frame or out-of-frame transcripts in which additional exons adjacent to exon23 were skipped. In this study, we specifically focused on inducing multi-exon skipping. By use of combinations of AONs, the double skipping of exons 43-44 and 45-51 was induced in patient-derived myotube cultures. Immunohistochemical analysis of single myotubes indicated that this allowed the synthesis of dystrophins in up to 70% of myotubes. This percentage is not significantly lower than those obtained in our previous single exon-skipping studies (75%-80%) (Aartsma-Rus et al.2003). This suggests that both the transfection and the skipping performance of two AONs simultaneously are not significantly less efficient than those of a single AON. However, in a number of the remaining dystrophin-negative myotubes only single-exon skipping occurred. Western blot analysis of protein extracts from patient derived total myotube cultures showed relatively low levels (less than 3%) of dystrophin. Considering the high levels(70%) observed in the immunohistochemical analysis, there seems to be a discrepancy. However, this can be explained by the fact that in the immunohistochemical analysis we focus on single myosin-positive myotubes, whereas the samples in the Western blot analysis also include a significant number of dystrophin-negative cells. Furthermore, the control protein sample was derived from a culture that exhibited a two-fold higher degree of myogenicity (i.e., approximately 40% in the patient cultures vs. more than 90% in the control sample) and that accordingly contained a higher number of dystrophin-producing cells. Finally, the control protein sample in the Western blot analysis was derived from myotubes expressing dystrophin over the entire period of differentiation (2wk), whereas in the patient-derived myotubes the dystrophin synthesis was only just induced at the time of analysis (at most, 4 d). Since myotubes are viable for only several days after transfection, longer expression periods were unachievable. Different ratios of exon 45- and 51-specific AONs were evaluated, and the highest levels of double-exon skipping were obtained with a 1:1 ratio (data not shown). Both AONs were apparently equally efficient at entering the nucleus. Besides double-exon skipping, we also observed single-exon skipping of exon 44 (patientDL90.3) and exon 51 (patient DL470.2), in particular. This suggests that the local secondary premRNA structure was probably more affected by AONs targeting these exons, which subsequently rendered exons 43 and 45 inaccessible. To increase the probability not only that both AONs are taken up by the same nucleus but also that they hybridize to the same RNA molecule, we designed an AON that consisted of two AONs linked with 10 uracil nucleotides. Compared to the mixture of the two AONs, the U-linked AON indeed induced higher levels of multi-exon skipping in myotubes from a human control and a patient carrying an exon 48-50 deletion. In contrast, for yet-unknown reasons it was less efficient in inducing double-exon skipping in myotubes from a patient carrying an exon 46-50 deletion. In follow-up studies, we assessed the influence of the length of the U-linker. We did not observe significant differences (data not shown).There are two possible explanations for the mechanism of multi-exon skipping. First, the entire region of both exons and introns between exon 45 and 51 may have been spliced out because of an overall AON-induced alteration in the secondary structure of the pre-mRNA. Second, the splicing of the individual exons 45 through 51 may have occurred earlier than that of exon 44 to exon 45 (which is not unlikely, given that intron 44 is 270 kb long). In that case, the splicing machinery regarded exons 45 to 51 as one large exon and omitted it because of the AONs that hybridized to exons 45 and 51. The latter explanation suggests that multi-exon skipping may only be effective in those areas of the *DMD* gene in which downstream introns are spliced prior to upstream introns. We are currently verifying this by using different combinations of (U-linked) AONs in other regions of the gene. When only single-exon skipping is taken into account, the antisense-based reading frame correction therapy would theoretically be beneficial for more than 75% of all patients with DMD. Multi-exon skipping would significantly extend this percentage to most *DMD* mutations, except those that affect the functionally critical domains of the N-terminus or the cysteine-rich C-terminal domain, or those that involve the promoter re region, the first exon, or translocations. However, these latter mutations are found in less than 8% of all patients with DMD reported in our database (den Dunnen 1996). Multi-exon skipping not only increases the number of patients that would benefit from this approach but, more importantly, also decreases its mutation specificity. The multi-exon skipping of exons 45-51 shown in this study would be frame restoring for 14% of all the deletions and 6% of all small mutations reported in the DMD mutation database (den Dunnen 1996). Furthermore, it offers the therapeutic potential of generating relatively large in-frame deletions known to be associated with mild BMD phenotypes (England et al. 1990;Winnard et al. 1993; Mirabella et al. 1998). Its eventual therapeutic application will largely depend on the efficiencies in vivo. Lu and colleagues (2003) recently demonstrated AON-induced exon 23-skipping in *mdx* muscle by using the Pluronic copolymer F127 as a delivery reagent. Close-to-normal levels of a nearly full-length dystrophin were observed in many myofibers, which improved muscle function. Although the effect was optimal at 2-4 wk, dystrophin was still detectable at 3 mo. after injection (Lu et al. 2003). In our experience, the in vitro and in vivo effects of AONs in muscle cells correlate relatively well, which provides a promising basis for our current studies on multi-exon skipping in mice.

The URL for data presented herein is as follows: Online Mendelian Inheritance in Man (OMIM), http://www.ncbi.nlm.nih.gov/Omim/ (for DMD and BMD)

### References to example 5

Aartsma-Rus A, Bremmer-Bout M, Janson A, den Dunnen J, van Ommen G, van Deutekom J (2002) Targeted exon skipping as a potential gene correction therapy for Duchenne Muscular dystrophy. Neuromuscul Disord 12:S71
Aartsma-Rus A, Janson AA, Kaman WE, Bremmer-Bout M, den Dunnen JT, Baas F, Van Ommen GJ, et al (2003) Therapeutic antisense-induced exon skipping in cultured muscle cells from six different DMD patients. Hum Mol Genet 12:907-914
Bertoni C, Lau C, Rando TA (2003) Restoration of dystrophin expression in mdx muscle cells by chimeraplast-mediated exon skipping. Hum Mol Genet 12:1087-1099
Buratti E, Baralle FE, Pagani F (2003) Can a "patch" in a skipped exon make the pre-mRNA splicing machine run better? Trends Mol Med 9:229-232
Cartegni L, Chew SL, Krainer AR (2002) Listening to silence and understanding nonsense: exonic mutations that affect splicing. Nat Rev Genet 3:285-298
Cartegni L, Krainer AR (2003) Correction of disease-associated exon skipping by synthetic exon-specific activators. Nat Struct Biol 10:120-125
Corey DR, Abrams JM (2001) Morpholino antisense oligonucleotides: tools for investigating vertebrate development. Genome Biol 2:reviews1015.1-reviews1015.3
De Angelis FG, Sthandier O, Berarducci B, Toso S, Galluzzi G, Ricci E, Cossu G, Bozzini I (2002) Chimeric snRNA molecules carrying antisense sequences against the splice junctions of exon 51 of the dystrophin pre-mRNA induce exon skipping and restoration of a dystrophin synthesis in Delta 48-50 DMD cells. Proc Natl Acad Sci USA 99:9456-9461 den Dunnen J (1996) The Leiden Muscular Dystrophy pages.http://www.dmd.nl (accessed December 10, 2003)
den Dunnen JT, Grootscholten PM, Bakker E, Blonden LA,Ginjaar HB, Wapenaar MC, van Paassen HM, van Broeckhoven C, Pearson PL, van Ommen GJ (1989) Topography of the Duchenne muscular dystrophy (DMD) gene: FIGE and cDNA analysis of 194 cases reveals 115 deletions and 13 duplications. Am J Hum Genet 45:835-847
Dennis JU, Dean NM, Bennett CF, Griffith JW, Lang CM, Welch DR (1998) Human melanoma metastasis is inhibited following ex vivo treatment with an antisense oligonucleotide to protein kinase C-alpha. Cancer Lett 128:65-70
Di Blasi C, Morandi L, Barresi R, Blasevich F, Cornelio F, Mora M (1996) Dystrophin-associated protein abnormalities in dystrophin-deficient muscle fibers from symptomatic and asymptomatic Duchenne/Becker muscular dystrophy carriers. Acta Neuropathol (Berl) 92:369-377 Dove A (2002) Anti-sense and sensibility. Nat Biotechnol 20:121-124
Dunckley MG, Manoharan M, Villiet P, Eperon IC, Dickson G (1998) Modification of splicing in the dystrophin gene in cultured Mdx muscle cells by antisense oligoribonucleotides. Hum Mol Genet 7:1083-1090
England SB, Nicholson LV, Johnson MA, Forrest SM, Love DR, Zubrzycka-Gaarn EE, Bulman DE, Harris JB, DaviesKE (1990) Very mild muscular dystrophy associated with the deletion of 46% of dystrophin. Nature 343:180-182
Ervasti JM, Ohlendieck K, Kahl SD, Gaver MG, Campbell KP(1990) Deficiency of a glycoprotein component of the dystrophin complex in dystrophic muscle. Nature 345:315-319
Friedman KJ, Kole J, Cohn JA, Knowles MR, Silverman LM,Kole R (1999) Correction of aberrant splicing of the cysticfibrosis transmembrane conductance regulator (CFTR) gene by antisense oligonucleotides. J Biol Chem 274:36193-36199
Havenga MJ, Lemckert AA, Ophorst OJ, van Meijer M, Germeraad WT, Grimbergen J, van Den Doel MA, Vogels R, van Deutekom JCT, Janson AAM, de Bruijn JD, Uytdehaag F, Quax PH, Logtenberg T, Mehtali M, Bout A (2002) Exploiting the natural diversity in adenovirus tropism for therapy and prevention of disease. J Virol 76:4612-4620
Hoffman EP, Brown RH, Jr., Kunkel LM (1987) Dystrophin: the protein product of the Duchenne muscular dystrophyn locus. Cell 51:919-928
Hoffman EP, Fischbeck KH, Brown RH, Johnson M, Medori R, Loike JD, Harris JB, et al (1988) Characterization of dystrophin in muscle-biopsy specimens from patients with Duchenne's or Becker's muscular dystrophy. N Engl J Med318:1363-1368
Koenig M, Beggs AH, Moyer M, Scherpf S, Heindrich K, Bettecken T, Meng G, et al (1989) The molecular basis for Duchenne versus Becker muscular dystrophy: correlation of severity with type of deletion. Am J Hum Genet 45:498-506
Koenig M, Monaco AP, Kunkel LM (1988) The complete sequence of dystrophin predicts a rod-shaped cytoskeletal protein. Cell 53:219-226
Krawczak M, Reiss J, Cooper DN (1992) The mutational spectrum of single base-pair substitutions in mRNA splice junctions of human genes: causes and consequences. Hum Genet90:41-54
Lu QL, Mann CJ, Lou F, Bou-Gharios G, Morris GE, Xue SA, Fletcher S, Partridge TA, Wilton SD (2003) Functional amounts of dystrophin produced by skipping the mutated exon in the mdx dystrophic mouse. Nat Med 9:1009-1014
Lu QL, Morris GE, Wilton SD, Ly T, Artem'yeva OV, Strong P, Partridge, TA (2000) Massive idiosyncratic exon skipping corrects the nonsense mutation in dystrophic mouse muscle and produces functional revertant fibers by clonal expansion. J Cell Biol 148:985-996
Mann CJ, Honeyman K, Cheng AJ, Ly T, Lloyd F, Fletcher S, Morgan JE, Partridge TA, Wilton SD (2001) Antisense-inducedexon skipping and synthesis of dystrophin in the mdx mouse. Proc Natl Acad Sci USA 98:42-47
Mann CJ, Honeyman K, McClorey G, Fletcher S, Wilton SD(2002) Improved antisense oligonucleotide induced exon skipping in the mdx mouse model of muscular dystrophy. J Gene Med 4:644-654
Mercatante DR, Mohler JL, Kole R (2002) Cellular response to an antisense-mediated shift of Bcl-x pre-mRNA splicing and anti neoplastic agents. J Biol Chem 277:49374-49382
Mercatante DR, Sazani P, Kole R (2001) Modification of alternative splicing by antisense oligonucleotides as a potential chemotherapy for cancer and other diseases. Curr. Cancer Drug Targets 1:211-230
Mirabella M, Galluzzi G, Manfredi G, Bertini E, Ricci E, DeLeo R, Tonali P, Servidei S (1998) Giant dystrophin deletion associated with congenital cataract and mild muscular dystrophy. Neurology 51:592-595
Murry CE, Kay MA, Bartosek T, Hauschka SD, Schwartz SM(1996) Muscle differentiation during repair of myocardial necrosis in rats via gene transfer with MyoD. J Clin Invest 98:2209-2217
Nasevicius A, Ekker SC (2000) Effective targeted gene "knockdown" in zebrafish. Nat Genet 26:216-220
Roest PA, Roberts RG, van der Tuijn AC, Heikoop JC, van Ommen GJ, den Dunnen JT (1993) Protein truncation test(PTT) to rapidly screen the DMD gene for translation terminating mutations. Neuromuscul Disord 3:391-394
Roest PA, van der Tuijn AC, Ginjaar HB, Hoeben RC, Hoger-Vorst FB, Bakker E, den Dunnen JT, van Ommen GJ (1996) Application of in vitro Myo-differentiation of non-muscle cells to enhance gene expression and facilitate analysis of muscle proteins. Neuromuscul Disord 6:195-202
Sherratt TG, Vulliamy T, Dubowitz V, Sewry CA, Strong PN(1993) Exon skipping and translation in patients with frameshift deletions in the dystrophin gene. Am J Hum Genet 53:1007-1015
Sierakowska H, Sambade MJ, Agrawal S, Kole R (1996) Repair of thalassemic human beta-globin mRNA in mammalian cells by antisense oligonucleotides. Proc Natl Acad Sci USA 93:12840-12844
Sironi M, Cagliani R, Pozzoli U, Bardoni A, Comi GP, GiordaR, Bresolin N (2002) The dystrophin gene is alternatively spliced throughout its coding sequence. FEBS Lett 517:163-166
Stevenson JP, Yao KS, Gallagher M, Friedland D, Mitchell EP,Cassella A, Monia B, Kwoh TJ, Yu R., Holmlund J, DorrFA, O'Dwyer PJ (1999) Phase I clinical/pharmacokinetic and pharmacodynamic trial of the c-raf-1 antisense oligonucleotide ISIS 5132 (CGP 69846A). J Clin Oncol 17:2227-2236
Takeshima Y, Wada H, Yagi M, Ishikawa Y, Minami R, Nakamura H, Matsuo M (2001) Oligonucleotides against a splicing enhancer sequence led to dystrophin production in muscle cells from a Duchenne muscular dystrophy patient. Brain Dev 23:788-790
Thanh LT, Nguyen Thi M, Hori S, Sewry CA, Dubowitz V, Morris GE (1995) Characterization of genetic deletions inBecker muscular dystrophy using monoclonal antibodies against a deletion-prone region of dystrophin. Am J Med Genet 58:177-186
van Deutekom JC, Bremmer-Bout M, Janson AA, Ginjaar IB,Baas F, den Dunnen JT, van Ommen GJ (2001) Antisense induced exon skipping restores dystrophin expression in DMD patient derived muscle cells. Hum Mol Genet 10:1547-1554
Wilton SD, Lloyd F, Carville K, Fletcher S, Honeyman K, Agrawal S, Kole R (1999) Specific removal of the nonsense mutation from the mdx dystrophin mRNA using antisense oligonucleotides. Neuromuscul Disord 9:330-338 Winnard AV, Klein CJ, Coovert DD, Prior T, Papp A, Snyder P, Bulman DE, et al (1993) Characterization of translational frame exception patients in
Duchenne/Becker muscular dystrophy. Hum Mol Genet 2:737-744 Yoshida M, Ozawa E (1990) Glycoprotein complex anchoring dystrophin to sarcolemma. J Biochem (Tokyo) 108:748-752.

### Example 6

As small molecule drugs for Duchenne muscular dystrophy (DMD), antisense oligonucleotides (AONs) have been shown to restore the disrupted reading frame of DMD transcripts by inducing specific exon skipping. This allows the synthesis of largely functional BMD-like dystrophins and potential conversion of severe DMD into milder BMD phenotypes. Thus far we have used 2'O-methyl phosphorothioate (2OMePS) AONs. Here, we assessed the skipping efficiencies of different AON analogs containing morpholino-phosphorodiamidate, locked nucleic acid (LNA) or peptide nucleic acid (PNA) backbones. In contrast to PNAs and morpholinos, LNAs have not yet been tested as splice modulators. Compared to the most effective 2OMePS AON directed at exon 46, the LNA induced higher skipping levels in myotubes from a human control (85% vs. 20%) and an exon 45 deletion DMD patient (98% vs. 75%). The morpholino-induced skipping levels were only 5-6%, whereas the PNA appeared to be ineffective. Further comparative analysis of LNA and 2OMePS AONs containing up to three mismatches, revealed that LNAs, while inducing higher skipping efficiencies, show less sequence specificity. This limitation increases the risk of adverse effects elsewhere in the human genome. Awaiting further improvements in oligo-chemistry, we thus consider 2OMePS AONs currently the most favorable compounds for targeted DMD exon skipping.

Antisense oligonucleotides (AONs) have been reported to modulate pre-mRNA splicing in several studies (1). For instance, AONs have restored normal splicing by blocking cryptic splice sites (2,3), altered the ratio of alternative splicing from malignant to non-malignant isoforms (4), and induced exon inclusion for mutated exons, that were otherwise skipped (5). In these studies, the AON treatments aimed at the reestablishment of wild-type mRNA. Recently, AONs have alternatively been used to restore the disrupted reading frame of dystrophin mRNAs in Duchenne muscular dystrophy (DMD) gene therapy studies. DMD patients suffer from severe muscle degeneration due to frame-disrupting mutations in the DMD gene that prematurely abort the synthesis of the dystrophin protein (6-9). In contrast, mutations in the DMD gene that do not affect the reading frame generate internally deleted but partly functional dystrophins and result in less severe Becker muscular dystrophy (BMD) (10,11). AON-induced restoration of the DMD reading frame is based on inducing the skipping of specific exons. This was successfully applied in cultured muscle cells from Duchenne muscular dystrophy patients and in the *mdx* mouse model (12-19). High exon skipping levels of up to ∼90% were achieved, allowing the synthesis of significant levels of BMD-like dystrophins in over 75% of treated cells (17). These dystrophins located appropriately to the sarcolemma and restored the dystrophin glycoprotein complex, a strong indication of functional restoration.

The AONs used in these studies contained 2'O-methyl modified ribose molecules to render them RNase-H independent, and a full-length phosphorothioate backbone (2OMePS AONs) (Table 6). Although 2OMePS AONs have advantages such as increased resistance to nuclease degradation and increased uptake when compared to phosphodiester AONs, disadvantages are that a phosphorothioate backbone is to some extent cytotoxic, and may elicit an immunogenic response (20,21).

Recent developments in oligo-chemistry have provided AONs with different biophysical, biochemical and biological properties based on various modifications to the sugar or the backbone of the nucleotides. Modified AON analogs include morpholino-phosphorodiamidates (morpholinos), locked nucleic acids (LNAs) and peptide nucleic acids (PNAs) (reviewed in 21).

In morpholinos the sugar phosphate backbone of DNA is replaced by morpholino-phosphorodiamidate oligonucleotides (Table 6) (22,23). Morpholinos are non-toxic, nuclease resistant (23), have an increased affinity for RNA and are suggested to disrupt the secondary structure of RNA (1,22). On the other hand, morpholinos are uncharged and therefore difficult to transfect. Nonetheless, reasonable to good efficiencies have been obtained with the scrape loading technique or with ethoxylated polyethylenimine (EPEI) (24,25). Morpholinos have been used, amongst others, to study developmental processes by knocking down genes in zebra fish (26), and to modulate the splicing of the ß-globin gene in vitro and in vivo (25,27). Recently morpholinos have been applied in the *mdx* mouse model to induce the skipping of the mutated exon 23 (28). The morpholinos were transfected into the cells in combination with a sense oligo (leash) to allow formation of a cationic lipoplex. This morpholino treatment restored the dystrophin synthesis in *mdx* muscle cells both *in vitro* and *in vivo.*

LNAs are DNA analogs that contain a methylene bridge, which connects the 2'-oxygen of ribose with the 4'-carbon (Table 6). This bridge results in a locked 3'-endo conformation, which reduces the conformational flexibility of the ribose (1). LNAs are nuclease-resistant, non-toxic and have the highest affinity for complementary DNA and RNA yet reported for any DNA analog (29,30). This high affinity offers both advantages and disadvantages: LNAs will hybridize very efficiently to their target, but LNAs longer than 15 base pairs have shown thermally stable self-structuring. Furthermore, full-length LNAs seem to hybridize in a less sequence specific manner than PNA and 2OMePS AONs (29,31). LNAs are negatively charged and cationic lipid polymers are applied for delivery into cells. LNAs have shown to be potent inhibitors of the expression of a cancer related gene (31), but have not earlier been studied as splicing modulators.

In PNAs the sugar phosphate backbone of DNA is replaced by an achiral polyamide backbone (Table 6) (32). Compared to the 2OMePS backbone, PNAs have a highly increased affinity for DNA and RNA, are suggested to be more sequencespecific, protease- and nuclease-resistant, and non-toxic even at high concentrations (1,33). A drawback of the non-ionic nature of PNA is its poor water-solubility, which makes it complicated to transfect PNAs (27,34). Sazani and colleagues have recently bypassed this problem by coupling 4 lysine residues to the C-terminus of PNAs (27,35). The cationic nature of the lysine highly improved the water-solubility and allowed entrance into the cell and nucleus without transfection reagent. PNAs have successfully been used to modulate the splicing of the murine interleukin-5 receptor-α chain *in vitro* and the ß-globin gene *in vitro* and in vivo (27,35,36).

For future clinical studies, the preferred AON analog induces the highest levels of exon skipping at low levels of cytotoxicity. In this study we have compared the efficacy, efficiency, and applicability of morpholino, LNA and PNA AON analogs to those of a previously described 2OMePS AON (16,37) specific for DMD exon 46 in both control and patient-derived myotubes.

### RESULTS

### Comparative analysis of AON analogs in human control myotubes

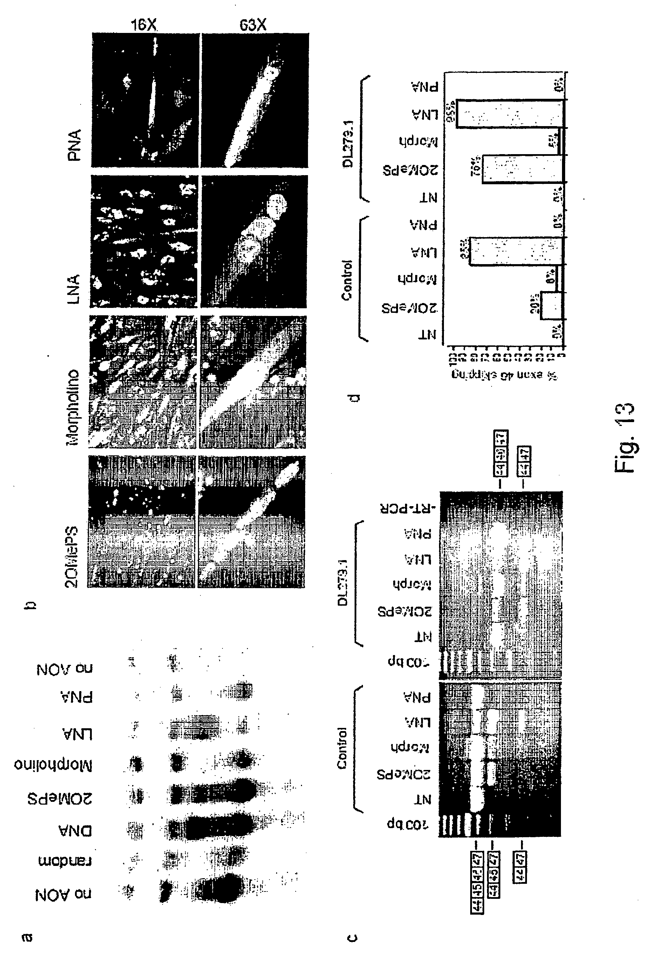

To determine the binding affinity of the different AON analogs (Table 6) to the target pre-mRNA, a gel mobility shift assay was performed. DNA, 2OMePS, morpholino, LNA and PNA AONs were hybridized to a ³²P-labeled exon 46 RNA fragment (Fig. 13 A). The DNA, 2OMePS and LNA AONs induced a clear mobility shift, indicating that these analogs are able to bind to the target RNA. No shift was detectable for the morpholino and PNA AONs. This suggested a low affinity of these analogs for the target RNA. However, in previous experiments we observed that some AONs, while not inducing a notable mobility shift, nevertheless did induce exon skipping (unpublished results). For this reason, we decided to still include the morpholino and PNA AONs in further analyses.

Transfection conditions for the different AON-analogs, all carrying a 5' fluorescein group, were optimized in human control myotubes (Fig. 13B). Based on the presence of nuclear fluorescent signals, transfection efficiencies were determined to be typically over 80% for the 2OMePS, morpholino and LNA AONs. The PNA transfection efficiencies were generally lower (∼60-70%). Notably, in contrast to the 2OMePS, LNA and PNA AONs, which showed most of the fluorescence within the nucleus, the morpholino was also clearly present in the cytoplasm.

Previous experiments revealed that most efficient skipping levels, as detected by RT-PCR, were obtained at a dose of 500 nM of 2OMePS AONs (17). Here we show that also LNA and, remarkably, morpholino AONs are effective in inducing exon 46 skipping (Fig. 13C). For the morpholino and LNA AONs, concentration series experiments indicated highest skipping efficiencies at doses of 1 µM and 500 nM, respectively. For both analogs, higher concentrations did not result in higher levels of exon 46 skipping (data not shown), but instead induced serious cytotoxic effects for the morpholino AON. Only minimal levels of cytotoxicity were present in myotube cultures transfected with 500 nM of LNA six days post-transfection, whereas at that time-point severe cell-death was observed for 200 nM of the 2OMePS AON (data not shown). Since more units of PEI were applied for LNA transfection, this difference is unlikely due to PEI-induced cytotoxicity. We occasionally observed the skipping of both exon 45 and 46 at low levels in response to high doses of the 2OMePS and LNA AONs (Fig. 13C). The PNA did persistently not induce exon 46 skipping, even at a 20 µM dose (data not shown). Exon 46 skipping efficiencies of the different analogs were assessed through quantification of the RT- PCR fragments (Fig. 13D). In comparison with the 2OMePS (20%), the LNA AON was more efficient (85%), whereas the skipping levels for the morpholino were not higher than 6%.

Comparative analysis of AON analogs in patient-derived myotubes We have previously observed that 2OMePS AONs induced higher levels of exon skipping in patient-derived myotubes when compared to control samples (16,17). This effect was here confirmed in myotubes derived from a DMD patient (DL279.1) affected by an exon 45 deletion. For this patient, exon 46 skipping generates in-frame transcripts. We observed 75% exon 46 skipping in DL279.1 vs. 20% in control myotubes for the 2OMePS AON (Fig. 13C and 13D). The LNA and morpholino AONs were also able to induce exon skipping (98% and 5%, respectively), whereas the PNA was not (Fig. 13C and 13D). Similar to the 2OMePS AON, the LNA showed higher skipping levels in DL279.1 when compared to those in control myotubes (98% vs. 85%). This effect however was not significant for the morpholino or PNA AONs.

### Dose-effects and sequence-specificity of LNA vs. 2OMePS AONs

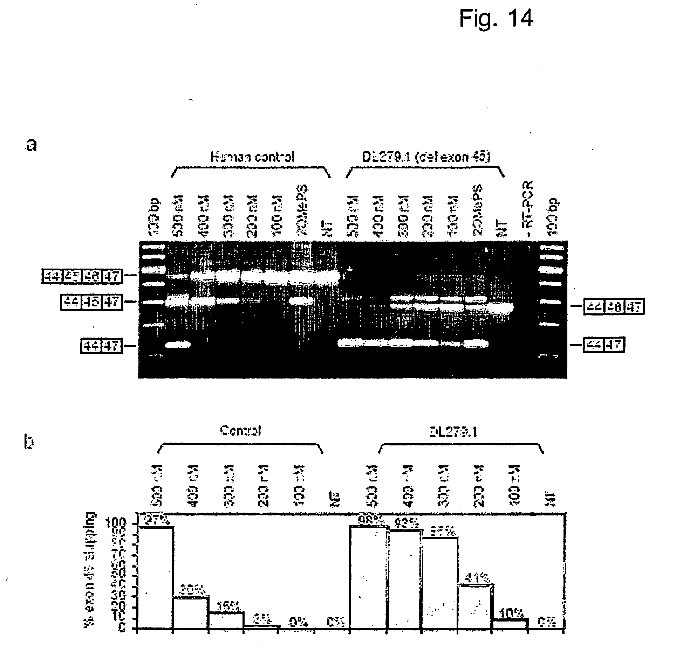

Since the LNAs induced highest skipping levels, we performed a concentration series to determine the minimally effective dose. RT-PCR (Fig. 14A) and quantitative analysis (Fig. 14B) showed that in human control myotubes the efficiency dropped markedly from 97% to 30% at doses lower than 500 nM, and that very low levels of exon 46 skipping (< 1%) were detectable at 100 nM. In patient-derived myotubes, however, the levels of exon skipping decreased more gradually at lower doses, with exon 46 skipping levels of 86% detectable at a dose of 300 nM, and still a significant level (10%) at 100 nM (Fig. 14AB).

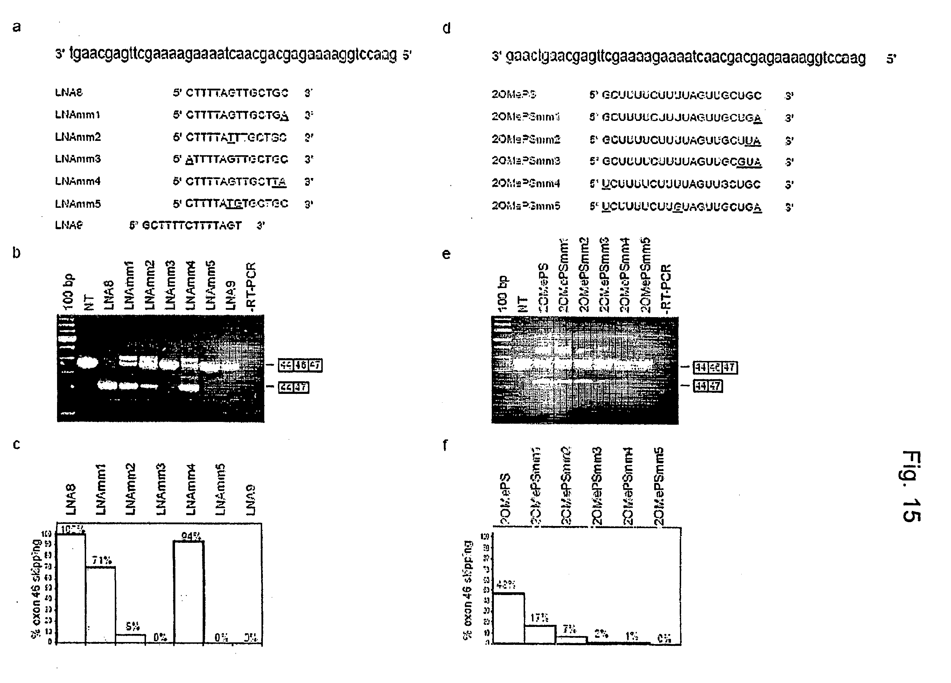

We then analyzed the sequence-specificity of LNA and 2OMePS AONs in patient myotubes (since they showed higher skipping levels than control myotubes). We studied five different LNAs, which contain one or two mismatches at the 5', 3' or center position (LNAmm1-5, Fig. 15A), and one LNA that was shifted in the 3' direction of exon 46 (LNA9, Fig. 15A). A gel mobility shift assay revealed that all LNAs were able to bind to the target pre-mRNA (data not shown). The LNAs were then transfected into patient myotubes at a dose of 500 nM. RT-PCR analysis (Fig. 15B) and quantification (Fig. 15C) showed that the LNAs containing 1 or 2 mismatches at the 3' end (LNAmm1 and LNAmm4) were able to induce exon 46 skipping at high levels, comparable to the original 100% complementary LNAS (71%-94% vs. 100%), suggesting that LNAs have a reduced sequence-specificity. The LNA containing a single mismatch in the center (LNAmm2) induced low levels (8%) of exon skipping, whereas the LNA containing two mismatches in the center (LNAmm5) and the 5' mismatched LNA (LNAmm3) did not induce detectable skipping. LNA9 did not induce exon 46 skipping, even though it is completely homologous to exon 46. We also assessed the effects of lower concentrations of the 3' mismatch-containing LNAs, and observed exon skipping at comparably lower levels as with LNA8 (data not shown).

We similarly tested five different 2OMePS oligos containing up to three mismatches when compared to the original 2OMePS oligo in patient myotubes (2OMePSmm1-5, Fig. 15D). In contrast to the LNAs, the 2OMePS AONs containing one or two mismatches at the 3' end (2OMePSmm1-2) induced exon 46 skipping at reduced levels when compared to the original oligo (7%-17% vs. 48%) (Fig. 15E, F). The oligos containing three mismatches at the 3' end (2OMePSmm3) or one at the 5' end (2OMePSmm4) induced barely detectable levels (1%-2%) of exon skipping, whereas the 2OMePS containing three mismatches dispersed throughout the oligo (2OMePSmm5) was unable to induce exon skipping. Transfection experiments and RT-PCR analyses of the mismatched 2OMePS AONs and LNAs were repeated several times and showed reproducible efficiencies. Our results indicate that the 2OMePS AONs have a higher sequence-specificity than the LNAs.

### DISCUSSION

In our previous studies on antisense-induced exon skipping 2OMePS AONs were applied (16,17,37). In this study additional AON analogs were tested for their efficacy, efficiency and applicability in inducing DMD exon 46 skipping in control and DMD patient-derived myotube cultures. Towards future clinical trials the most optimal AON analog should induce high levels of exon skipping, but also be non-toxic, easy to deliver and, preferably, relatively inexpensive. Out of the four alternative analogs tested here, only LNAs induced higher levels of exon skipping when compared to 2OMePS AONs. The morpholino was less efficient in both patient and control myotubes, whereas the PNA was completely ineffective. For both the LNA and 2OMePS AONs, the levels of exon skipping were higher in patient-derived cells than in control cells. We have observed this effect previously in cells from other DMD patients (17) and hypothesize that it is due to nonsense mediated RNA decay (NMD), which will selectively target the out-of-frame skip-product in control myotubes and thereby negatively influence the relative amount of skipped product. In patient derived cells, the original out-of-frame mRNA is subject to NMD, whereas the inframe skip-product is not. Another explanation may be that AON-mediated exon skipping is actually enhanced in the patient due to the presence of the deletion that already perturbed local splicing. The levels of morpholino-induced exon skipping were comparable in the patient and control samples (6% vs. 5%).

The gel mobility assay showed no shift for the morpholino and PNA AONs, but despite its apparent low affinity for to the target pre-mRNA, the morpholino was able to induce low levels of exon 46 skipping. This may be explained by the fact that the morpholino was hybridized to a sense DNA oligo (a "leash" allowing EPEI coupled transfection), which may have interfered with the proper hybridization to the target mRNA fragment. After transfection into cells, the leash detaches from the morpholino, which is then free to hybridize to the target RNA. Schmajuk and colleagues found that morpholinos were more effective in restoring the wild type splicing of the ß-globin gene when compared to 2OMePS AONs (25). However, since we observed fluorescence in both the cytoplasm and the nucleus after morpholino transfections, the low levels of exon skipping may be the result of poor nuclear uptake rather than the low efficiency of the morpholino. Further optimization of the morpholino itself and the leash required for EPEI transfection may thus increase the levels of exon 46 skipping. Indeed, Gebski and colleagues have recently shown that levels of exon 23 skipping in the *mdx* mouse varied when different leashes were used (28).

Even though the sequences of the PNA and the LNA analogs are completely identical, the LNA induces high levels of exon 46 skipping, whereas the PNA induces no detectable skipping. PNA oligos have been reported to induce higher levels of correctly spliced ß-globin mRNA than morpholino and 2OMePS analogs (27,35). This indicates that PNA is in fact able to modulate splicing and suggests that the lack of exon 46 skipping in our experiments may result from the inability of our PNA to bind to the specific target RNA sequence, or a poor stability of the PNA-RNA complex. Further experiments may identify PNAs with higher binding affinities to exon 46 target sequences.

LNAs are relatively new AON analogs that have thus far only been applied to inhibit the expression of target genes (29,31). We show here that LNAs are also very potent modulators of pre-mRNA splicing. In series of experiments the LNA induced exon 46 skipping in 85% of control transcripts and in 98% of transcripts from a DMD patient carrying an exon 45 deletion. In comparison, the 2OMePS AONs previously used, induced 20% exon 46 skipping in control cells and 75% skipping in exon 45-deleted transcripts. Notably, the LNA also seemed to be less toxic than 2OMePS AONs.

Based on these results, LNA in principle may be a promising alternative for antisense-induced exon skipping studies. Unfortunately however, due to the shorter sequence (14-mer), it shows complete homology to several other sequences in the human genome. Most of these were located within non-coding regions, either in or near genes. Increasing the length of the LNA enhances specificity (29). Our results with LNAs containing mismatches show that LNAs with one or two mismatches in the 3' part of the AON were almost as potent as the specific LNA. This suggests that the binding of only 12 base pairs is enough to induce the skipping of exon 46, which is not surprising, given the extremely high melting temperatures of LNA (predicted to be 131 °C for our 14mer LNA; Table 6). It does, however, imply that our exon 46 specific LNA may also adversely bind to other sequences in the human genome that contain these 12 base pairs. The LNA may bind to even shorter sequences, since, for instance, a 7-mer still has a predicted melting temperature of over 60 °C. We have recently injected the human LNA into mouse muscle. With one mismatch when compared to the mouse sequence, the human LNA was able to induce skipping of the murine exon 46, even at low concentrations. To decrease the affinity for RNA, chimeric LNA/2'-O-methyl RNA oligos have been generated (38). These chimeras have lower melting temperatures than full length LNA, but still have higher affinities for RNA than 2OMePS AONs. In fact, chimeric LNAs have been shown to block transcription of the HIV-1 transactivating responsive element *in vitro* (38).

Our results with mismatched 2OMePS AONs show that these analogs are more sequence-specific than the LNAs. The presence of one mismatch at the 3' end results in an almost three-fold decrease of exon 46 skipping levels, whereas no exon skipping was left with 2OMePS AONs containing three 3' mismatches. One might have anticipated a greater effect of a single mismatch in the shorter 14-mer LNA than in the longer 20-mer 2OMePS AON. However, the opposite was found true for the 3' end. Furthermore, for both the LNA and the 2OMePS analogs, mismatches at the 3' end induced higher levels of exon 46 skipping than those at the 5' end or in the center of the AONs. This suggests that, whether or not due to the presence of the fluorescent label, mismatches at the 5' end reduced the affinity for the target RNA to a larger extent.

### MATERIALS AND METHODS

### AONs and primers

The characteristics of the AON analogs in this study are reviewed in Table 6. The 2OMePS AONs (sequences shown in Fig. 15D; Eurogentec, Belgium) have a full length phosphorothioate backbone and 2'-O-methyl modified ribose molecules, as previously described (16,37). The morpholino (Gene-Tools, US) has a morpholinophosphoroamidate backbone and is linked to a sense DNA oligo for EPEI transfection. The LNAs (sequences shown in Fig. 15A; Proligo, France) have full length locked nucleic acid backbones. The PNA (Eurogentec, Belgium) has a peptide nucleic acid backbone and 4 lysine residues at the C-terminal end to improve water solubility and facilitate transfection into cells. All AONs contain a 5'fluorescein group (6-FAM).

### Gel mobility shift assay

Human dystrophin exon 46 RNA was *in vitro* transcribed as described previously (16). The binding affinity of the individual AONs (at a dose of 0.5 pmol) was determined by overnight incubation in a hybridization buffer (1 mM Tris-HC pH 7.4, 50 nM NaCl, 5 mM MgCl2) at 37°C, followed by 8% PAGE and PhosphoImager analysis (Molecular Dynamics).

### Myogenic cell cultures and transfection

Primary human myoblasts from an unaffected control and a DMD patient (DL279.1; carrying a deletion of exon 45) were isolated from muscle biopsies and cultured as described previously (37). Myotubes were obtained from confluent myoblast cultures, following 7 to 14 days of serum-deprivation. For the 2OMePS and LNA AONs, PEI was used for transfection, according to the manufacturer's instructions (ExGen500; MBI Fermentas), with 3.5 µl PEI per µg of transfected AON (*i..e.* 12.9 µl (70 µM) for the 2OMePS and 9.2 µl (50 µM) for the LNA oligo). EPEI was used to transfect the morpholino AON according to the manufacturers instructions, with 1 µl of 200 µM EPEI (Gene-Tools, USA) for each 200 nmol of morpholino. PNAs were applied to 1 ml culture medium, without any transfection reagent. Three hours post-transfection 2 ml medium was added.

### RNA isolation, RT-PCR and sequence analysis

mRNA isolation, RT-PCR analysis and direct sequencing were performed as described (17). Primary PCRs included primers in exon 43 (CCTGTGGAAAGGGTGAAGC) and 48 (CTGAACGTCAAATGGTCCTTC); nested PCRs included primers in exon 44 (CGATTTGACAGATCTGTTGAG) and 47 (GAGCACTTACAAGCACGGG). All primers were synthesized by Eurogentec (Belgium). For quantification, the skipproducts were analyzed using the DNA 1000 LabChip® Kit on the Agilent 2100 bioanalyzer (Agilent Technologies, USA).

### References to example 6

1 Braasch DA, Corey DR. Novel antisense and peptide nucleic acid strategies for controlling gene expression. Biochemistry 2002; 41: 4503-4510.
2 Suwanmanee T et al. Repair of a Splicing Defect in Erythroid Cells from Patients with beta-Thalassemia/HbE Disorder. Mol Ther 2002; 6: 718-726.
3 Friedman KJ et al. Correction of aberrant splicing of the cystic fibrosis transmembrane conductance regulator (CFTR) gene by antisense oligonucleotides. J Biol Chem 1999; 274: 36193-36199.
4 Mercatante DR, Sazami P, Kole R. Modification of alternative splicing by antisense oligonucleotides as a potential chemotherapy for cancer and other diseases. Curr Cancer Drug Targets 2001; 1: 211-230.
5 Cartegni L, Krainer AR. Correction of disease-associated exon skipping by synthetic exon-specific activators. Nat Struct Biol 2003; 10: 120-125.
6 Hoffman EP, Brown RH, Jr., Kunkel LM. Dystrophin: the protein product of the Duchenne muscular dystrophy locus. Cell 1987; 51: 919-928.
7 Hoffman EP et al. Characterization of dystrophin in muscle-biopsy specimens from patients with Duchenne's or Becker's muscular dystrophy. N Engl J Med 1988; 318: 1363-1368.
8 Koenig M et al. The molecular basis for Duchenne versus Becker muscular dystrophy: correlation of severity with type of deletion. Am J Hum Genet 1989; 45: 498-506.
9 Ervasti JM et al. Deficiency of a glycoprotein component of the dystrophin complex in dystrophic muscle. Nature 1990; 345: 315-319.
10 Di Blasi C et al. Dystrophin-associated protein abnormalities in dystrophin-deficient muscle fibers from symptomatic and asymptomatic Duchenne/Becker muscular dystrophy carriers. Acta Neuropathol (Berl) 1996; 92: 369-377.
11 Koenig M, Monaco AP, Kunkel LM. The complete sequence of dystrophin predicts a rod-shaped cytoskeletal protein. Cell 1988; 53: 219-226.
12 Dunckley MG et al. Modification of splicing in the dystrophin gene in cultured Mdx muscle cells by antisense oligoribonucleotides. Hum Mol Genet 1998; 7: 1083-1090.
13 Wilton SD et al. Specific removal of the nonsense mutation from the mdx dystrophin mRNA using antisense oligonucleotides. Neuromuscul Disord 1999; 9: 330-338.
14 De Angelis FG et al. Chimeric snRNA molecules carrying antisense sequences against the splice junctions of exon 51 of the dystrophin pre-mRNA induce exon skipping and restoration of a dystrophin synthesis in Delta 48-50 DMD cells. Proc Natl Acad Sci USA 2002; 99: 9456-9461.
15 Takeshima Y et al. Oligonucleotides against a splicing enhancer sequence led to dystrophin production in muscle cells from a Duchenne muscular dystrophy patient. Brain Dev 2001; 23: 788-790.
16 van Deutekom JC et al. Antisense-induced exon skipping restores dystrophin expression in DMD patient derived muscle cells. Hum Mol Genet 2001; 10: 1547-1554.
17 Aartsma-Rus A et al. Therapeutic antisense-induced exon skipping in cultured muscle cells from six different DMD patients. Hum Mol Genet 2003; 12: 907- 914.
18 Aartsma-Rus A et al. Antisense-induced multiexon skipping for Duchenne muscular dystrophy makes more sense. Am. J Hum Genet 2004; 74: 83-92. Epub 2003 Dec 2016.
19 Lu QL et al. Functional amounts of dystrophin produced by skipping the mutated exon in the mdx dystrophic mouse. Nat Med 2003; 6: 6.
20 Agrawal S. Importance of nucleotide sequence and chemical modifications of antisense oligonucleotides. Biochim Biophys Acta 1999; 1489: 53-68.
21 Manoharan M. Oligonucleotide conjugates as potential antisense drugs with improved uptake, biodistribution, targeted delivery, and mechanism of action. Antisense Nucleic Acid Drug Dev 2002; 12: 103-128.
22 Ekker SC, Larson JD. Morphant technology in model developmental systems. Genesis 2001; 30: 89-93.
23 Summerton J. Morpholino antisense oligomers: the case for an RNase H independent structural type. Biochim Biophys Acta 1999; 1489: 141-158.
24 Morcos PA. Achieving efficient delivery of morpholino oligos in cultured cells. Genesis 2001; 30: 94-102.
25 Schmajuk G, Sierakowska H, Kole R. Antisense oligonucleotides with different backbones. Modification of splicing pathways and efficacy of uptake. J Biol Chem 1999; 274: 21783-21789.
26 Nasevicius A, Ekker SC. Effective targeted gene 'knockdown' in zebrafish. Nat Genet 2000; 26: 216-220.
27 Sazani P et al. Systemically delivered antisense oligomers upregulate gene expression in mouse tissues. Nat Biotechnol 2002; 20: 1228-1233.
28 Gebski BL et al. Morpholino antisense oligonucleotide induced dystrophin exon 23 skipping in mdx mouse muscle. Hum Mol Genet 2003; 12: 1801-1811.
29 Braasch DA, Liu Y, Corey DR. Antisense inhibition of gene expression in cells by oligonucleotides incorporating locked nucleic acids: effect of mRNA target sequence and chimera design. Nucleic Acids Res 2002; 30: 5160-5167.
30 Leumann CJ. DNA analogues: from supramolecular principles to biological properties. Bioorg Med Chem 2002; 10: 841-854.
31 Fluiter K et al. In vivo tumor growth inhibition and biodistribution studies of locked nucleic acid (LNA) antisense oligonucleotides. Nucleic Acids Res 2003; 31: 953-962.
32 Larsen HJ, Bentin T, Nielsen PE. Antisense properties of peptide nucleic acid. Biochim Biophys Acta 1999; 1489: 159-166.
33 Mologni L et al. Additive antisense effects of different PNAs on the in vitro translation of the PML/RARalpha gene. Nucleic Acids Res 1998; 26: 1934-1938.
34 Ray A, Norden B. Peptide nucleic acid (PNA): its medical and biotechnical applications and promise for the future. Faseb J 2000; 14: 1041-1060.
35 Sazani P et al. Nuclear antisense effects of neutral, anionic and cationic oligonucleotide analogs. Nucleic Acids Res 2001; 29: 3965-3974.
36 Karras JG et al. Deletion of individual exons and induction of soluble murine interleukin-5 receptor-alpha chain expression through antisense oligonucleotide-mediated redirection of pre-mRNA splicing. Mol Pharmacol 2000; 58: 380-387.
37 Aartsma-Rus A et al. Targeted exon skipping as a potential gene correction therapy for Duchenne muscular dystrophy. Neuromuscul Disord 2002; 12: S71.
38 Arzumanov A et al. Inhibition of HIV-1 Tat-dependent trans activation by steric block chimeric 2'-O-methyl/LNA oligoribonucleotides. Biochemistry 2001; 40: 14645-14654.

### Example 7

### Multi-exon skipping

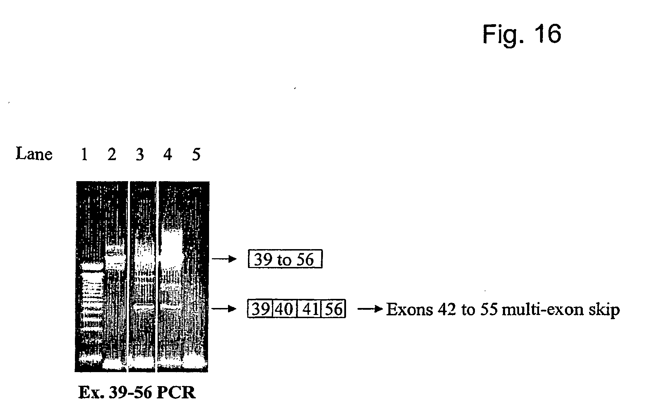

Other examples of frame-restoring multi-exon skipping may include the stretches between exons 17 and 48 (an exon 17-48 deletion is a very large mutation known to be associated with e mild BMD-like phenotype) which would be therapeutic for ∼20% of DMD mutations, exons 19 to 51 (35% applicability), 48 to 59 (18% applicability), or 42 to 55 (65% applicability). Especially the latter combination, included here as another example (Figure 8), would thus apply to large set of patients. We demonstrated the feasibility of multi-exon 42-55 skipping in human control myotubes that were either treated with a mixture h42AON1 and h55AON1 (see Table 2), or with one combined AON carrying both these AONs linked by 10 uracil nucleotides. Following transfection of these mixed or "U-linked AONs" into the myotubes, RT-PCR analysis demonstrated similar efficiencies in inducing the anticipated in-frame transcript with exon 42 spliced to exon 55 (Fig.16). Sequence analysis conformed that this particular multi-exon skipping occurred specifically and precisely at the exon-boundaries (data not shown).

These experiments were performed as described for the multi-exon 45-51 skip (see Material and Methods to example 5)

### Brief description of the drawings

Figure 1. RT-PCR and sequence analysis of dystrophin mRNA fragments of the AON-treated DMD patient myotube cultures, focussing on the regions encompassing the exons targeted for skipping. Shorter, novel transcripts were observed when compared to the untransfected myotube cultures (NT). Sequence analysis confirmed the precise skipping of the targeted exons. An alternatively spliced product, detected for patient 50685.1 (C) was sequenced and found to be derived from activation of a cryptic splice site in exon 51. Shorter fragments, detected in untransfected myotube cultures from DL 363.2 (B), DL 589.2 (D) and 53914.1 (E), were sequenced and found to be the result of the spontaneous skipping of exons 44, 50 and 53, respectively. Note that in some analyses, additional fragments, slightly shorter than the wild-type products, were present. This was due to heteroduplex formation. 100 bp: size marker, -RT-PCR: negative control.
Figure 2. Immuno-histochemical analysis of the AON-treated myotube cultures from the 6 different DMD patients. Cells were stained for myosin to identify fully differentiated myotubes (not shown). Monoclonal antibodies MANDYS1 (middle panel) and Dys2 (right panel) were used to detect dystrophin 1 to 4 days post-transfection. No dystrophin signals could be detected in untreated cells stained with MANDYS1 (left panel) nor Dys2 (not shown), whereas clear, mainly cytoplasmatic, dystrophin signals could be detected for each patient upon the induced exon skipping. In patients DL 363.2 (B), DL 589.2 (D) and 53914.1 (E) dystrophin membrane signals could be observed. We note that membrane signals were more often found for Dys2, which recognizes the full-length dystrophin. MANDYS1 recognizes an internal part of dystrophin and is more prone to generate cytoplasmatic signals, since it also detects dystrophin in the first stages of synthesis. Magnification 63x.
Figure 3. Western blot analysis of the AON-treated myotube cultures. Monoclonal antibody DY4 was used to detect dystrophin. Protein extracts isolated from human control myotube cultures (HC) were used as a positive control (C and F). To avoid overexposure, this sample was 1 to 10 diluted. To demonstrate equal loading of protein samples, blots were additionally stained with an antibody against myosin. No, or, as a result of spontaneous exon skipping, very low (B and C) levels of dystrophin were detected in non-transfected myotube cultures (NT). Clear dystrophin signals were observed in AON-treated myotube cultures for each of the patients. For 50685.1 and DL 363.2, a time-course experiment was performed. Dystrophin could be detected 16 h post-transfection and was found at increasing levels at 24 h and 48 h post-transfection for 50685.1 (D). For DL 363.2 dystrophin could be detected in increasing levels up to 7 days post-transfection (B). For patients DL 515.2 (A), DL 363.2 (B) and DL 589.2 (E) the detected dystrophin was significantly shorter than the control dystrophin. This is due to the size of the deletions in these patients.
Figure 4. Immuno-histochemical analysis of 4 DGC proteins from treated myotube cultures from patient DL 363.2. Cells were stained for myosin to identify sufficiently differentiated myotubes (not shown). Monoclonal antibodies NCL-a-SARC, NCL-b-SARC, NCL-g-SARC and NCL-b-DG were used to detect α-sarcoglycan, ß-sarcoglycan, γ-sarcoglycan and ß-dystroglycan, respectively. These proteins were detected in reduced percentages (∼40%) in untreated myotubes, and were mainly located in the cytoplasm (A). Following AON treatment, however, α-sarcoglycan was detected in 70%, ß-sarcoglycan was detected in 90%, γ-sarcoglycan was detected in 90% and ß-dystroglycan was detected in 80% of the myotubes, and the proteins were mostly membrane-bound (B). Magnification 63x.
Fig. 5. RT-PCR analysis of human dystrophin mRNA in the regions encompassing the exons targeted for skipping. Shorter, novel transcript fragments were observed following transfection with the different AONs when compared to non-transfected myotube cultures (NT). Sequence analysis (not shown) confirmed the skipping of the targeted exons, as indicated by the labels adjacent to the images. Alternatively spliced products, detected in the regions around exon 2 (b), exon 29 (c), and exon 51 (h), were sequenced and found to be derived from either co-skipping of adjacent exons or usage of a cryptic splice site. No specific (RT-) PCR products were obtained. In some analyses, additional fragments, lightly shorter than the wild-type products, were present. This was due to heteroduplex formation.
Figure 6. Double-exon skipping in DMD patient DL90.3 carrying a nonsense mutation in the out-of-frame exon 43. RT-PCR analysis of dystrophin mRNA fragments of AON-treated myotubes from this patient showed a shorter, novel transcript not present in untransfected myotubes (NT). Sequence analysis confirmed the precise skipping of the targeted exons 43 and 44. Besides this double-skip, we also detected a single exon 44 skip. Note that the additional fragment, slightly shorter than the wild-type product, is due to heteroduplex formation. 100 bp: size marker, -RT-PCR: negative control.
Figure 7 Double- and multi-exon skipping in human control myotubes (KM 109), DMD patient DL 470.2, carrying a deletion of exons 46 to 50, and DMD patient 50685.1, carrying a deletion of exons 48 to 50. (A) RT-PCR analysis of dystrophin mRNA fragments in the myotube cultures treated with either a mixture of h45AON5 and h51AON2 (mix) or with a U-linked combination of AONs (U: h45AON5 linked to h51AON2 by 10 uracil nucleotides). In all samples treated with either the mix of AONs or the U-linker AON, a shorter transcript fragment was detected that contained exon 44 spliced to exon 52, and that was not present in untreated myotubes (NT). This novel, in-frame transcript arose from double-exon skipping in patient DL 470.2 (the targeted exons 45 and 51 are directly flanking the deletion), but from multi-exon skipping in both the human control and patient 50685.1. In the treated patient myotube cultures, additional shorter fragments were observed due to single-exon 45 and single-exon 51 skipping. Note that in some lanes, other fragments, slightly shorter than the wild-type products, were present. This was due to heteroduplex formation. 100 bp: size marker, -RT-PCR: negative control. (B) All fragments were quantified using the DNA 7500 labchip® and the Bioanalyzer (Agilent). The percentage of double- or multi-exon 45 to 51 skipping was determined by the ratio of this fragment to the total of transcript fragments. The U-combined AON seems less efficient in DL 470.2, but more efficient in KM 109 and 50685.1, when compared to the mixture of AONs.
Figure 8
   Schematic overview of double and multiexon skipping in three patients with and one human control. AONs are indicated by blue lines. Primers for RT-PCR analysis are indicated by arrows. A, Patient DL90.3 has a nonsense mutation in exon 43 (indicated by an asterisk). The resulting out-of-frame transcript is indicated in red. In contrast to single-exon skipping of exon 43 or 44, double-exon skipping of both exons restores the reading frame (in-frame transcript indicated in green). When myotubes derived from this patient are targeted by AONs specific for exons 43 and 44, single-exon skipping of exon 43 and exon 44 (indicated by red dotted lines) can be expected in addition to the anticipated double-exon skipping. Patient DL470.2 carries an exon 46-50 deletion, which results in a frameshift and a stop codon in exon 51. Single-exon skipping of exon 45 or exon 51 is not frame restoring, whereas double-exon skipping of both exons 45 and 51 is. B, Multiexon skipping of exons 45-51 preserves the reading frame in a control transcript (individual KM109). Patient 50685.1 has a deletion of exons 48-50, resulting in a stop codon in exon 51. Multiexon skipping of exons 45, 46, 47, and 51 restores the reading frame for this patient. In addition to the skipping of exons 45-51, single-exon skipping of exon 45 and exon 51 can also be expected.
Figure 9
   Double-exon skipping in patient DL90.3, who carries a nonsense mutation in the out-of-frame exon 43. A, RT-PCR analysis of dystrophin mRNA fragments in AON-treated myotube cultures showed a shorter, novel transcript not present in nontransfected (NT) myotube cultures. Sequence analysis confirmed the precise skipping of both exon 43 and exon 44. Along with the double skip, we also detected a single skip of exon 44 but not a single-exon skip of exon 43. Note that weak additional fragments, slightly shorter than the wild-type products, were present. These were derived from heteroduplex formation. 100 bppsize marker; RT-PCRpnegative control. B, Immunohistochemical analysis of the AON-treated myotube cultures. Cells were stained for myosin to identify fully differentiated myotubes (lower panel). Monoclonalantibodies MANDYS1 and Dys2 were used to detect dystrophin 2 d after transfection. No dystrophin signals could be detected in untreated cells stained with MANDYS1 (left panel) or Dys2 (not shown), whereas clear dystrophin signals could be detected in treated cells for both dystrophin antibodies. Magnification x 63. C, Western blot analysis of the AON-treated myotube cultures. The monoclonal antibody Dys1 was used to detect dystrophin. Protein extracts isolated from MyoD-transduced human control (HC) fibroblasts were used as a positive control. To avoid overexposure, this sample was diluted 1:5. To confirm equal loading of protein samples, blots were additionally stained with an antibody against myosin. No dystrophin was detected in NT myotube cultures. Clear dystrophin signals were observed in AON-treated myotube cultures after 2 d, which were increased at 4 d.
Figure 10
   Double-exon skipping in patient DL470.2, who carries a deletion of exons 46-50. A, RT-PCR analysis of dystrophin mRNA fragments of AON-treated myotube cultures showed a shorter, novel transcript not present in NT myotube cultures. The precise skipping of both exon 45 and exon 51 was confirmed by sequence analysis. Along with the double skip, we also detected a single skip of exon 51 but no single skip of exon 45. Because of heteroduplex formation, we observed weak additional fragments, slightly shorter than the wild-type products. 100 bp = size marker; RT-PCR = negative control. B, Immunohistochemical analysis of the AON-treated myotube cultures. Cells were stained for myosin to identify fully differentiated myotubes (lower panel). Monoclonal antibodies MANDYS1 and Dys2 were used to detect dystrophin 2 d after transfection. In treated cells, clear dystrophin signals could be detected for both antibodies, in contrast to untreated cells stained with MANDYS1 (left panel) or Dys2 (not shown). Magnification x 63. C, Western blot analysis of the AON-treated myotube cultures. Monoclonal antibody Dys1 was used to detect dystrophin. Protein extracts isolated from human control myotubes were used as a positive control, which was diluted 1: 10 to avoid overexposure. Blots were additionally stained with an antibody against myosin to confirm equal loading of all samples. No dystrophin was detected in NT myotube cultures, whereas clear dystrophin signals were observed in AON-treated myotube cultures after 1 d, which increased after 2 d. Note that the dystrophin from patient DL470.2 is shorter than the control dystrophin. This correlates with the size of the deletion.
Figure 11
   RT-PCR analysis of the entire DMD transcript from RNA, isolated from untreated (-) and treated (+) myotubes from both patients. The anticipated specific-exon skipping patterns are present in the fragments containing exons 34-45 (patient DL90.3) and 42-53 (patients DL90.3 and DL470.2). Note that because of the deletion of exons 46-50, the wild-type fragment for this patient is shorter than that of patient DL90.3. No unexpected splicing anomalies were detected in other parts of the DMD gene, confirming the sequence specificity of the AONs. "M" is the 100-bp size marker.
Figure 12
   Double and multi-exon skipping in myotubes from the human control (individual KM109) and from patients DL470.2 (deletion of exons 46-50) and 50685.1 (deletion of exons 48-50). A, RT-PCR analysis of dystrophin mRNA fragments in myotube cultures treated with either a mixture of h45AON5 and h51AON2 (mix) or a U-linked combination of these AONs (U). In all samples treated with either the mixture of AONs or the U-linked AON, a shorter transcript fragment was detected that contained exon 44 precisely spliced to exon 52 (confirmed by sequence analysis; data not shown). This was not present in NT myotubes. The novel in-frame transcript arose from double-exon skipping in patient DL470.2 (the targeted exons 45 and 51 are flanking the deletion), but in both the control and patient 50685.1 the transcript was a result of multiexon skipping. Also observed were additional shorter fragments, which were caused by single-exon skipping (exon 45 or 51) or partial multi-exon skipping (exons 46-51). Note that in some lanes, other fragments, slightly shorter than the wild-type products, were present. This was a result of heteroduplex formation. 100 bp = size marker; RT-PCR = negative control. B, Schematic drawing of the U-linked AON. The exon 51-specific AON (h51AON2) is linked to the exon 45-specific AON (h45AON5) by 10 uracil nucleotides. C, All fragments were quantified using the DNA 1000 LabChip and the Bioanalyzer (Agilent). The percentage of double or multi-exon skipping of exons 45-51 was determined by the ratio of this fragment to the total of transcript fragments. In contrast to patient DL470.2, the U-linked AON was more efficient for patients KM109 and 50685.1, when compared to the mixture of individual AONs.
Figure 13
   Comparative analysis of DNA, 2OMePS, morpholino, LNA and PNA
   AONs directed at exon 46.
   A) Gel mobility shift assay with a radiolabeled exon 46 RNA fragment hybridized to the different analogs. A clear mobility shift was observed for the AONs with the DNA and LNA backbone, which is less prominent for the 2OMePS AON. No shift was observed for the morpholino, the PNA or a random AON.
   B) Transfection of the different AON analogs into control myotubes. Efficiencies of over 80% were obtained for the 2OMePS, the morpholino and the LNA AONs, whereas for the PNAs efficiencies were 60% to 70%. In contrast to the other AON analogs, the morpholino was also abundant in the cytoplasm.
   C) RT-PCR analysis of dystrophin mRNA fragments from unaffected control and DMD patient-derived (DL279.1) myotubes following transfection of the different AON analogs. With the exception of the PNA, exon 46 skipping was detected for each analog, both in patient and control myotubes. The precise skipping of exon 46 was confirmed by sequence analysis (data not shown). 100 bp is the DNA size marker.
   D) Quantification of RT-PCR products. The percentage of exon 46 skipping was determined by the ratio of the shorter fragment to the total of transcript fragments, and is shown above each column. The LNA induced highest levels of exon skipping in both unaffected control (85%) and patient-derived (98%) myotubes. The 2OMePS AON was remarkably more efficient in the patient-derived myotubes when compared to the control (75% vs. 20%, respectively). The morpholino was only moderately effective (5-6%) both in control and patient-derived myotubes.
Figure 14.
   Concentration series of LNA8 in control and patient-derived (DL279.1) myotube cultures.
   A) RT-PCR analysis of dystrophin mRNA fragments. Significant levels of exon 46 skipping were observed at each dose tested for the patient (DL279.1), whereas only low levels were detected for a dose of 200 nM and 100 nM in the human control. In control myotubes skipping of both exon 45 and 46 was sometimes observed at the highest doses (400 and 500 nM). For some DL279.1 fragments, products slightly larger than the wild type fragments can be observed. This is due to heteroduplex formation of first and second PCR products. 100 bp is the DNA size marker.
   B) Quantification of the RT-PCR fragments showed that the levels of exon skipping decrease considerably at doses below 500 nM for the human control (from 97% at 500 nM to 30% at 400 nM and below 1% at 100 nM), but remain high (86%) at a dose of 300 nM for the patient and still significant (10%) at a 100 nM concentration.
Figure 15.
   Analysis of LNA and 2OMePS AONs containing mismatches in patientderived myotubes.
   A) Sequences of the different LNAs aligned to their target sequence in exon 46 (depicted at the top from 3' to 5'). Mismatches to the target sequence are underlined. LNA9 is shifted to the 3' side of the target sequence when compared with the original LNA (LNA8) and is completely homologous to the target sequence.
   B) RT-PCR analysis and C) quantification of dystrophin mRNA fragments from LNA treated patient (DL279.1) myotubes. The LNAs containing mismatches in the 3' end (LNAmm1 and 4) induced exon 46 skipping to levels that are comparable to those induced by the original LNA (71%-94% vs. 100%). The mismatches in the 5' or central part (LNAmm2, 3 and 5) seriously reduced the skipping capacity (<8%). Fragments slightly larger than the wild type band were observed. This is due to heteroduplex formation between first and second PCR products. 100 bp is the DNA size marker.
   D) Sequences of the different 2OMePS AONs aligned to the target sequence in exon 46 (depicted at the top from 3' to 5'). Mismatches to the target sequence are underlined.
   E) RT-PCR analysis and F) quantification of dystrophin mRNA fragments from 2OMePS treated patient (DL279.1) myotubes. The presence of 1 mismatch at the 3' side (2OMePSmm1) already results in an over 2.5 fold reduction in exon skipping levels, while even more reduced levels are found for the AONs containing 2 and 3 mismatches (2OMePSmm2 and 3). Virtually no skipping was induced by the AONs that contain a single mismatch at the 5' part (2OMePSmm4) or a single mismatch in the 3', central as well as the 5' part (2OMePSmm5) of the oligo. 100 bp is the DNA size marker.
Figure 16
   Multi-exon 42 to 55 skipping in human control myotubes (KM 109). RT-PCR analysis of dystrophin mRNA fragments in the myotube cultures treated with either a mixture of h42AON1 and h55AON1 (Lane 4: mix) or with a U-linked combination of these AONs (Lane 3, U: h42AON1 linked to h55AON1 by 10 uracil nucleotides). In all samples treated with either the mix of AONs or the U-linker AON, a shorter transcript fragment was detected that contained exon 42 spliced to exon 55, and that was not present in untreated myotubes (Lane 2 "NT"). This novel, in-frame transcript arose from multi-exon skipping of a stretch of exons between 42 and 55. Additional shorter fragments were observed due to additional alternative splicing patterns. In addition, note that in some lanes, other fragments, slightly shorter than the wild-type products, were present. This was due to heteroduplex formation. Lane (1) =100 bp: size marker, Lane 5= -RT-PCR: negative control.

### References (to the general part, excluding example 2, 5 and 6)

1. Hoffman, E. P., Brown, R. H., Jr., Kunkel, L. M. (1987) Dystrophin: the protein product of the Duchenne muscular dystrophy locus. Cell, 51, 919-928.
2. Hoffman, E. P., Fischbeck, K. H., Brown, R. H., Johnson, M., Medori, R., Loike, J. D., Harris, J. B., Waterston, R., Brooke, M., Specht, L., et al. (1988) Characterization of dystrophin in muscle-biopsy specimens from patients with Duchenne's or Becker's muscular dystrophy. N. Engl. J. Med., 318, 1363-1368.
3. Den Dunnen, J. T., Grootscholten, P. M., Bakker, E., Blonden, L. A., Ginjaar, H. B., Wapenaar, M. C., van Paassen, H. M., van Broeckhoven, C., Pearson, P. L., van Ommen, G. J. (1989) Topography of the Duchenne muscular dystrophy (DMD) gene: FIGE and cDNA analysis of 194 cases reveals 115 deletions and 13 duplications. Am. J. Hum. Genet., 45, 835-847.
4. Koenig, M., Beggs, A. H., Moyer, M., Scherpf, S., Heindrich, K., Bettecken, T., Meng, G., Muller, C. R., Lindlof, M., Kaariainen, H., et al. (1989) The molecular basis for Duchenne versus Becker muscular dystrophy: correlation of severity with type of deletion. Am. J. Hum. Genet., 45, 498-506.
5. Tuffery-Giraud, S., Chambert, S., Demaille, J., Claustres, M. (1999) Point mutations in the dystrophin gene: evidence for frequent use of cryptic splice sites as a result of splicing defects. Hum. Mutat., 14, 359-368.
6. Prior, T. W., Bartolo, C., Pearl, D. K., Papp, A. C., Snyder, P. J., Sedra, M. S., Burghes, A. H., Mendell, J. R. (1995) Spectrum of small mutations in the dystrophin coding region. Am. J. Hum. Genet., 57, 22-33.
7. Moser, H. (1984) Duchenne muscular dystrophy: pathogenetic aspects and genetic prevention. Hum. Genet., 66, 17-40.
8. Emery, A. E. (2002) The muscular dystrophies. Lancet, 359, 687-695.
9. Yoshida, M., Ozawa, E. (1990) Glycoprotein complex anchoring dystrophin to sarcolemma. J. Biochem. (Tokyo), 108, 748-752.
10. Ervasti, J. M., Campbell, K. P. (1991) Membrane organization of the dystrophin-glycoprotein complex. Cell, 66, 1121-1131.
11. Di Blasi, C., Morandi, L., Barresi, R., Blasevich, F., Cornelio, F., Mora, M. (1996) Dystrophin-associated protein abnormalities in dystrophin-deficient muscle fibers from symptomatic and asymptomatic Duchenne/Becker muscular dystrophy carriers. Acta Neuropathol. (Berl), 92, 369-377.
12. Ervasti, J. M., Ohlendieck, K., Kahl, S. D., Gaver, M. G., Campbell, K. P. (1990) Deficiency of a glycoprotein component of the dystrophin complex in dystrophic muscle. Nature, 345, 315-319.
13. Matsumura, K., Burghes, A. H., Mora, M., Tome, F. M., Morandi, L., Cornello, F., Leturcq, F., Jeanpierre, M., Kaplan, J. C., Reinert, P., et al. (1994) Immunohistochemical analysis of dystrophin-associated proteins in Becker/Duchenne muscular dystrophy with huge in-frame deletions in the NH2-terminal and rod domains of dystrophin. J. Clin. Invest., 93, 99-105.
14. Monaco, A. P., Bertelson, C. J., Liechti-Gallati, S., Moser, H., Kunkel, L. M. (1988) An explanation for the phenotypic differences between patients bearing partial deletions of the DMD locus. Genomics, 2, 90-95.
15. Clemens, P. R., Duncan, F. J. (2001) Progress in gene therapy for Duchenne muscular dystrophy. Curr. Neurol. Neurosci. Rep., 1, 89-96.
16. Khan, M. A. (1993) Corticosteroid therapy in Duchenne muscular dystrophy. J. Neurol. Sci., 120, 8-14.
17. De Angelis, F. G., Sthandier, O., Berarducci, B., Toso, S., Galluzzi, G., Ricci, E., Cossu, G., Bozzoni, I. (2002) Chimeric snRNA molecules carrying antisense sequences against the splice junctions of exon 51 of the dystrophin pre-mRNA induce exon skipping and restoration of a dystrophin synthesis in Delta 48-50 DMD cells. Proc. Natl. Acad. Sci. USA, 99, 9456-9461.
18. Mann, C. J., Honeyman, K., Cheng, A. J., Ly, T., Lloyd, F., Fletcher, S., Morgan, J. E., Partridge, T. A., Wilton, S. D. (2001) Antisense-induced exon skipping and synthesis of dystrophin in the mdx mouse. Proc. Natl. Acad. Sci. USA, 98, 42-47.
19. van Deutekom, J. C., Bremmer-Bout, M., Janson, A. A., Ginjaar, I. B., Baas, F., den Dunnen, J. T., van Ommen, G. J. (2001) Antisense-induced exons skipping restores dystrophin expression in DMD patient derived muscle cells. Hum. Mol. Genet., 10, 1547-1554.
20. Wilton, S. D., Lloyd, F., Carville, K., Fletcher, S., Honeyman, K., Agrawal, S., Kole, R. (1999) Specific removal of the nonsense mutation from the mdx dystrophin mRNA using antisense oligonucleotides. Neuromuscul. Disord., 9, 330-338.
21. Dunckley, M. G., Manoharan, M., Villiet, P., Eperon, I. C., Dickson, G. (1998) Modification of splicing in the dystrophin gene in cultured Mdx muscle cells by antisense oligoribonucleotides. Hum. Mol. Genet., 7, 1083-1090.
22. Takeshima, Y., Wada, H., Yagi, M., Ishikawa, Y., Minami, R., Nakamura, H., Matsuo, M. (2001) Oligonucleotides against a splicing enhancer sequence led to dystrophin production in muscle cells from a Duchenne muscular dystrophy patient. Brain Dev., 23, 788-790.
23. Aartsma-Rus, A., Bremmer-Bout, M., Janson, A., den Dunnen, J., van Ommen, G., van Deutekom, J. (2002) Targeted exon skipping as a potential gene correction therapy for Duchenne muscular dystrophy. Neuromuscul. Discord., 12, S71.
24. Shiga, N., Takeshima, Y., Sakamoto, H., Inoue, K., Yokota, Y., Yokoyama, M., Matsuo, M. (1997) Disruption of the splicing enhancer sequence within exon 27 of the dystrophin gene by a nonsense mutation induces partial skipping of the exon and is responsible for Becker muscular dystrophy. J. Clin. Invest., 100, 2204-2210.
25. Cartegni, L., Chew, S. L., Krainer, A. R. (2002) Listening to silence and understanding nonsense: exonic mutations that affect splicing. Nat. Rev. Genet., 3, 285-298.
26. Schaal, T. D., Maniatis, T. (1999) Multiple distinct splicing enhancers in the protein-coding sequences of a constitutively spliced pre-mRNA. Mol. Cell. Biol., 19, 261-273.
27. Takeshima, Y., Nishio, H., Sakamoto, H., Nakamura, H., Matsuo, M. (1995) Modulation of in vitro splicing of the upstream intron by modifying an intra-exon sequence which is deleted from the dystrophin gene in dystrophin Kobe. J. Clin. Invest., 95, 515-520.
28. Pramono, Z. A., Takeshima, Y., Alimsardjono, H., Ishii, A., Takeda, S., Matsuo, M. (1996) Induction of exon skipping of the dystrophin transcript in lymphoblastoid cells by transfecting an antisense oligodeoxynucleotide complementary to an exon recognition sequence. Biochem. Biophys. Res. Commun., 226, 445-449.
29. Koenig, M., Monaco, A. P., Kunkel, L. M. (1988) The complete sequence of dystrophin predicts a rod-shaped cytoskeletal protein. Cell, 53, 219-226.
30. Den Dunnen, J. (1996) The Leiden Muscular Dystrophy pages; http://www.dmd.nl.
31. Mann, C. J., Honeyman, K., McClorey, G., Fletcher, S., Wilton, S. D. (2002) Improved antisense oligonucleotide induced exon skipping in the mdx mouse model of muscular dystrophy. J. Gene Med., 4, 644-654.
32. Kerr, T. P., Sewry, C. A., Robb, S. A., Roberts, R. G. (2001) Long mutant dystrophins and variable phenotypes: evasion of nonsense-mediated decay? Hum. Genet., 109, 402-407.
33. Klein, C. J., Coovert, D. D., Bulman, D. E., Ray, P. N., Mendell, J. R., Burghes, A. H. (1992) Somatic reversion/suppression in Duchenne muscular dystrophy (DMD): evidence supporting a frame-restoring mechanism in rare dystrophin-positive fibers. Am. J. Hum. Genet., 50, 950-959.
34. Sherratt, T. G., Vulliamy, T., Dubowitz, V., Sewry, C. A., Strong, P. N. (1993) Exon skipping and translation in patients with frameshift deletions in the dystrophin gene. Am. J. Hum. Genet., 53, 1007-1015.
35. Lu, Q. L., Morris, G. E., Wilton, S. D., Ly, T., Artem'yeva, O. V., Strong, P., Partridge, T. A. (2000) Massive idiosyncratic exon skipping corrects the nonsense mutation in dystrophic mouse muscle and produces functional revertant fibers by clonal expansion. J. Cell Biol., 148, 985-996.
36. Nicholson, L. V., Johnson, M. A., Bushby, K. M., Gardner-Medwin, D. (1993) Functional significance of dystrophin positive fibres in Duchenne muscular dystrophy. Arch. Dis. Child, 68, 632-636.
37. Vainzof, M., Passos-Bueno, M. R., Takata, R. I., Pavanello Rde, C., Zatz, M. (1993) Intrafamilial variability in dystrophin abundance correlated with difference in the severity of the phenotype. J. Neurol. Sci., 119, 38-42.
38. Singh, V., Sinha, S., Mishra, S., Chaturvedi, L. S., Pradhan, S., Mittal, R. D., Mittal, B. (1997) Proportion and pattern of dystrophin gene deletions in north Indian Duchenne and Becker muscular dystrophy patients. Hum. Genet., 99, 206-208.
39. Melacini, P., Fanin, M., Danieli, G. A., Fasoli, G., Villanova, C., Angelini, C., Vitiello, L., Miorelli, M., Buja, G. F., Mostacciuolo, M. L., et al. (1993) Cardiac involvement in Becker muscular dystrophy. J. Am. Coll. Cardiol., 22, 1927-1934.
40. Melis, M. A., Cau, M., Muntoni, F., Mateddu, A., Galanello, R., Boccone, L., Deidda, F., Loi, D., Cao, A. (1998) Elevation of serum creatine kinase as the only manifestation of an intragenic deletion of the dystrophin gene in three unrelated families. Europ. J. Paediatr. Neurol., 2, 255-261.
41. Onengut, S., Kavaslar, G. N., Battaloglu, E., Serdaroglu, P., Deymeer, F., Ozdemir, C., Calafell, F., Tolun, A. (2000) Deletion pattern in the dystrophin gene in Turks and a comparison with Europeans and Indians. Ann. Hum. Genet., 64, 33-40.
42. Rosenberg, C., Navajas, L., Vagenas, D. F., Bakker, E., Vainzof, M., Passos-Bueno, M. R., Takata, R. I., Van Ommen, G. J., Zatz, M., Den Dunnen, J. T. (1998) Clinical diagnosis of heterozygous dystrophin gene deletions by fluorescence in situ hybridization. Neuromuscul. Disord., 8, 447-452.
43. Sertic, J., Barisic, N., Sostarko, M., Brzovic, Z., Stavljenic-Rukavina, A. (1997) Deletion screening of the Duchenne/Becker muscular dystrophy gene in Croatian population. Coll. Antropol., 21, 151-156.
44. Rando, T. A., Blau, H. M. (1994) Primary mouse myoblast purification, characterization, and transplantation for cell-mediated gene therapy. J. Cell Biol., 125, 1275-1287.
45. Murry, C. E., Kay, M. A., Bartosek, T., Hauschka, S. D., Schwartz, S. M. (1996) Muscle differentiation during repair of myocardial necrosis in rats via gene transfer with MyoD. J. Clin. Invest., 98, 2209-2217.
46. Roest, P. A., van der Tuijn, A. C., Ginjaar, H. B., Hoeben, R. C., Hoger-Vorst, F. B., Bakker, E., den Dunnen, J. T., van Ommen, G. J. (1996) Application of in vitro Myo-differentiation of non-muscle cells to enhance gene expression and facilitate analysis of muscle proteins. Neuromuscul. Disord., 6, 195-202.
47. Havenga, M. J., Lemckert, A. A., Ophorst, O. J., van Meijer, M., Germeraad, W. T., Grimbergen, J., van Den Doel, M. A., Vogels, R., van Deutekom, J., Janson, A. A., et al. (2002) Exploiting the natural diversity in adenovirus tropism for therapy and prevention of disease. J. Virol., 76, 4612-4620.
48. Anderson, L. V., Davison, K. (1999) Multiplex Western blotting system for the analysis of muscular dystrophy proteins. Am. J. Pathol., 154, 1017-1022.
49. Neugebauer, KM, et al., J Cell Biol 129:899-908 (1995).
50. Tacke R and Manley JL, Proc Soc Exp Biol Med 220:59-63 (1999).
51. Graveley BR et al., Curr Biol 9:R6-7 (1999).
52. Misteli T et al., Nature 387:523-527 (1997).
53. Gorman L, Suter D, Emerick V, et al. Stable alteration of pre mRNA splicing patterns by modified U7 small nuclear RNAs. Proc Natl Acad Sci USA 1998;95:4929 4934.
54. Suter D, Tomasini R, Reber U, et al. Double target antisense U7 snRNAs promote efficient skipping of an aberrant exon in three human beta thalassemic mutations. Hum Mol Genet 1999;8:2415 2423

**Table 1.**

| Overview of the patients, the AONs and the primer sets used in this study | | | | | | |
|---|---|---|---|---|---|---|
| **Patients** | **Mutations** | **Targeted exons** | **AONs^{a}** | **RT-primers^{b}** | **Primary PCR sets^{b}** | **Nested PCR sets^{b}** |
| DL 515.2 | Deletion exon 45-50 | Exon 51 | h51AON1 | h53r | h41f-h53r | h42f-h52r |
| DL 363.2 | Deletion exon 45-54 | Exon 44 | h44AON1 | h55r2 | h42f-h55r2 | h44f-h55r |
| 50685.1 | Deletion exon 48-50 | Exon 51 | h51AON1 | h53r | h46f-h53r | h47f-h52r |
| DL 589.2 | Deletion exon 51-55 | Exon 50 | h50AON1 | h58r | h47f-h58r | h49f-h57r |
| 53914.1 | Deletion exon 52 | Exon 51 | h51AON1 | h55r | h49f-h55r | h50f-h54r |
| | " | Exon 53 | h53AON1 | " | " | " |
| 50423.1 | Point mutation exon 49 | Exon 49 | h49AON1 | h52r | h46f-h52r | h47f-h51r |

| | | | | | | |
|---|---|---|---|---|---|---|
| ^{a}AON sequences were published previously (23). ^{b}Primer sequences available upon request. | | | | | | |

**Table 2**

| Characteristics of the AONs used to study the targeted skipping of 15 different DMD exons^{a} | | | | | | |
|---|---|---|---|---|---|---|
| Name | Antisense sequence (5'-3') | Length (bp) | G/C% | U/C% | Exon skip | Transcript |
| h2AON 1 | cccauuuugugaauguuuucuuuu | 24 | 29 | 75 | + | OF |
| h2AON 2 | uugugcauuuacccauuuugug | 22 | 36 | 68 | - | OF |
| h17AON1 | ccauuacaguugucuguguu | 20 | | | + | |
| h17AON2 | uaaucugccucuucuuuugg | 20 | | | + | |
| h29AON 1 | uauccucugaaugucgcauc | 20 | 45 | 65 | + | IF |
| h29AON 2 | gguuauccucugaaugucgc | 20 | 50 | 60 | + | IF |
| h40AON 1 | gagccuuuuuucuucuuug | 19 | 37 | 79 | + | IF |
| h40AON 2 | uccuuucgucucugggcuc | 19 | 58 | 79 | + | IF |
| h41AON 1 | cuccucuuucuucuucugc | 19 | 47 | 95 | + | IF |
| h41AON 2 | cuucgaaacugagcaaauuu | 20 | 35 | 50 | + | IF |
| h42AON 1 | cuugugagacaugagug | 17 | 47 | 41 | + | IF |
| h42AON 2 | cagagacuccucuugcuu | 18 | 50 | 67 | + | IF |
| h43AON 1 | ugcugcugucuucuugcu | 18 | 50 | 78, | - | OF |
| h43AON 2 | uuguuaacuuuuucccauu | 19 | 26 | 79 | + | OF |
| h43AON5 | cuguagcuucacccuuucc | 19 | | | + | |
| h44AON 1 | cgccgccauuucucaacag | 19 | 58 | 63 | + | OF |
| h44AON 2 | uuuguauuuagcauguuccc | 20 | 35 | 70 | + | OF |
| h45AON 1 | gcugaauuauuucuucccc | 19 | 42 | 74 | - | OF |
| h45AON 5 | gcccaaugccauccugg | 17 | 65 | 58 | + | OF |
| h46AON 4b | cugcuuccuccaacc | 15 | 60 | 80 | + | OF |
| h46AON 8b | gcuuuucuuuuaguugcugc | 20 | 40 | 75 | + | OF |
| h46AON26: | agguucaagugggauacua | 19 | 42 | 37 | + | OF |
| h47AON 1 | ucuugcucuucugggcuu | 18 | 50 | 78 | - | IF |
| h47AON 2 | cuugagcuuauuuucaaguuu | 21 | 29 | 67 | - | IF |
| h48AON 1 | uuucuccuuguuucuc | 16 | 38 | 94 | - | IF |
| h48AON 2 | ccauaaauuuccaacugauuc | 21 | 33 | 62 | - | IF |
| h48AON6 | gcuucaauuucuccuuguu | 19 | | | + | |
| h48AON7 | uuuauuugagcuucaauuu | 19 | | | + | |
| h49AON 1 | cuuccacauccgguuguuu | 19 | 47 | 74 | + | IF |
| h49AON 2 | guggcugguuuuuccuugu | 19 | 47 | 68 | + | IF |
| h50AON 1 | cucagagcucagaucuu | 17 | 47 | 59 | + | OF |
| h50AON 2 | ggcugcuuugcccuc | 15 | 67 | 73 | - | OF |
| h51AON 1 | ucaaggaagauggcauuucu | 20 | 40 | 45 | + | OF |
| h51AON 2 | ccucugugauuuuauaacuugau | 23 | 30 | 65 | + | OF |
| h53AON 1 | cuguugccuccgguucug | 18 | 61 | 72 | + | OF |
| h53AON 2 | uuggcucuggccuguccu | 18 | 61 | 72 | - | OF |
| h55AONl | cuguugcaguaaucuaugag | 20 | | | + | |
| h55AON6 | gagucuucuaggagccuu | 13 | | | + | |
| h59AON2 | uugaaguuccuggagucuu | 19 | | | + | |

| | | | | | | |
|---|---|---|---|---|---|---|
| a Two AONs were tested per exon. Their different lengths and G/C contents (%) did not correlate to their effectivity in exon skipping (1, induced skipping, 2, no skipping). The AONs were directed to purine (A/G) - rich sequences as indicated by their (antisense) U/C content (%). Skipping of the target exons resulted in either an in-frame (IF) or an out-of-frame (OF) transcript. b van Deutekom et al., 2001 [21]. | | | | | | |

**Table 3**

| Primer sets used for the RT-PCR analyses to detect the skipping of the targeted exons^{a} | | | |
|---|---|---|---|
| Target exon | RT-primer | Primary PCR primer set | Nested PCR primer set |
| 2 | h4r | h1f1-h4r | h1f2-h3r |
| 2 | h9r | hlfl-h9r | hlf2-h8r |
| 29 | h31r | h25f-h31r | h26f-h30r |
| 40 | h44r | h38f-h44r | h39f-h43r |
| 41 | h44r | h38f-h44r | h39f-h43r |
| 42 | h44r | h38f-h44r | h39f-h43r |
| 43 | h47r | h41f-h47r | h42f-h46r |
| 44 | h47r | h41f-h47r | h42f-h46r |
| 45 | h47r | h41f-h47r | h42f-h46r |
| 46 | h48r | h44f-h48r | h45f-h47r |
| 47 | h52r | h44f-h52r | h46f-h51r |
| 48 | h52r | h44f-h52r | h46f-h51r |
| 49 | h52r | h44f-h52r | h46f-h51r |
| 50 | h52r | h44f-h52r | h46f-h51r |
| 51 | h53r | h47f-h53r | h49f-h52r |
| 53 | h55r | h50f-h55r | h51f-h54r |

| | | | |
|---|---|---|---|
| ^{a} Primer sequences are available upon request. | | | |

**Table 4**

| Overview and frequency of the DMD-causing mutations in the Leiden DMD (LDMD) Database, theoretically correctable by skipping one of the 12 exons successfully targeted in this study | | | | | |
|---|---|---|---|---|---|
| Skippable exon | Therapeutic for DMD-mutations: | | | | |
| | Deletions (exons) (exons) | % of deletions in LDMD Database | Duplications (exons) | % of duplications in LDMD Database | No. of nonsense mutations in LDMD Database |
| 2 | 3-7, 3-19, 3-21 | 2.9 | 2 | 9.0 | |
| 29 | | | | | 5 |
| 40 | | | | | 1 |
| 41 | | | | | 4 |
| 42 | | | | | 0 |
| 43 | 44, 44-47, 44-49, 44-51 | 3.7 | 43 | 3.0 | |
| 44 | 5-43, 14-43, 19-43, 30-43, 35-43, 36-43, 40-43, 42-43, 45, 45-54 | 7.8 | 44 | 3.0 | |
| 46 | 21-45, 45, 47-54, 47-56 | 5.6 | | | |
| 49 | | | | | 1 |
| 50 | 51, 51-53, 51-55 | 5.2 | 50 | 3.0 | |
| 51 | 45-50, 47-50, 48-50, 49-50, 50, 52, 52-63 | 17.5 | 51 | 1.5 | |
| 53 | 10-52, 45-52, 46-52, 47-52, 48-52, 49-52, 50-52, 52 | 7.5 | | | |

**Table 5.**

| Overview of the patients, the AONs and the primer sets used in example 3 | | | | | | |
|---|---|---|---|---|---|---|
| **Patients** | **Mutations** | **Targeted exons** | **AONs** | **RT-rimers^{b}** | **Primary PCR primer sets^{b}** | **Nested PCR primer sets^{b}** |
| DL 90.3 | Nonsense mutation exon | Exon 43 | h43AON2^{a} | h48r | h41f-h48r | h42f-h47r |
| | 43 | Exon 44 | h44AON1^{a} | | | |
| DL470.2 | Deletion exon 46-50 | Exon 45 | h45AON5 | h53r | h42f-h53r | h43-h52r |
| | | Exon 51 | h51AON2^{a} | | | |
| | | Exon 45 | U-linker | h53r | h42f-h53r | h43f-h52r |
| | | Exon 51 | AON^{c} | | | |

| | | | | | | |
|---|---|---|---|---|---|---|
| ^{a}Seperate AON sequences were published previously [Aartsma-Rus, 2002]. ^{b}Primer sequences available upon request. ^{c}U linker AON consists of h45AON5 linked to h51AON2 by 10 uracils. | | | | | | |

**Table 6**

| Table 1. Characteristics of the AON analogs used in this study | | | | | |
|---|---|---|---|---|---|
| Backbone | Sequence (5' to 3') | Charge | Length | Predicted Tm³ | Monomer³ |
| 2'O-methyl phosphorothioate RNA (20MePS) | 6-FAM-GCUUUUCUUUUAGUUGCUGC | Negative | 20 bp | 44.9 °C | 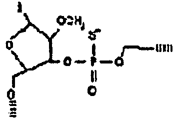 |
| Morpholino-phosphorodiamidate DNA (morpholino) | 6-FAM-GCTTTTCTTTTAGTTGCTGCTC (Linked to sense DNA leash for EPEI transfection) | Uncharged | 22 bp | ∼72-77 °C | 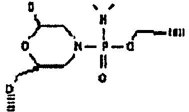 |
| Locked nucleic acid DNA (LNA) | 6-FAM-CTTTTAGTTGCTGC | Negative | 14 bp | 131 °C | 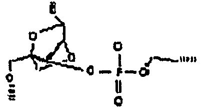 |
| Peptide nucleic acid (PNA) | 6-FAM-CTTTTAGTTGCTGC-(Lys)₄ | Positive | 14 bp | ∼61-66 °C | 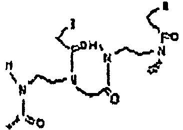 |

| | | | | | |
|---|---|---|---|---|---|
| ^{a}Tm is melting temperature; ^{b}Chemical structure of the backbone. B is base (A, C, G, T, U) | | | | | |

## Claims

1. A method for generating an oligonucleotide comprising determining, from a secondary structure of RNA from an exon, a region that is hybridised to another part of said RNA (closed structure) and a region that is not hybridised in said structure (open structure), and subsequently generating an oligonucleotide, of which at least three consecutive nucleotides of said oligonucleotide are complementary to said closed structure and of which at least another three consecutive nucleotides of said oligonucleotide are complementary to said open structure, and wherein the gene from which said RNA comprising said exon is transcribed, encodes a Duchenne muscular dystrophy gene (DMD), a collagen VI alpha 1 gene (COL6A1), a myotubular myopathy 1 gene (MTM1), a dysferlin gene (DYSF), a laminin-alpha 2 gene (LAMA2), an emery-dreyfuss muscular dystrophy gene (EMD), and/or a calpain 3 gene (CAPN3).

2. A method according to claim 1, wherein said open and closed structures are adjacent to each other.

3. A method according to claim 1 or claim 2, wherein said oligonucleotide is complementary to a consecutive part of between 14 and 50 nucleotides of said RNA.

4. A method according to any one of claims 1-3, wherein said oligonucleotide comprises RNA.

5. A method according to claim 4, wherein said RNA contains a modification, preferably an 2'-O-methyl modified ribose (RNA) or deoxyribose (DNA) modification.

6. A method according to any one of claims 1-5, wherein pre-mRNA comprising said exon exhibits undesired splicing in a subject.

7. A method according to claim 6, wherein the absence of said exon from mRNA produced from said pre-mRNA, generates a coding region for a protein.

8. A method according to claim 6 or claim 7, wherein said gene is the Duchenne muscular dystrophy gene.

9. A method according to claim 8, wherein said exon comprises exon 2, 8, 9, 17, 19, 29, 40-46, 48-53, 55 or 59.

10. A method according to any one of claims 1-8, wherein an equivalent of said oligonucleotide is generated comprising similar hybridisation characteristics in kind not necessarily in amount.

11. A method according to claim 10, wherein said equivalent comprises peptide nucleic acid, locked nucleic acid and/or a morpholino phosphorodiamidate, or a combination thereof.

12. A method according to claim 11, wherein said equivalent comprises locked nucleic acid.

13. A method according to any one of claims 1-12, wherein the oligonucleotide or equivalent thereof, does not overlap a splice donor and/or a splice acceptor sequence of said exon.

14. A method for producing a compound capable of hybridising to at least two exons in a pre-mRNA encoded by a gene, comprising producing a compound comprising at least two parts wherein a first part comprises an oligonucleotide having at least 8 consecutive nucleotides that are complementary to a first of said at least two exons, and wherein a second part comprises an oligonucleotide having at least 8 consecutive nucleotides that are complementary to a second exon in said pre-mRNA, said compound comprising between 16 to 80 nucleotides, wherein said gene is a duchenne muscular dystrophy gene, a collagen VI alpha 1 gene (COL6A1), a myotubular myopathy 1 gene (MTM1), a dysferlin gene (DYSF), a laminin-alpha 2 gene (LAMA2), an emery-dreyfuss muscular dystrophy gene (EMD), and/or a calpain 3 gene (CAPN3).

15. A method according to claim 14, wherein said first and said second exon are separated in said pre-mRNA by at least one exon to which said oligonucleotide is not complementary.

16. A method according to claim 14 or claim 15, wherein stretches of nucleotides complementary to said at least two exons are separated by a linking moiety.

17. A method according to claim 16, wherein said linking moiety comprises between 4-40 nucleotides, preferably comprising at least 4 Uracil nucleotides.

18. A method according to any one of claims 14-17, wherein an equivalent of said compound is produced, preferably an equivalent according to claim 11.

## Patentansprüche

1. Verfahren zur Erzeugung eines Oligonukleotids, welches ein Bestimmen einer Region, aus einer Sekundärstruktur von RNA von einem Exon, die an einen anderen Teil der RNA hybridisiert ist (geschlossene Struktur), und einer Region, die nicht in der Struktur hybridisiert ist (offene Struktur), und nachfolgend ein Erzeugen eines Oligonukleotids umfasst, von dem zumindest drei aufeinander folgende Nukleotide des Oligonukleotids komplementär zu der geschlossenen Struktur sind und von dem zumindest weitere drei aufeinander folgende Nukleotide des Oligonukleotids komplementär zu der offenen Struktur sind, und wobei das Gen, von dem die RNA, die das Exon umfasst, transkribiert wird, ein Duchenne-Muskeldystrophie-Gen (DMD), ein Kollagen VI-alpha 1-Gen (COL6A1), ein myotubuläre Myopathie 1-Gen (MTM1), ein Dysferlin-Gen (DYSF), ein Laminin-alpha 2-Gen (LAMA2), ein Emery-Dreyfuss-Muskeldystrophie-Gen (EMD) und/oder ein Calpain 3-Gen (CAPN3) kodiert.

2. Verfahren nach Anspruch 1, bei dem die offene und die geschlossene Struktur aneinander angrenzen.

3. Verfahren nach Anspruch 1 oder Anspruch 2, bei dem das Oligonukleotid zu einem fortlaufenden Teil von zwischen 14 und 50 Nukleotiden der RNA komplementär ist.

4. Verfahren nach einem der Ansprüche 1 bis 3, bei dem das Oligonukleotid RNA umfasst.

5. Verfahren nach Anspruch 4, bei dem die RNA eine Modifizierung umfasst, vorzugsweise eine 2'-O-Methyl-modifizierte Ribose (RNA)- oder Desoxyribose (DNA)-Modifizierung.

6. Verfahren nach einem der Ansprüche 1 bis 5, bei dem prä-mRNA, die das Exon umfasst, ein unerwünschtes Spleißen in einem Lebewesen zeigt.

7. Verfahren nach Anspruch 6, bei dem das Fehlen des Exons von mRNA, die aus der prä-mRNA erzeugt worden ist, eine kodierende Region für ein Protein erzeugt.

8. Verfahren nach Anspruch 6 oder Anspruch 7, bei dem das Gen das Duchenne-Muskeldystrophie-Gen ist.

9. Verfahren nach Anspruch 8, bei dem das Exon das Exon 2, 8, 9, 17, 19, 29, 40 bis 46, 48 bis 53, 55 oder 59 umfasst.

10. Verfahren nach einem der Ansprüche 1 bis 8, bei dem ein Äquivalent des Oligonukleotids erzeugt wird, das ähnliche Hybridisierungseigenschaften bezogen auf die Art, nicht notwendigerweise bezogen auf die Menge, aufweist.

11. Verfahren nach Anspruch 10, bei dem das Äquivalent eine Peptidnukleinsäure, eine blockierte Nukleinsäure und/oder ein Morpholinophosphorodiamidat oder eine Kombination davon umfasst.

12. Verfahren nach Anspruch 11, bei dem das Äquivalent eine blockierte Nukleinsäure umfasst.

13. Verfahren nach einem der Ansprüche 1 bis 12, bei dem das Oligonukleotid oder ein dessen Äquivalent keine Spleißdonor- und/oder Spleißakzeptorsequenz des Exons überlappt.

14. Verfahren zur Herstellung einer Verbindung, die an zumindest zwei Exons in einer prä-mRNA hybridisieren kann, die durch ein Gen kodiert wird, umfassend ein Herstellen einer Verbindung, die zumindest zwei Teile umfasst, wobei ein erster Teil ein Oligonukleotid umfasst, das zumindest 8 aufeinander folgende Nukleotide aufweist, die zu einem ersten der zumindest zwei Exons komplementär sind, und wobei ein zweiter Teil ein Oligonukleotid umfasst, das zumindest 8 aufeinander folgende Nukleotide aufweist, die zu einem zweiten Exon in der prä-mRNA komplementär sind, wobei die Verbindung zwischen 16 bis 80 Nukleotide umfasst, wobei das Gen ein Duchenne-Muskeldystrophie-Gen, ein Kollagen VI-alpha 1-Gen (COL6A1), ein myotubuläre Myopathie 1-Gen (MTM1), ein Dysferlin-Gen (DYSF), ein Laminin-alpha 2-Gen (LAMA2), ein Emery-Dreyfuss-Muskeldystrophie-Gen (EMD) und/oder ein Calpain 3-Gen (CAPN3) ist.

15. Verfahren nach Anspruch 14, bei dem das erste Exon und das zweite Exon in der prä-mRNA durch zumindest ein Exon getrennt sind, zu dem das Oligonukleotid nicht komplementär ist.

16. Verfahren nach Anspruch 14 oder Anspruch 15, bei dem Stränge von Nukleotiden, die zu den zumindest zwei Exons komplementär sind, durch einen Verknüpfungsrest getrennt sind.

17. Verfahren nach Anspruch 16, bei dem der Verknüpfungsrest zwischen 4 bis 40 Nukleotide umfasst, vorzugsweise mit zumindest 4 Uracil-Nukleotiden.

18. Verfahren nach einem der Ansprüche 14 bis 17, bei dem ein Äquivalent der Verbindung hergestellt wird, vorzugsweise ein Äquivalent nach Anspruch 11.

## Revendications

1. Procédé de formation d'un oligonucléotide comprenant les étapes qui consistent à déterminer, à partir d'une structure secondaire d'ARN d'un exon, une région qui est hybridée à une autre partie dudit ARN (structure fermée) et une région qui n'est pas hybridée dans ladite structure (structure ouverte), et à former par la suite un oligonucléotide, au moins trois nucléotides consécutifs dudit oligonucléotide étant complémentaires de ladite structure fermée et au moins trois autres nucléotides consécutifs dudit oligonucléotide étant complémentaires de ladite structure ouverte, et dans lequel le gène à partir duquel ledit ARN comprenant ledit exon est transcrit, code pour un gène de la dystrophie musculaire de Duchenne (DMD, *« Duchenne muscular dystrophy »),* un gène du collagène VI alpha 1 (COL6A1), un gène de la myopathie myotubulaire 1 (MTM1, *« myotubular myopathy 1 gene »),* un gène de la dysferline (DYSF), un gène de la laminine alpha 2 (LAMA2), un gène de la dystrophie musculaire d'Emery-Dreifuss (EMD, *« Emery-Dreifuss muscular dystrophy gene* »), et/ou un gène de la calpaïne 3 (CAPN3).

2. Procédé selon la revendication 1, dans lequel lesdites structures ouvertes et fermées sont adjacentes l'une de l'autre.

3. Procédé selon la revendication 1 ou la revendication 2, dans lequel ledit oligonucléotide est complémentaire d'une partie consécutive de 14 à 50 nucléotides dudit ARN.

4. Procédé selon l'une quelconque des revendications 1 à 3, dans lequel ledit oligonucléotide comprend de l'ARN.

5. Procédé selon la revendication 4, dans lequel ledit ARN contient une modification, de préférence une modification de type ribose (ARN) ou désoxyribose (ADN) 2'-O-méthyl-modifié.

6. Procédé selon l'une quelconque des revendications 1 à 5, dans lequel un pré-ARNm comprenant ledit exon révèle un épissage non souhaité chez un sujet.

7. Procédé selon la revendication 6, dans lequel l'absence dudit exon de l'ARNm produit à partir dudit pré-ARNm engendre une région codante pour une protéine.

8. Procédé selon la revendication 6 ou la revendication 7, dans lequel ledit gène est le gène de la dystrophie musculaire de Duchenne.

9. Procédé selon la revendication 8, dans lequel ledit exon comprend l'exon 2, 8, 9, 17, 19, 29, 40 à 46, 48 à 53, 55 ou 59.

10. Procédé selon l'une quelconque des revendications 1 à 8, dans lequel un équivalent dudit oligonucléotide est engendré, comprenant des caractéristiques d'hybridation similaires en qualité, mais pas forcément en quantité.

11. Procédé selon la revendication 10, dans lequel ledit équivalent comprend un acide nucléique peptidique, un acide nucléique verrouillé et/ou un morpholinophosphorodiamidate, ou une combinaison de ceux-ci.

12. Procédé selon la revendication 11, dans lequel ledit équivalent comprend un acide nucléique verrouillé.

13. Procédé selon l'une quelconque des revendications 1 à 12, dans lequel l'oligonucléotide, ou un équivalent de celui-ci, ne se chevauche pas avec une séquence de type donneur d'épissage et/ou accepteur d'épissage dudit exon.

14. Procédé de production d'un composé capable de s'hybrider à au moins deux exons dans un pré-ARNm codé par un gène, comprenant l'étape qui consiste à produire un composé comprenant au moins deux parties, une première partie comprenant un oligonucléotide ayant au moins 8 nucléotides consécutifs qui sont complémentaires d'un premier desdits au moins deux exons, et une seconde partie comprenant un oligonucléotide ayant au moins 8 nucléotides consécutifs qui sont complémentaires d'un second exon dans ledit pré-ARNm, ledit composé comprenant de 16 à 80 nucléotides, ledit gène étant un gène de la dystrophie musculaire de Duchenne, un gène du collagène VI alpha 1 (COL6A1), un gène de la myopathie myotubulaire 1 (MTM1, *« Myotubular myopathy 1 gene »),* un gène de la dysferline (DYSF), un gène de la laminine alpha 2 (LAMA2), un gène de la dystrophie musculaire d'Emery-Dreifuss (EMD, *« Emery-Dreifuss muscular dystrophy gene* »), et/ou un gène de la calpaïne 3 (CAPN3).

15. Procédé selon la revendication 14, dans lequel ledit premier et ledit second exon sont séparés dans ledit pré-ARNm par au moins un exon duquel ledit oligonucléotide n'est pas complémentaire.

16. Procédé selon la revendication 14 ou la revendication 15, dans lequel les segments de nucléotides complémentaires desdits au moins deux exons sont séparés par un fragment de liaison.

17. Procédé selon la revendication 16, dans lequel ledit fragment de liaison comprend de 4 à 40 nucléotides, de préférence comprenant au moins 4 nucléotides uraciles.

18. Procédé selon l'une quelconque des revendications 14 à 17, dans lequel un équivalent dudit composé est produit, de préférence un équivalent selon la revendication 11.
